# EUROPEAN PATENT SPECIFICATION

(11) **EP 1 745 002 B1**
(45) Date of publication and mention of the grant of the patent: **03.11.2010**
(21) Application number: 05747382.9
(22) Date of filing: 03.05.2005
(51) Int. Cl.: C07C 59/70, C07C 323/20, C07D 213/30, C07D 295/08, C07D 333/16, C07D 409/06, A61K 31/192, A61P 3/04, A61P 3/06, A61P 3/10, A61P 5/50

(54) **NOVEL COMPOUNDS, THEIR PREPARATION AND USE**
NEUE VERBINDUNGEN, IHRE HERSTELLUNG UND VERWENDUNG
NOUVEAUX COMPOSES, LEUR PREPARATION ET LEUR UTILISATION

(30) Priority: 05.05.2004 DK 200400716
(43) Date of publication of application: 24.01.2007
(73) Proprietor: High Point Pharmaceuticals, LLC, High Point, NC 27265 (US)
(72) Inventor: HAVRANEK, Miroslav, 198 00 Praha 9 (CZ); SAUERBERG, Per, DK-3520 Farum (DK); PETTERSSON, Ingrid, DK-1864 Frederiksberg (DK)
(74) Representative: Russell, Lindsey
(86) International application number: PCT/EP2005/052010
(87) International publication number: WO 2005/105725

(56) References cited:
- WO-A-00/63153
- WO-A-2004/022533
- US-A1- 2001 041 709

## Description

### FIELD OF THE INVENTION

The present invention relates to novel compounds, to the use of these compounds as pharmaceutical compositions, and to pharmaceutical compositions comprising the compounds. More specifically, the compounds of the invention can be utilised in the treatment and/or prevention of conditions mediated by the Peroxisome Proliferator-Activated Receptors (PPAR), in particular the PPARδ subtype.

### BACKGROUND OF THE INVENTION

Coronary artery disease (CAD) is the major cause of death in Type 2 diabetic and metabolic syndrome patients (i.e. patients that fall within the 'deadly quartet' category of impaired glucose tolerance, insulin resistance, hypertriglyceridaemia and/or obesity).

The hypolipidaemic fibrates and antidiabetic thiazolidinediones separately display moderately effective triglyceride-lowering activities although they are neither potent nor efficacious enough to be a single therapy of choice for the dyslipidaemia often observed in Type 2 diabetic or metabolic syndrome patients. The thiazolidinediones also potently lower circulating glucose levels of Type 2 diabetic animal models and humans. Studies on the molecular actions of these compounds indicate that thiazolidinediones and fibrates exert their action by activating distinct transcription factors of the peroxisome proliferator activated receptor (PPAR) family, resulting in increased and decreased expression of specific enzymes and apolipoproteins respectively, both key-players in regulation of plasma triglyceride content. Fibrates, on the one hand, are PPARα activators, acting primarily in the liver. Thiazolidinediones, on the other hand, are high affinity ligands for PPARyacting primarily on adipose tissue.

Adipose tissue plays a central role in lipid homeostasis and the maintenance of energy balance in vertebrates. Adipocytes store energy in the form of triglycerides during periods of nutritional affluence and release it in the form of free fatty acids at times of nutritional deprivation. The development of white adipose tissue is the result of a continuous differentiation process throughout life. Much evidence points to the central role of PPARγ activation in initiating and regulating this cell differentiation. Several highly specialised proteins are induced during adipocyte differentiation, most of them being involved in lipid storage and metabolism. The exact link from activation of PPARγ to changes in glucose metabolism, most notably a decrease in insulin resistance in muscle, has not yet been clarified. A possible link is via free fatty acids such that activation of PPARγ induces Lipoprotein Lipase (LPL), Fatty Acid Transport Protein (FATP) and Acyl-CoA Synthetase (ACS) in adipose tissue but not in muscle tissue. This, in turn, reduces the concentration of free fatty acids in plasma dramatically, and due to substrate competition at the cellular level, skeletal muscle and other tissues with high metabolic rates eventually switch from fatty acid oxidation to glucose oxidation with decreased insulin resistance as a consequence.

PPARα is involved in stimulating β-oxidation of fatty acids. In rodents, a PPARα-mediated change in the expression of genes involved in fatty acid metabolism lies at the basis of the phenomenon of peroxisome proliferation, a pleiotropic cellular response, mainly limited to liver and kidney and which can lead to hepatocarcinogenesis in rodents. The phenomenon of peroxisome proliferation is not seen in man. In addition to its role in peroxisome proliferation in rodents, PPARα is also involved in the control of HDL cholesterol levels in rodents and humans. This effect is, at least partially, based on a PPARα-mediated transcriptional regulation of the major HDL apolipoproteins, apo A-I and apo A-II. The hypotriglyceridemic action of fibrates and fatty acids also involves PPARα and can be summarised as follows: (I) an increased lipolysis and clearance of remnant particles, due to changes in lipoprotein lipase and apo C-III levels, (II) a stimulation of cellular fatty acid uptake and their subsequent conversion to acyl-CoA derivatives by the induction of fatty acid binding protein and acyl-CoA synthase, (III) an induction of fatty acid β-oxidation pathways, (IV) a reduction in fatty acid and triglyceride synthesis, and finally (V) a decrease in VLDL production. Hence, both enhanced catabolism of triglyceride-rich particles as well as reduced secretion of VLDL particles constitutes mechanisms that contribute to the hypolipidemic effect of fibrates.

PPARδ activation was initially reported not to be involved in modulation of glucose or triglyceride levels. (Berger et al., j. Biol. Chem. , 1999, Vol 274, pp. 6718-6725). Later it has been shown that PPARδ activation leads to increased levels of HDL cholesterol in db/db mice (Leibowitz et al. FEBS letters 2000, 473, 333-336). Further, a PPARδ agonist when dosed to insulin-resistant middle-aged obese rhesus monkeys caused a dramitic dose-dependent rise in serum HDL cholesterol while lowering the levels of small dense LDL, fasting triglycerides and fasting insulin (Oliver et al. PNAS 2001, 98, 5306-5311 ).The same paper also showed that PPARδ activation increased the reverse cholesterol transporter ATP-binding cassette A1 and induced apolipoprotein A1-specific cholesterol efflux. The involvement of PPARδ in fatty acid oxidation in muscles was further substantiated in PPARα knockout mice. Muoio et al. (J. Biol. Chem. 2002, 277, 26089-26097) showed that the high levels of PPARδ in skeletal muscle can compensate for deficiency in PPARα.

Recently, two different transgenic mouse models over-expressing PPARδ in either adipose tissue (Cell 2003, 113, 159-170) or in muscle tissue (FASEB J. published online 2 October 2003, doi : 10.1096/fj.03-0269fje) have both shown up-regulation of genes (LPL, FABP, FAT, CD36, CPT1b, and ACS) and proteins (UCP-2) responsible for lipid uptake and metabolism and energy uncoupling. Both types of mice had reduced adipose tissue and were protected against high fat diet induced body weight gain. Further, pharmacological treatment of both high fat diet induced insulin resistant mice and diabetic ob/ob with the potent PPARδ agonist GW501516 showed lowering of plasma glucose and insulin and improved insulin sensitivity (PNAS 2003, 100, 15924-15929). In vivo increased oxygen consumption suggesting fuel-switch from glucose to FFA, as well as FFA oxidation in skeletal muscle was demonstrated both in vivo and in vitro. Supportive for the hypothesis of skeletal muscle being the major target organ were two publications on in vitro treatment of C2C12 muscle cells with GW501516 showing regulation of genes involved with TG hydrolysis and FFA oxidation (LPL↑, ACS4↑, CTP1↑), preferential lipid utilization (PDK4↑), energy expenditure (UCP1↑,-2↑, -3↑) and lipid efflux (ABCA↿/G1↑) (BioChem. Biophys. Acta 2003, 1633, 43-50; Mol. Endocrin. 2003, 17, 2477-2493). Direct and an indirect mechanisms recently demonstrated prompted the authors to suggest that "PPARδ and its ligands may serve as therapeutic targets to attenuate inflammation and slow the progression of atherosclerosis" (Science 2003, 302, 453-457)*.*

Taken together these observations suggest that PPARδ activation is useful in the treatment and prevention of cardiovascular diseases and conditions including atherosclerosis, hypertriglyceridemia, and mixed dyslipidaemia as well as type 2 diabetes.

A number of PPARδ compounds have been reported to be useful in the treatment of hyperglycemia, hyperlipidemia and hypercholesterolemia (WO 01/00603, WO 02/59098, WO 03/084916, WO 031074050, WO 03/074051, WO 03/074052, WO 03/035603, WO 03/97607, WO 04/005253, WO 03/33493, WO 03/16291, WO 02/76957,02/46154, WO 03/16265, WO 02/100812, WO 02/98840, WO 02/80899, WO 02/79162, W003/0721 00, WO 01/25181, WO 02/14291, WO 01/79197, WO 99/4815, WO 97/28149, WO 98/27974, WO 97/28115, WO 97/27857, WO 97/28137, WO 97/27847).

Glucose lowering as a single approach does not overcome the macrovascular complications associated with Type 2 diabetes and metabolic syndrome. Novel treatments of Type 2 diabetes and metabolic syndrome must therefore aim at lowering both the overt hypertriglyceridaemia associated with these syndromes as well as alleviation of hyperglycaemia.

This indicate that research for compounds displaying various degree of PPARα, PPARγ and PPARδ activation should lead to the discovery of efficacious triglyceride and/or cholesterol and/or glucose lowering drugs that have great potential in the treatment of diseases such as type 2 diabetes, dyslipidemia, syndrome X (including the metabolic syndrome, i.e. impaired glucose tolerance, insulin resistance, hypertriglyceridaemia and/or obesity), cardiovascular diseases (including atherosclerosis) and hypercholesteremia.

WO 2004/022533 discloses compounds of formula: 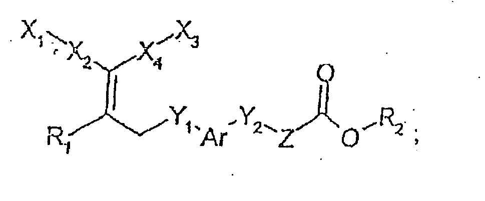 which are said to be useful in the treatment and/or prevention of conditions mediated by PPAR.

### DEFINITIONS

In the structural formulas given herein and throughout the present specification the following terms have the indicated meaning:

The term "C₁₋₆-alkyl" as used herein, alone or in combination, represent a linear or branched, saturated hydrocarbon chain having the indicated number of carbon atoms. Representative examples include, but are not limited to methyl, ethyl, n-propyl, isopropyl, butyl, isobutyl, *sec*-butyl, *tert*-butyl*,* pentyl, isopentyl, hexyl, isohexyl and the like.

The term "C₁₋₆-alkylcarbonyl as used herein, represents a "C₁₋₆-alkyl" group as defined above having the indicated number of carbon atoms linked through a carbonyl group. Representative examples include, but are not limited to, methylcarbonyl, ethylcarbonyl, n-propylcarbonyl, isopropylcarbonyl, butylcarbonyl, isobutylcarbonyl, *sec*-butylcarbonyl, *tert*butylcarbonyl, n-pentylcarbonyl, isopentylcarbonyl, neopentylcarbonyl, *tert*-pentylcarbonyl, n-hexylcarbonyl, isohexylcarbonyl and the like.

The term "C₁₋₆-alkylsulfonyl" as used herein refers to a monovalent substituent comprising a "C₁₋₆-alkyl" group as defined above linked through a sulfonyl group. Representative examples include, but are not limited to, methylsulfonyl, ethylsulfonyl, n-propylsulfonyl, isopropylsulfonyl, n-butylsulfonyl, isobutylsulfonyl, *sec*-butylsulfonyl, *tert*-butylsulfonyl,n-pentylsulfonyl, isopentylsulfonyl, neopentylsulfonyl, *tert*-pentylsulfonyl, n-hexylsulfonyl, isohexylsulfonyl and the like.

The term "C₁₋₆-alkylamido" as used herein, refers to an acyl group linked through an amino group; Representative examples include, but are not limited to acetylamino, propionylamino, butyrylamino, isobutyrylamino, pivaloylamino, valerylamino and the like.

The term "C₃₋₆-cycloalkyl" as used herein, alone or in combination, represent a saturated monocyclic hydrocarbon group having the indicated number of carbon atoms. Representative examples include, but are not limited to cyclopropyl, cyclobutyl, cyclopentyl, cyclohexyl and the like.

The term "C₃₋₈-cycloalkyl-C₁₋₆-alkyl" as used herein refers to a "C₃₋₆-cycloalkyl" group as defined above whereto is attached a "C₁₋₆-alkyl" group as defined above. Representative examples include, but are not limited to cyclopropylmethyl, cyclobutylethyl, cyclopentylpropyl, cyclohexylbutyl and the like.

The term "C₃₋₆-cycloalkyl-C₁₋₆-alkoxy" as used herein refers to a "C₃₋₆-cycloalkyl" group as defined above whereto is attached a "C₁₋₆-alkoxy" group as defined above. Representative examples include, but are not limited to cyclopropylmethoxy, cyclobutylethoxy, cyclopentylpropoxy, cyclohexylbutoxy and the like.

The term "C₃₋₆-cycloalkyl-C₁₋₆-alkylthio" as used herein refers a "C₃₋₆-cycloalkyl" group as defined above whereto is attached a "C₁₋₆-alkylthio" group as defined above. Representative examples include, but are not limited to cyclopropylmethylthio, cyclobutylethylthio, cyclopentylbutylthio, cyclohexylpentylthio and the like.

The term "C₃₋₆-cycloalkyl-C₁₋₆-alkyl-carbonyl" as used herein refers to a "C₃₋₆-cycloalkyl" group as defined above whereto is attached a "C₁₋₆-alkyl-carbonyl" group as defined above. Representative examples include, but are not limited to cyclopropylmethylcarbonyl, cyclobutylethylcarbonyl, cyclopentylpropylcarbonyl, cyclohexylbutylcarbonyl and the like.

The term "C₃₋₆-cycloalkylcarbonyl" as used herein refers to a "C₃₋₆-cycloalkyl" group as defined above linked through a carbonyl group. Representative examples include, but are not limited to cyclopropylcarbonyl, cyclobutylcarbonyl, cyclopentylcarbonyl, cyclohexylcarbonyl and the like.

The term "C₂₋₆-alkenyl" as used herein, represent an olefinically unsaturated branched or straight hydrocarbon group having from 2 to the specified number of carbon atoms and at least one double bond. Representative examples include, but are not limited to, vinyl, 1-propenyl, 2-propenyl, allyl, iso-propenyl, 1,3-butadienyl, 1-butenyl, hexenyl, pentenyl and the like.

The term "C₂₋₆-alkynyl" as used herein, represent an unsaturated branched or straight hydrocarbon group having from 2 to the specified number of carbon atoms and at least one triple bond. Representative examples include, but are not limited to, 1-propynyl, 2-propynyl, 1-butynyl, 2-butynyl, 1-pentynyl, 2-pentynyl and the like.

The term "C₄₋₆-alkenynyl" as used herein, represent an unsaturated branched or straight hydrocarbon group having from 4 to the specified number of carbon atoms and both at least one double bond and at least one triple bond. Representative examples include, but are not limited to, 1-penten-4-ynyl, 3-penten-1-ynyl, 1,3-hexadiene-5-ynyl and the like.

The term "C₁₋₆-alkoxy" as used herein, alone or in combination, refers to a straight or branched configuration linked through an ether oxygen having its free valence bond from the ether oxygen. Examples of linear alkoxy groups are methoxy, ethoxy, propoxy, butoxy, pentoxy, hexoxy and the like. Examples of branched alkoxy are isopropoxy, sec-butoxy, tert-butoxy, isopentyloxy, isohexyloxy and the like.

The term "C₁₋₆-alkoxycarbonyl" as used herein refers to a "C₁₋₆-alkoxy" group as defined above linked through a carbonyl group. Representative examples include, but are not limited to methoxycarbonyl, ethoxycarbonyl, propoxycarbonyl, butoxycarbonyl, pentoxycarbonyl, hexoxycarbonyl, isopropoxycarbonyl, sec-butoxycarbonyl, tert-butoxycarbonyl, isopentyloxycarbonyl, isohexyloxycarbonyl and the like.

The term "C₃₋₆-cycloalkoxy" as used herein, alone or in combination, represent a saturated monocyclic hydrocarbon group having the indicated number of carbon atoms linked through an ether oxygen having its free valence bond from the ether oxygen. Examples of cycloalkoxy groups are cyclopropyloxy, cyclobutyloxy, cyclopentyloxy, cyclohexyloxy and the like.

The term "C₃₋₆-cycloalkoxycarbonyl" as used herein refers to as used herein refers to a "C₃₋₆-cycloalkoxy" group as defined above linked through a carbonyl group. Representative examples include, but are not limited to cyclopropyloxycarbonyl, cyclobutyloxycarbonyl, cyclopentyloxycarbonyl, cyclohexyloxycarbonyl and the like.

The term "C₃₋₆-cycloalkyl-C₁₋₆-alkoxycarbonyl" as used herein refers to a "C₃₋₆-cycloalkyl" group as defined above whereto is attached a "C₁₋₆-alkoxycarbonyl" group as defined above. Representative examples include, but are not limited to cyclopropylmethoxycarbonyl, cyclobutylethoxycarbonyl, cyclopentylpropoxycarbonyl, cyclohexylbutoxycarbonyl and the like.

The term "C₁₋₆-alkylthio" as used herein, alone or in combination, refers to a straight or branched monovalent substituent comprising a "C₁₋₆-alkyl" group as defined above linked through a divalent sulfur atom having its free valence bond from the sulfur atom and having 1 to 6 carbon atoms. Representative examples include, but are not limited to, methylthio, ethylthio, propylthio, butylthio, pentylthio and the like.

The term "C₃₋₆-cycloalkylthio" as used herein, alone or in combination, represent a saturated monocyclic hydrocarbon group having the indicated number of carbon atoms linked through a divalent sulfur atom having its free valence bond from the sulfur atom. Examples of cycloalkoxy groups are cyclopropylthio, cyclobutylthio, cyclopentylthio, cyclohexylthio and the like.

The term "C₁₋₆-alkylamino" as used herein, alone or in combination, refers to a straight or branched monovalent substituent comprising a "C₁₋₆-alkyl" group as defined above linked through amino having a free valence bond from the nitrogen atom. Representative examples include, but are not limited to, methylamino, ethylamino, propylamino, butylamino, pentylamino and the like.

The term "C₁₋₆-alkylamino-C₁₋₆-alkyl" as used herein refers to a "C₁₋₆-alkylamino" group as defined above whereto is attached a "C₁₋₆-alkyl" group as defined above. Representative examples include, but are not limited to methylaminomethyl, ethylaminoethyl, propylaminopropyl, butylaminopentyl, pentylaminohexyl and the like.

The term "C₁₋₆-alkyaminocarbonyl" as used herein refers to a monovalent substituent comprising a C₁₋₆-monoalkylamino group linked through a carbonyl group such as e.g. methylaminocarbonyl, ethylaminocarbonyl, n-propylaminocarbonyl, isopropylaminocarbonyl, n-butylaminocarbonyl, sec-butylaminocarbonyl, isobutylaminocarbonyl, tert-butylaminocarbonyl, n-pentylaminocarbonyl, 2-methylbutylaminocarbonyl, 3-methylbutylaminocarbonyl, n-hexylaminocarbonyl, 4-methylpentylaminocarbonyl, neopentylaminocarbonyl, n-hexylaminocarbonyl and 2-2-dimethylpropylaminocarbonyl and the like.

The term "C₃₋₆-cycloalkylamino" as used herein, alone or in combination, represent a saturated monocyclic hydrocarbon group having the indicated number of carbon atoms linked through amino having a free valence bond from the nitrogen atom. Representative examples include, but are not limited to, cyclopropylamino, cyclobutylamino, cyclopentylamino, cyclohexylamino and the like.

The term "C₁₋₆-alkoxyC₁₋₆-alkyl" as used herein, alone or in combination, refers to a "C₁₋₆-alkyl" group as defined above whereto is attached a "C₁₋₆-alkoxy" group as defined above. Representative examples include, but are not limited to, methoxymethyl, ethoxymethyl, methoxyethyl, ethoxyethyl and the like.

The term "aryl" as used herein refers to an aromatic monocyclic or an aromatic fused bi- or tricyclic hydrocarbon group. Representative examples include, but are not limited to, phenyl, naphthyl, anthracenyl, phenanthrenyl, azulenyl, fluorenyl, indenyl, pentalenyl and the like.

The term "arylene" as used herein refers to divalent aromatic monocyclic or a divalent aromatic fused bi- or tricyclic hydrocarbon group. Representative examples include, but are not limited to, phenylene, naphthylene and the like.

The term "arylcarbonyl" as used herein represents an "aryl" group as defined above linked through a carbonyl group. Representative examples include, but are not limited to, phenylcarbonyl, naphthylcarbonyl, anthracenylcarbonyl, phenanthrenylcarbonyl, azulenylcarbonyl and the like.

The term "arylsulfonyl" as used herein refers to an "aryl" group as defined above linked through a sulfonyl group. Representative examples include, but are not limited to, phenylsulfonyl, naphthylsulfonyl, anthracenylsulfonyl, phenanthrenylsulfonyl, azulenylsulfonyl, and the like.

The term "arylamido" as used herein refers to an arylcarbonyl group linked through an amino group. Representative examples include, but are not limited to phenylcarbonylamino, naphthylcarbonylamino, anthracenylcarbonylamino, phenanthrenylcarbonylamino, azulenylcarbonylamino and the like.

The term "halogen" means fluorine, chlorine, bromine or iodine.

The term "perhalomethyl" means trifluoromethyl, trichloromethyl, tribromomethyl or triiodomethyl.

The term "perhalomethoxy" means trifluoromethoxy, trichloromethoxy, tribromomethoxy or triiodomethoxy.

The term "C₁₋₆-dialkylamino" as used herein refers to an amino group wherein the two hydrogen atoms independently are substituted with a straight or branched, saturated hydrocarbon chain having the indicated number of carbon atoms. Representative examples include, but are not limited to, dimethylamino, N-ethyl-N-methylamino, diethylamino, dipropylamino, N-(n-butyl)-N-methylamino, di(n-pentyl)amino and the like.

The term "acyl" as used herein refers to a monovalent substituent comprising a "C₁₋₆-alkyl" group as defined above linked through a carbonyl group. Representative examples include, but are not limited to, acetyl, propionyl, butyryl, isobutyryl, pivaloyl, valeryl and the like.

The term "heteroaryl" as used herein, alone or in combination, refers to a monovalent substituent comprising a 5-7 membered monocyclic aromatic system or a 8-10 membered bicyclic aromatic system containing one or more heteroatoms selected from nitrogen, oxygen and sulfur, e.g. furyl, thienyl, pyrrolyl, imidazolyl, pyrazolyl, triazolyl, pyridyl, pyrazinyl, pyrimidinyl, pyridazinyl, isothiazolyl, isoxazolyl, oxazolyl, oxadiazolyl, thiadiazolyl, quinolyl, isoquinolyl, quinazolinyl, quinoxalinnyl, isoindolyl, indolyl, benzimidazolyl, benzoxazolyl, benzothiazolyl, benzofuranyl, tetrazolyl, carbazolyl, benzothienyl, pteridinyl and purinyl and the like.

The term "heteroarylene" as used herein, alone or in combination, refers to divalent 5-7 membered monocyclic aromatic system or a 8-10 membered bicyclic aromatic system containing one or more heteroatoms selected from nitrogen, oxygen and sulfur, e.g. furylene, thienylene, pyrrolylene, imidazolylene, pyrazolylene, triazolylene, pyridylene, pyrazinylene, pyrimidinylene, pyridazinylene, isothiazolylene, isoxazolylene, oxazolylene, oxadiazolylene, thiadiazolylene, quinolylene, isoquinolylene, quinazolinylene, quinoxalinnylene, indolylene, benzimidazolylene, benzofuranylene, benzothienylene, pteridinylene and purinylene and the like.

The term "heteroaryloxy" as used herein, alone or in combination, refers to a heteroaryl as defined herein linked to an oxygen atom having its free valence bond from the oxygen atom e.g. pyrrolyloxy, imidazolyloxy, pyrazolyloxy, triazolyloxy, pyrazinyloxy, pyrimidinyloxy, pyridazinyloxy, isothiazolyloxy, isoxazolyloxy, oxazolyloxy, oxadiazolyloxy, thiadiazolyloxy, quinolinyloxy, isoquinolinyloxy, quinazolinyloxy, quinoxalinyloxy, indoltloxy, benzimidazolyloxy, benzofuranyloxy, pteridinyloxy and purinyloxy and the like.

The term "heteroarylcarbonyl" as used herein refers to a "heteroaryl" group as defined above linked through a carbonyl group. Representative examples include, but are not limited to furylcarbonyl, thienylcarbonyl, pyrrolylcarbonyl, imidazolylcarbonyl, pyrazolylcarbonyl, triazolylcarbonyl, pyridylcarbonyl, pyrazinylcarbonyl, pyrimidinylcarbonyl, pyridazinylcarbonyl, isothiazolylcarbonyl, isoxazolylcarbonyl, oxazolylcarbonyl, oxadiazolylcarbonyl, thiadiazolylcarbonyl, quinolylcarbonyl, isoquinolylcarbonyl, quinazolinylcarbonyl, quinoxalinnylcarbonyl, isoindolylcarbonyl, indolylcarbonyl, benzimidazolylcarbonyl, benzoxazolylcarbonyl, benzothiazolylcarbonyl, benzofuranylcarbonyl, tetrazolylcarbonyl, carbazolylcarbonyl, benzothienylcarbonyl, pteridinylcarbonyl, purinylcarbonyl and the like.

The term "heteroarylsulfonyl" as used herein refers to a "heteroaryl" group as defined above linked through a "sulfonyl" group. Representative examples include, but are not limited to furylsulfonyl, thienylsulfonyl, pyrrolylsulfonyl, imidazolylsulfonyl, pyrazolylsulfonyl, triazolylsulfonyl, pyridylsulfonyl, pyrazinylsulfonyl, pyrimidinylsulfonyl, pyridazinylsulfonyl, isothiazolylsulfonyl, isoxazolylsulfonyl, oxazolylsulfonyl, oxadiazolylsulfonyl, thiadiazolylsulfonyl, quinolylsulfonyl, isoquinolylsulfonyl, quinazolinylsulfonyl, quinoxalinnylsulfonyl, isoindolylsulfonyl, indolylsulfonyl, benzimidazolylsulfonyl, benzoxazolylsulfonyl, benzothiazolylsulfonyl, benzofuranylsulfonyl, tetrazolylsulfonyl, carbazolylsulfonyl, benzothienylsulfonyl, pteridinylsulfonyl and purinylsulfonyl and the like.

The term "aralkyl" as used herein refers to a straight or branched saturated carbon chain containing from 1 to 6 carbons substituted with an aromatic carbohydride. Representative examples include, but are not limited to, benzyl, phenethyl, 3-phenylpropyl, 1-naphthylmethyl, 2-(1-naphthyl)ethyl and the like.

The term "aryloxy" as used herein refers to phenoxy, 1-naphthyloxy, 2-naphthyloxy and the like.

The term "aralkoxy" as used herein refers to a C₁₋₆-alkoxy group substituted with an aromatic carbohydride, such as benzyloxy, phenethoxy, 3-phenylpropoxy, 1-naphthyl-methoxy, 2-(1-naphtyl)ethoxy and the like.

The term "heteroaralkyl" as used herein refers to a straight or branched saturated carbon chain containing from 1 to 6 carbons substituted with a heteroaryl group; such as (2-furyl)methyl, (3-furyl)methyl, (2-thienyl)methyl, (3-thienyl)methyl, (2-pyridyl)methyl, 1-methyl-1-(2-pyrimidyl)ethyl and the like.

The term "heteroaralkoxy" as used herein refers to a heteroarylalkyl as defined herein linked to an oxygen atom having its free valence bond from the oxygen atom. Representative examples include, but are not limited to, (2-furyl)methyl, (3-furyl)methyl, (2-thienyl)methyl, (3-thienyl)methyl, (2-pyridyl)methyl, 1-methyl-1-(2-pyrimidyl)ethyl linked to oxygen, and the like.

The term "arylthio" as used herein, alone or in combination, refers to an aryl group linked through a divalent sulfur atom having its free valence bond from the sulfur atom. The aryl group is optionally mono- or polysubstituted as defined in the claims. Representative examples include, but are not limited to, phenylthio and the like.

The term "heteroarylthio" as used herein refers to a "heteroaryl" group as defined above linked through a divalent sulfur atom having its free valence bond from the sulfur atom. Representative examples include, but are not limited to furylthio, thienylthio, pyrrolylthio, imidazolylthio, pyrazolylthio, triazolylthio, pyridylthio, pyrazinylthio, pyrimidinylthio, pyridazinylthio, isothiazolylthio, isoxazolylthio, oxazolylthio, oxadiazolylthio, thiadiazolylthio, quinolylthio, isoquinolylthio, quinazolinylthio, quinoxalinnylthio, isoindolylthio, indolylthio, benzimidazolylthio, benzoxazolylthio, benzothiazolylthio, benzofuranylthio, tetrazolylthio, carbazolylthio, benzothienylthio, pteridinylthio,purinylthio and the like.

The term "aryl-C₁₋₆-alkylthio" as used herein refers to an "aryl" group as defined above whereto is attached a "C₁₋₆-alkylthio" group as defined above. Representative examples include, but are not limited to phenylmethylthio, naphthylethylthio, anthracenylpropylthio, phenanthrenylbutylthio, azulenylpentylthio, fluorenylhexylthio, indenylmethylthio, pentalenylethylthio and the like.

The term "heteroaryl-C₁₋₆-alkylthio" as used herein refers to a "heteroaryl" group as defined above whereto is attached a "C₁₋₆-alkylthio" group as defined above. Representative examples include, but are not limited to furylmethylthio, thienylethylthio, pyrrolylpropylthio, imidazolylbutylthio, pyrazolylpentylthio, triazolylhexylthio, pyridylmethylthio, pyrazinylethylthio, pyrimidinylpropylthio, pyridazinylbutylthio, isothiazolylpentylthio, isoxazolylhexyithio, oxazolylmethylthio, oxadiazolylethylthio, thiadiazolylpropylthio, quinolyibutythio, isoquinolypentyllthlo, quinazolinylhexylthio, quinoxalinnylmethylthio, isoindolylethylthio, indolylpropylthio, benzimidazolylbutylthio, benzoxazolylpentylthio, benzothiazolylhexylthio, benzofuranylmethylthio, tetrazolylethylthio, carbazolylpropylthio, benzothienylbutylthio, pteridinypentyllthio,purinylhexylthio and the like.

The term "C₃₋₆-cycloalkylsulfonyl" as used herein refers to a"C₃₋₆-cycloalkyl" group as defined above linked through a sulfunyl group. Representative examples include, but are not limited to cyclopropylsulfonyl, cyclobutylsulfonyl, cyclopentylsulfonyl, cyclohexylsulfonyl and the like.

The term "C₃₋₆-cycloalkyl-C₁₋₆-alkylsulfonyl" as used herein refers to a "C₃₋₆-cycloalkyl" group as defined above whereto is attached a "C₁₋₆-alkylsulfonyl" group as defined above. Representative examples include, but are not limited to cyclopropylmethylsulfonyl, cyclobutylethylsulfonyl, cyclopentylpentylsulfonyl, cyclohexylbutylsulfonyl and the like.

The term "amino-C₁₋₆-alkyl" as used herein refers to, but are not limited to, aminomethyl, aminoethyl, aminon-propyl, aminoisopropyl, aminobutyl, aminoisobutyl, amino*sec-*butyl, amino *tert*-butyl, aminopentyl, aminoisopentyl, aminohexyl, aminoisohexyl and the like.

The term "C₃₋₆-cycloalkylamino" as used herein refers to a "C₃₋₆-cycloalkyl" group as defined above linked through amino having a free valence bond from the nitrogen atom. Representative examples include, but are not limited to cyclopropylamino, cyclobutylamino, cyclopentylamino, cyclohexylamino and the like.

The term "C₃₋₆-cycloalkylamido" as used herein refers to a "C₃₋₆-cycloalkylcarbonyl" group as defined above linked through an amino group. Representative examples include, but are not limited to cyclopropylamido, cyclobutylamido, cyclopentylamido, cyclohexylamido and the like.

The term "C₃₋₆-cycloalkyl-C₁₋₆-alkylamido" as used herein refers to "C₃₋₆-cycloalkyl" group as defined above whereto is attached a "C₁₋₆-alkylamido" group as defined above. Representative examples include, but are not limited to cyclopropylmethylamido, cyclobutylethylamido, cyclopentylbutylamido, cyclohexylpentylamido and the like.

The term "C₃₋₆-cycloalkylaminocarbonyl" as used herein refers to a "C₃₋₆-cycloalkylamino" group as defined above linked through a carbonyl group. Representative examples include, but are not limited to cyclopropylaminocarbonyl, cyclobutylaminocarbonyl, cyclopentylaminocarbonyl, cyclohexylaminocarbonyl and the like.

The term "C₃₋₆-cycloalkyl-C₁₋₆-alkylaminocarbonyl" as used herein refers to a "C₃₋₆-cycloalkyl" group as defined above whereto is attached a "C₁₋₆-alkylaminocarbonyl" group as defined above. Representative examples include, but are not limited to cyclopropylmethylaminocarbonyl, cyclobutylethylaminocarbonyl, cyclopentylpropylaminocarbonyl, cyclohexylbytylaminocarbonyl and the like.

The term "C₃₋₆-cycloalkyl-C₁₋₆-alkylamino" as used herein refers to a "C₃₋₆-cycloalkyl" group as defined above whereto is attached a "C₁₋₆-alkylamino" group as defined above. Representative examples include, but are not limited to cyclopropylmethylamino, cyclobutylethylamino, cyclopentylpropylamino, cyclohexylbytylamino and the like.

The term "C₁₋₆-alkylsulfamoyl" as used herein refers to "C₁₋₆-alkylamino" group as defined above linked through an sulfonyl group. Representative examples include, but are not limited to methylsulfamoyl, ethylsulfamoyl, n-propylsulfamoyl, isopropylsulfamoyl, butylsulfamoyl, isobutylsulfamoyl, *sec*-butylsulfamoyl, *tert*-butylsulfamoyl, pentylsulfamoyl, isopentylsulfamoyl, hexylsulfamoyl, isohexylsulfamoyl and the like.

The term "di-(C₁₋₆-alkyl)sulfamoyl" as used herein refers to two "C₁₋₆-alkyl" groups defined as above linked to the nitrogen atom in the sulfamoyl group. Representative examples include, but are not limited to N,N-dimethylsulfamoyl, N-methyl-N-ethylsulfamoyl, N-ethyl-N-n-propylsulfamoyl, N-hexyl-N-isopropylsulfamoyl, N,N-dibutylsulfamoyl, N-methyl-N-isobutylsulfamoyl, N-pentyl-N-ethyl-*sec*-butylsulfamoyl, N,propyl-*tert*-butylsulfamoyl, N-hexyl-N-pentylsulfamoyl, N-methyl-N-isopentylsulfamoyl, N,N-dihexylsulfamoyl, N-propyl-N-isohexylsulfamoyl and the like.

The term "di-(C₁₋₆-alkyl)amino-C₁₋₆-alkyl" as used herein refers to to two "C₁₋₆-alkyl" groups defined as above linked through a nitrogen atom to a "C₁₋₆-alkyl" group as defined above. Representative examples include, but are not limited to N,N-dimethylaminomethyl, N-methyl-N-ethylaminoethyl, N-ethyl-N-propylaminopropyl, N,N-dibutylaminopentyl, N-hexyl-N-pentylaminohexyl and the like

The term "di-(C₁₋₆-alkyl)aminocarbonyl" as used herein refers to to two "C₁₋₆-alkyl" groups defined as above linked through a nitrogen atom to a carbonyl group. Representative examples include, but are not limited to N,N-dimethylaminocarbonyl, N-methyl-N-ethylaminocarbonyl, N-ethyl-N-propylaminocarbonyl, N,N-dibutylaminocarbonyl, N-hexyl-N-pentylaminocarbonyl and the like.

The term "di-(C₃₋₆-cycloalkyl-C₁₋₆-alkyl)aminocarbonyl" as used herein refers to two "C₃₋₆-cycloalkyl-C₁₋₆-alkyl" groups as defined above linked through a nitrogen atom to a carbonyl group.

The term "di-(C₁₋₆-alkyl)amino" as used herein refers to two "C₁₋₆-alkyl" groups as defined above linked through an amino group.

The term "di-(C₃₋₆-cycloalkyl)amino" as used herein refers to two "C₃₋₆-cycloalkyl" groups as defined above linked through an amino group.

The term "di-(C₃₋₆-cycloalkyl-C₁₋₆-alkyl)amino" as used herein refers to two "C₃₋₆-cycloalkyl-C₁₋₆-alkyl" groups as defined above linked through an amino group.

The term "heterocyclyl" as used herein represents a saturated 3 to 12 membered monocyclic ring containing one or more heteroatoms selected from nitrogen, oxygen, sulfur, S(=O) and S(=O)₂. Representative examples are aziridinyl, azetidinyl, oxetanyl, pyrrolidinyl, imidazolidinyl, oxazolidinyl, thiazolidinyl, isothiazolidinyl, piperidinyl, homopiperidinyl, piperazinyl, morpholinyl, thiomorpholinyl, 1-oxo-thiomorpholinyl, 1,1-dioxo-thiomorpholinyl, tetrahydrofuranyl, tetrahydrothienyl, tetrahydro-1,1-dioxothienyl, tetrahydropyranyl, tetrahydrothiopyranyl, 1,4-dioxanyl, 1,3-dioxanyl, and the like. Heterocyclyl is also intended to represent a saturated bicyclic ring containing one or more heteroatoms selected from nitrogen, oxygen, sulfur, S(=O) and S(=O)₂. Representative examples are octahydroindolyl, decahydroquinoxalinyl, and the like. Heterocyclyl is also intended to represent a saturated heterocyclic ring containing one or more heteroatoms selected from nitrogen, oxygen, sulfur, S(=O) and S(=O)₂ and having one or two bridges. Representative examples are 3-azabicylo[3.2.2]nonyl, 2-azabicyclo[2.2.1]heptyl, 3-azabicyclo[3.1.0]hexyl, 2,5-diazabicyclo[2.2.1]heptyl, atropinyl, tropinyl, quinuclidinyl, 1,4-diazabicyclo[2.2.2]octanyl, and the like. Heterocyclyl is also intended to represent a saturated heterocyclic ring containing one or more heteroatoms selected from nitrogen, oxygen, sulfur, S(=O) and S(=O)₂ and containing one or more spiro atoms. Representative examples are 1,4-dioxaspiro[4.5]decanyl, 8-azaspiro[4.5]decanyl, 2,8-diazaspiro[4.5]decanyl, and the like.

The term "five to eight member ring" as used herein refers to a saturated or unsaturated, substituted or unsubstituted hydrocarbon chain or hydrocarbon-heteroatom chain having from 3 to 6 atoms together with the the carbon atom in Ar, to which they are attached, and the adjacent carbon atom form a five to eight member ring.

Certain of the above defined terms may occur more than once in the structural formulae, and upon such occurrence each term shall be defined independently of the other.

The term "optionally substituted" as used herein means that the groups in question are either unsubstituted or substituted with one or more of the substituents specified. When the groups in question are substituted with more than one substituent the substituents may be the same or different.

The term "treatment" is defined as the management and care of a patient for the purpose of combating or alleviating the disease, condition or disorder, and the term includes the administration of the active compound to prevent the onset of the symptoms or complications, or alleviating the symptoms or complications, or eliminating the disease, condition, or disorder.

The term "pharmaceutically acceptable" is defined as being suitable for administration to humans without adverse events.

### DESCRIPTION OF THE INVENTION

The present invention relates to compounds of the general formula (I): 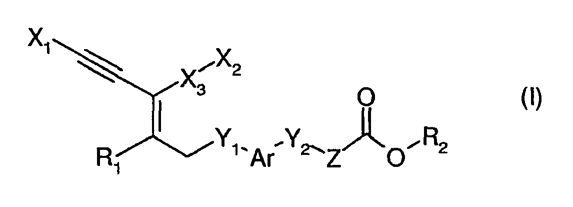
wherein X₁ is aryl or heteroaryl each of which is optionally substituted with one or more substituents selected from
   - halogen, hydroxy, cyano, amino or carboxy; or
   - C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloakyllcarbonyl, C₃₋₆-cycloalkyl- C₁₋₆-alkylcarbonyl, arylcarbonyl, heteroarylcarbonyl, C₁₋₆-alkylsulfonyl, C₃₋₆-cycloalkylsulfonyl, C₃₋₆-cycloalkyl-C₁₋₆₋alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocatbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more halogens, CN and OH; or
X₁ is C₁₋₆-alkyl, C₃₋₆-cycloalkyl, C₂₋₆-alkenyl or C₃₋₆-cycloalkyl-C₁₋₆-alkyl each of which is optionally substituted with one or more substituents selected from
   - halogen, hydroxy, cyano, amino or carboxy; or
   - C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylamino-carbonyl, C₃₋₆-cycloalkyl-C₁₋₆-alkylaminocarbonyl, di-(C₁₋₆-alkyl)aminocarbonyl, di-(C₃₋₆-₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more halogens, CN and OH; or
X₁ is aralkyl or heteroaralkyl each of which is optionally substituted with one or more substituents selected from
   - halogen, hydroxy, cyano, amino or carboxy; or
   - C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylaminocarbonyl, di-(C₁₋₆-alkyl)aminocarbonyl, C₁₋₆-alkylamino, di-(C₁₋₆-alkyl)amino or C₃₋₆-cycloalkylamino, each of which is optionally substituted with one or more halogens, CN and OH; and
X₂ is aryl or heteroaryl each of which is optionally substituted with one or more substituents selected from
   - halogen, hydroxy, cyano, amino or carboxy; or
   - C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylaminocarbonyl, di-(C₁₋₆-alkyl)aminocarbonyl, C₁₋₆-alkylamino, di-(C₁₋₆-alkyl)amino or C₃₋₆-cycloalkylamino, each of which is optionally substituted with one or more halogens, CN and OH; or
X₂ is selected from
   - hydrogen, halogen, hydroxy, cyano, amino or carboxy; or
   - C₁₋₆-alkyl, C₃₋₈-_{G}ycloalkyl, C₂₋₆-alkenyl, C₂₋₆-alkynyl, aralkyl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylaminocarbonyl, di-(C₁₋₆-alkyl)-aminocarbonyl, C₁₋₆-alkylamino, di-(C₁₋₆-alkyl)amino or C₃₋₆-cycloalkylamino, each of which is optionally substituted with one or more halogens, CN and OH; and
X₃ is arylene or heteroarylene each of which is optionally substituted with one or more substituents selected from
   - halogen, hydroxy, cyano, amino or carboxy; or
   - C₁₋₆-alkyl, C₃₋₆-cycloalkyl, C₂₋₆-alkenyl, C₂₋₆-alkynyl, C₁₋₆-alkoxy, C₃₋₈-cycloalkoxy, C₁₋₆-alkylthio, C₃₋₆-cycloalkylthio, C₁₋₆-alkylamino, C₁₋₆-dialkylamino or C₃₋₆-cycloalkylamino, each of which is optionally substituted with one or more halogens; and
Ar is phenylene which is optionally substituted with one or more substituents selected from
   - halogen; or
   - C₁₋₆-alkyl, optionally substituted with one or more halogens; and
Y₁ is O or S; and
Y₂ is O or S; and
Z is -(CH₂)ₙ- wherein n is 1, 2 or 3; and
R₁ is hydrogen, halogen or a substituent selected from
   - C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio, each of which is optionally substituted with one or more halogens; and
R₂ is hydrogen, C₁₋₆-alkyl, C₃₋₆-cycloalkyl, C₂₋₆-alkenyl, C₂₋₆-alkynyl, C₄₋₆-alkenynyl or aryl; or
a pharmaceutically acceptable salt thereof, or a pharmaceutically acceptable solvate thereof, or any tautomeric forms, stereoisomers, mixture of stereoisomers including a racemic mixture, or polymorphs.

In one embodiment, the present invention is concerned with compounds of formula (I) wherein X₁ is aryl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

In another embodiment, the present invention is concerned with compounds of formula (I) wherein X₁ is aryl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

In another embodiment, the present invention is concerned with compounds of formula (I) wherein X₁ is aryl optionally substituted with one or more substituents selected from
- halogen; or
- C₁₋₆-alkyl, C₁₋₆-alkoxy or C₁₋₆-alkylthio, each of which is optionally substituted with one or more halogens.

In another embodiment, the present invention is concerned with compounds of formula (I) wherein X₁ is phenyl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

In another embodiment, the present invention is concerned with compounds of formula (I) wherein X₁ is phenyl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

In another embodiment, the present invention is concerned with compounds of formula (I) wherein X₁ is phenyl optionally substituted with one or more substituents selected from
- halogen; or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₁ is phenyl optionally substituted with C₁₋₆-alkoxy.

In another embodiment, the present invention is concerned with compounds of formula (I) wherein X₁ is phenyl.

In another embodiment, the present invention is concerned with compounds of formula (I) wherein X₁ is heteroaryl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

In another embodiment, the present invention is concerned with compounds of formula (I) wherein X₁ is heteroaryl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

In another embodiment, the present invention is concerned with compounds of formula (I) wherein X₁ is heteroaryl optionally substituted with one or more substituents selected from
- halogen; or
- C₁₋₆-alkyl, C₁₋₆-alkoxy or C₁₋₆-ailkylthio each of which is optionally substituted with one or more halogens.

In another embodiment, the present invention is concerned with compounds of formula (I) wherein X₁ is pyridyl, thienyl, furyl, thiazolyl, oxazolyl, benzofuranyl, benzothienyl or quinolinyl each of which is optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

In another embodiment, the present invention is concerned with compounds of formula (I) wherein X₁ is pyridyl, thienyl, furyl, thiazolyl, oxazolyl benzofuranyl, benzothienyl or quinolinyl each of which is optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

In another embodiment, the present invention is concerned with compounds of formula (I) wherein X₁ is pyridyl, thienyl, furyl, thiazolyl, oxazolyl benzofuranyl, benzothienyl, or quinolinyl each of which is optionally substituted with one or more substituents selected from
- halogen; or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₁ is C₁₋₆-alkyl optionally substituted with one or more substituents selected from
- halogen or hydroxy; or
- aryl, heteroaryl, heterocyclyl, C₁₋₆-alkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is C₁₋₆-alkyl optionally substituted with one or more substituents selected from
- halogen or hydroxy; or
- aryl, heteroaryl, C₁₋₆-alkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is C₁₋₆-alkyl optionally substituted with one or more substituents selected from
- halogen or hydroxy; or
- aryl, heteroaryl, heterocyclyl, C₁₋₆-alkoxy, C₁₋₆-alkylthio, C₁₋₆-alkylcarbonyl, C₁₋₆-alkylsulfonyl, C₁₋₆-alkylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is C₁₋₆-alkyl optionally substituted with one or more substituents selected from
- halogen or hydroxy; or
- aryl, heteroaryl, C₁₋₆-alkoxy, C₁₋₆-alkylthio, C₁₋₆-alkylcarbonyl, C₁₋₆-alkylsulfonyl, C₁₋₆-alkylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is C₁₋₆-alkyl optionally substituted with one or more substituents selected from halogen or hydroxy.

In another embodiment, the present invention is concerned with compounds of formula (I) wherein X₁ is C₁₋₆-alkyl substituted with hydroxy.

In another embodiment, the present invention is concerned with compounds of formula (I) wherein X₁ is C₁₋₆-alkyl substituted with heterocyclyl.

In another embodiment, the present invention is concerned with compounds of formula (I) wherein X₁ is C₁₋₆-alkyl substituted with morpholinyl.

In another embodiment, the present invention is concerned with compounds of formula (I) wherein X₁ is C₃₋₆-cycloalkyl optionally substituted with one or more substituents selected from
- halogen or hydroxy; or
- aryl, heteroaryl, heterocyclyl, C₁₋₆-alkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is C₃₋₆-cycloalkyl optionally substituted with one or more substituents selected from
- halogen or hydroxy; or
- aryl, heteroaryl, C₁₋₆-alkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is C₃₋₆-cycloalkyl optionally substituted with one or more substituents selected from
- halogen or hydroxy; or
- aryl, heteroaryl, heterocyclyl, C₁₋₆-alkoxy, C₁₋₆-alkylthio, C₁₋₆-alkylcarbonyl, C₁₋₆-alkylsulfonyl, C₁₋₆-alkylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is C₃₋₆-cycloalkyl optionally substituted with one or more substituents selected from
- halogen or hydroxy; or
- aryl, heteroaryl, C₁₋₆-alkoxy, C₁₋₆-alkylthio, C₁₋₆-alkylcarbonyl, C₁₋₆-alkylsulfonyl, C₁₋₆-alkylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is C₃₋₆-cycloalkyl optionally substituted with one or more substituents selected from halogen or hydroxy.

In another embodiment, the present invention is concerned with compounds of formula (I) wherein X₁ is aralkyl or heteroaralkyl each of which is optionally substituted with one or more substituents selected from
- halogen or hydroxy; or
- C₁₋₆-alkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

In another embodiment, the present invention is concerned with compounds of formula (I) wherein X₁ is aralkyl or heteroaralkyl each of which is optionally substituted with one or more substituents selected from
- halogen or hydroxy; or
- C₁₋₆-alkoxy which is optionally substituted with one or more halogens.

In another embodiment, the present invention is concerned with compounds of formula (I) wherein X₁ is benzyl which is optionally substituted with one or more substituents selected from
- halogen or hydroxy; or
- C₁₋₆-alkoxy which is optionally substituted with one or more halogens.

In another embodiment, the present invention is concerned with compounds of formula (I) wherein X₁ is benzyl.

In another embodiment, the present invention is concerned with compounds of formula (I) wherein X₂ is aryl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

In another embodiment, the present invention is concerned with compounds of formula (I) wherein X₂ is aryl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

In another embodiment, the present invention is concerned with compounds of formula (I) wherein X₂ is aryl optionally substituted with one or more substituents selected from
- halogen; or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₂ is phenyl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

In another embodiment, the present invention is concerned with compounds of formula (I) wherein X₂ is phenyl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

In another embodiment, the present invention is concerned with compounds of formula (I) wherein X₂ is phenyl optionally substituted with one or more substituents selected from
- halogen; or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₂ is phenyl.

In another embodiment, the present invention is concerned with compounds of formula (I) wherein X₂ is heteroaryl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

In another embodiment, the present invention is concerned with compounds of formula (I) wherein X₂ is heteroaryl optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

In another embodiment, the present invention is concerned with compounds of formula (I) wherein X₂ is heteroaryl optionally substituted with one or more substituents selected from
- halogen; or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₂ is pyridyl, thienyl, furyl, thiazolyl, oxazolyl, benzofuranyl, benzothienyl or quinolinyl each of which is optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

In another embodiment, the present invention is concerned with compounds of formula (I) wherein X₂ is pyridyl, thienyl, furyl, thiazolyl, oxazolyl, benzofuranyl, benzothienyl or quinolinyl each of which is optionally substituted with one or more substituents selected from
- halogen, hydroxy; or
- C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

In another embodiment, the present invention is concerned with compounds of formula (I) wherein X₂ is pyridyl, thienyl, furyl, thiazolyl, oxazolyl, benzofuranyl, benzothienyl or quinolinyl each of which is optionally substituted with one or more substituents selected from
- halogen; or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₂ is hydrogen, halogen, hydroxy or cyano; or
- C₁₋₆-alkyl, aralkyl or heteroaralkyl each of which is optionally substituted with one or more halogens, CN and OH.

In another embodiment, the present invention is concerned with compounds of formula (I) wherein X₂ is halogen or hydroxy; or
- C₁₋₆-alkyl optionally substituted with one or more halogens.

In another embodiment, the present invention is concerned with compounds of formula (I) wherein X₂ is halogen.

In another embodiment, the present invention is concerned with compounds of formula (I) wherein X₃ is arylene optionally substituted with one or more substituents selected from
- halogen or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₃ is phenylene optionally substituted with one or more substituents selected from
- halogen or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₃ is phenylene optionally substituted with one or more substituents selected from halogen or C₁₋₆-alkyl, which is optionally substituted with one or more halogens.

In another embodiment, the present invention is concerned with compounds of formula (I) wherein X₃ is phenylene.

In another embodiment, the present invention is concerned with compounds of formula (I) wherein X₃ is heteroarylene optionally substituted with one or more substituents selected from
- halogen or
- C₁₋₆-alkyl, C₁₋₆-alkoxy or C₁₋₆-alkylthio each of which is optionally substituted with one or more halogens.

In another embodiment, the present invention is concerned with compounds of formula (I) wherein X₃ is heteroarylene optionally substituted with one or more substituents selected from halogen or C₁₋₆-alkyl which is optionally substituted with one or more halogens.

In another embodiment, the present invention is concerned with compounds of formula (I) wherein X₃ is oxazolylene, benzofuranylene or benzothienylene.

In another embodiment, the present invention is concerned with compounds of formula (I) wherein X₃ is thienylene.

In another embodiment, the present invention is concerned with compounds of formula (I) wherein Ar is phenylene which is optionally substituted with methyl.

In another embodiment, the present invention is concerned with compounds of formula (I) wherein Y₁ is S.

In another embodiment, the present invention is concerned with compounds of formula (I) wherein Y₁ is O.

In another embodiment, the present invention is concerned with compounds of formula(I)wherein Y₂ is O.

In another embodiment, the present invention is concerned with compounds of formula(I)wherein Y₂ is S.

In another embodiment, the present invention is concerned with compounds of formula (I) wherein n is 1.

In another embodiment, the present invention is concerned with compounds of formula (I) wherein R₁ is hydrogen or a substituent selected from
- C₁₋₆-alkyl, aralkyl, C₁₋₆-alkoxy, aryloxy, aralkoxy each of which is optionally substituted with one or more halogens.

In another embodiment, the present invention is concerned with compounds of formula (I) wherein R₁ is hydrogen or a substituent selected from
- C₁₋₆-alkyl, C₁₋₆-alkoxy each of which is optionally substituted with one or more halogens.

In another embodiment, the present invention is concerned with compounds of formula (I) wherein R₁ is hydrogen.

In another embodiment, the present invention is concerned with compounds of formula (I) wherein R₁ is methyl or ethyl.

In another embodiment, the present invention is concerned with compounds of formula (I) wherein R₁ is methoxy or ethoxy.

In another embodiment, the present invention is concerned with compounds of formula (I) wherein R₂ is hydrogen or C₁₋₆-alkyl.

In another embodiment, the present invention is concerned with compounds of formula (I) wherein R₂ is hydrogen.

In another embodiment, the present invention is concerned with compounds of formula I wherein alkyl is methyl, ethyl or isopropyl.

In another embodiment, the present invention is concerned with compounds of formula I wherein alkenyl is vinyl or 1-propenyl.

In another embodiment, the present invention is concerned with compounds of formula I wherein alkynyl is 1-propynyl.

In another embodiment, the present invention is concerned with compounds of formula I wherein alkenynyl is 1-pentene-4-yne.

In another embodiment, the present invention is concerned with compounds of formula I wherein alkoxy is methoxy, ethoxy, isopropoxy or cyclopropoxy.

In another embodiment, the present invention is concerned with compounds of formula I wherein aryl is phenyl.

In another embodiment, the present invention is concerned with compounds of formula I wherein arylene is phenylene.

In another embodiment, the present invention is concerned with compounds of formula I wherein halogen is fluorine, bromine or chlorine.

In another embodiment, the present invention is concerned with compounds of formula i wherein perhalomethyl is trifluoromethyl.

In another embodiment, the present invention is concerned with compounds of formula I wherein perhalomethoxy is trifluoromethoxy,

In another embodiment, the present invention is concerned with compounds of formula I wherein heteroaryl is pyridyl, furyl, thienyl, benzofuryl, or benzothienyl, benzofuranyl or benzothienyl.

In another embodiment, the present invention is concerned with compounds of formula I wherein heteroaryl is benzofuryl.

In another embodiment, the present invention is concerned with compounds of formula I wherein aralkyl is benzyl.

In another embodiment, the present invention is concerned with compounds of formula I wherein aryloxy is phenoxy.

In another embodiment, the present invention is concerned with compounds of formula I wherein aralkoxy is benzyloxy.

In another embodiment, the present invention is concerned with compounds of formula I wherein the substituents R₁ and -X₂X₃ are arranged in a trans-configuration.

In another embodiment, the present invention is concerned with compounds of formula I wherein the substituents R₁ and -X₂X₃ are arranged in a cis-configuration.

In another embodiment, the present invention is concerned with compounds of formula I which are PPARδ agonists.

In another embodiment, the present invention is concerned with compounds of formula I which are selective PPARδ agonists.

Examples of specific compounds of the invention are:
(Z)-[4-[3-(4-Bromophenyl)-5-phenylpent-2-en-4-ynylsulfanyl]-2-methylphenoxy]acetic acid;
(Z)-[4-[3-(4-Bromophenyl)-6-hydroxyhex-2-en-4-ynylsulfanyl]-2-methylphenoxy]acetic acid;
[4-[3-(Biphenyl-4-yl)-6-hydroxyhex-2-en-4-ynylsulfanyl]-2-methylphenoxy]acetic acid;
(Z)-[4-[3-(Biphenyl-4-yl)-6-hydroxy-6-methylhept-2-en-4-ynylsulfanyl]-2-methylphenoxy]acetic acid;
(Z)-[4-[5-(4-Methoxyphenyl)-3-(biphenyl-4-yl)-pent-2-en-4-ynylsulfanyl]-2-methylphenoxy]-acetic acid;
(Z)-[4-[5-(4-methoxyphenyl)-3-(4-trifluoromethylphenyl)pent-2-en-4-ynylsulfanyl]-2-methyl-phenoxy]acetic acid; or
a salt thereof with a pharmaceutically acceptable acid or base, or any optical isomer or mixture of optical isomers, including a racemic mixture, or any tautomeric forms.

Other examples of specific compounds of the invention are:
(Z) [4-(3,6-Diphenyl-pent-2-en-4-ynyloxy)-2-methyl-phenoxy]-acetic acid
(Z)-[4-[3-(4-Bromophenyl)-5-phenylpent-2-en-4-ynyloxy]-2-methylphenoxy]acetic acid;
(Z)-[4-[3-(4-Bromophenyl)-5-(pyridin-2-yl)pent-2-en-4-ynyloxy]-2-methylphenoxy]acetic acid;
{4-[3-(4-Bromo-phenyl)-5-(5-methyl-thiophen-2-yl)-pent-2-en-4-ynyloxy]-2-methyl-phenoxy}-acetic acid;
{2-Methyl-4-[3-(2-methyl-benzofuran-5-yl)-5-(5-methyl-thiophen-2-yl)-pent-2-en-4-ynyloxy]-phenoxy}-acetic acid;
{4-[3-(4-Bromo-phenyl)-6-morpholin-4-yl-hex-2-en-4-ynyloxy]-2-methyl-phenoxy}-acetic acid; (2-Methyl-4-{3-[4-(5-methyl-thiophen-2-yl)-phenyl]-5-phenyl-pent-2-en-4-ynyloxy}-phenoxy)-acetic acid; or
a salt thereof with a pharmaceutically acceptable acid or base, or any optical isomer or mixture of optical isomers, including a racemic mixture, or any tautomeric forms.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2. Examples of metal salts include lithium, sodium, potassium, magnesium, zinc, calcium salts and the like. Examples of amines and organic amines include ammonium, methylamine, dimethylamine, trimethylamine, ethylamine, diethylamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-glucamine, guanidine and the like. Examples of cationic amino acids include lysine, arginine, histidine and the like.

The pharmaceutically acceptable salts are prepared by reacting the compound of formula I with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol etc. Mixture of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (*R*)- or (S)-phenylethylamine, cinchona alkaloids and their derivatives and the like. Commonly used methods are compiled by Jaques et al in "Enantiomers, Racemates and Resolution" (Wiley Interscience, 1981). More specifically the compound of formula I may be converted to a 1:1 mixture of diastereomeric amides by treating with chiral amines, aminoacids, aminoalcohols derived from aminoacids; conventional reaction conditions may be employed to convert acid into an amide; the dia-stereomers may be separated either by fractional crystallization or chromatography and the stereoisomers of compound of formula I may be prepared by hydrolysing the pure diastereomeric amide.

Various polymorphs of compound of general formula I forming part of this invention may be prepared by crystallization of compound of formula I under different conditions. For example, using different solvents commonly used or their mixtures for recrystallization; crystallizations at different temperatures; various modes of cooling, ranging from very fast to very slow cooling during crystallizations. Polymorphs may also be obtained by heating or melting the compound followed by gradual or fast cooling. The presence of polymorphs may be determined by solid probe nmr spectroscopy, ir spectroscopy, differential scanning calorimetry, powder X-ray diffraction or such other techniques.

This disclosure encompasses prodrugs of the present compounds, which on administration undergo chemical conversion by metabolic processes before becoming active pharmacological substances. In general, such prodrugs will be functional derivatives of the present compounds, which are readily convertible in vivo into the required compound of the formula (I). Conventional procedures for the selection and preparation of suitable prodrug derivatives are described, for example, in "Design of Prodrugs", ed. H. Bundgaard, Elsevier, 1985.

The disclosure also encompasses active metabolites of the present compounds.

The invention also relates to pharmaceutical compositions comprising, as an active ingredient, at least one compound of the formula I or any optical or geometric isomer or tautomeric form thereof including mixtures of these or a pharmaceutically acceptable salt thereof together with one or more pharmaceutically acceptable carriers or diluents.

Furthermore, the invention relates to the use of compounds of the general formula I or their tautomeric forms, their stereoisomers, their polymorphs, their pharmaceutically acceptable salts or pharmaceutically acceptable solvates thereof for the preparation of a pharmaceutical composition for the treatment and/or prevention of conditions mediated by nuclear receptors, in particular the Peroxisome Proliferator-Activated Receptors (PPAR) such as the conditions mentioned above.

In another aspect, the present invention relates to compounds for use in treating and/or preventing Type I or Type II diabetes.

In a still further aspect, the present invention relates to the use of one or more compounds of the general formula I or pharmaceutically acceptable salts thereof for the preparation of a pharmaceutical composition for the treatment and/or prevention of Type I or Type I diabetes.

In a still further aspect, the present compounds are useful for the treatment and/or prevention of IGT.

In a still further aspect, the present compounds are useful for the treatment and/or prevention of Type 2 diabetes.

In a still further aspect, the present compounds are useful for the delaying or prevention of the progression from IGT to Type 2 diabetes.

In a still further aspect, the present compounds are useful for the delaying or prevention of the progression from non-insulin requiring Type 2 diabetes to insulin requiring Type 2 diabetes.

In another aspect, the present compounds reduce blood glucose and triglyceride levels and are accordingly useful for the treatment and/or prevention of ailments and disorders such as diabetes and/or obesity.

In still another aspect, the present compounds are useful for the treatment and/or prophylaxis of insulin resistance (Type 2 diabetes), impaired glucose tolerance, dyslipidemia, disorders related to Syndrome X such as hypertension, obesity, insulin resistance, hyperglycaemia, hypertriglyceridemia atherosclerosis, artherosclerosis, hyperlipidemia, hypercholesterolemia coronary artery disease, myocardial ischemia and other cardiovascular disorders.

In still another aspect, the present compounds are useful for the treatment and/or prophylaxis of diseases or complications related to atherosclerosis such as coronary artery diseases, coronary heart diseases, heart attack, myocardial infarct, coronary infarct, transient ischemic attack (TIA) or stroke.

In still another aspect, the present compounds are effective in decreasing apoptosis in mammalian cells such as beta cells of Islets of Langerhans.

In still another aspect, the present compounds are useful for the treatment of certain renal diseases including glomerulonephritis, glomerulosclerosis, nephrotic syndrome, hypertensive nephrosclerosis.

In still another aspect, the present compounds may also be useful for improving cognitive functions in dementia, treating diabetic complications, psoriasis, polycystic ovarian syndrome (PCOS) and prevention and treatment of bone loss, e.g. osteoporosis.

In yet another aspect, the invention also relates to the use of the present compounds, which after administration lower the bio-markers of atherosclerosis like, but not limited to, c-reactive protein (CRP), TNFα and IL-6.

The present compounds may also be administered in combination with one or more further pharmacologically active substances eg., selected from antiobesity agents, antidiabetics, antihypertensive agents, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators or TRβ agonists.

In one embodiment of the invention the antiobesity agent is leptin.

In another embodiment the antiobesity agent is dexamphetamine or amphetamine.

In another embodiment the antiobesity agent is fenfluramine or dexfenfluramine.

In still another embodiment the antiobesity agent is sibutramine.

In a further embodiment the antiobesity agent is orlistat.

In another embodiment the antiobesity agent is mazindol or phentermine.

Suitable antidiabetics comprise insulin, GLP-1 (glucagon like peptide-1) derivatives such as those disclosed in WO 98/08871 to Novo Nordisk A/S as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as HMG CoA inhibitors (statins), compounds lowering food intake, RXR agonists and agents acting on the ATP-dependent potassium channel of the β-cells.

In one embodiment of the invention the present compounds are administered in combination with insulin.

In a further embodiment the present compounds are administered in combination with a sulphonylurea eg. tolbutamide, glibenclamide, glipizide or glicazide.

In another embodiment the present compounds are administered in combination with a biguanide eg. metformin.

In yet another embodiment the present compounds are administered in combination with a meglitinide eg. repaglinide or senaglinide.

In a further embodiment the present compounds are administered in combination with an α-glucosidase inhibitor eg. miglitol or acarbose.

In another embodiment the present compounds are administered in combination with an agent acting on the ATP-dependent potassium channel of the β-cells eg. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent eg. cholestyramine, colestipol, clofibrate, gemfibrozil, fenofibrate, bezafibrate, tesaglitazar, EML-4156, LY-518674, LY-519818, MK-767,atorvastatin, fluvastin, lovastatin, pravastatin, simvastatin, cerivastin, acipimox, ezetimibe, probucol, dextrothyroxine or nicotinic acid.

In yet another embodiment the present compounds are administered in combination with a thiazolidinedione e.g. troglitazone, ciglitazone, pioglitazone or rosiglitazone.

In a further embodiment the present compounds are administered in combination with more than one of the above-mentioned compounds eg. in combination with a sulphonylurea and metformin, a sulphonylurea and acarbose, repaglinide and metformin, insulin and a sulphonylurea, insulin and metformin, insulin, insulin and lovastatin, etc.

Furthermore, the present compounds may be administered in combination with one or more antihypertensive agents. Examples of antihypertensive agents are β-blockers such as alprenolol, atenolol, timolol, pindolol, propranolol and metoprolol, ACE (angiotensin converting enzyme) inhibitors such as benazepril, captopril, enalapril, fosinopril, lisinopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin. Further reference can be made to Remington: The Science and Practice of Pharmacy, 19th Edition, Gennaro, Ed., Mack Publishing Co., Easton, PA, 1995.

It should be understood that any suitable combination of the compounds according to the invention with one or more of the above-mentioned compounds and optionally one or more further pharmacologically active substances are considered to be within the scope of the present invention.

The present invention also relates to a process for the preparation of the above said novel compounds, their derivatives, their analogs, their tautomeric forms, their stereoisomers, their polymorphs, their pharmaceutically acceptable salts or pharmaceutically acceptable solvates.

### PHARMACEUTICAL COMPOSITIONS

The compounds of the invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995. The compositions may appear in conventional forms, for example capsules, tablets, aerosols, solutions, suspensions or topical applications.

Typical compositions include a compound of formula I or a pharmaceutically acceptable acid addition salt thereof, associated with a pharmaceutically acceptable excipient which may be a carrier or a diluent or be diluted by a carrier, or enclosed within a carrier which can be in the form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier which may be in the form of an ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, peanut oil, olive oil, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art.

The pharmaceutical compositions can be sterilized and mixed, if desired, with auxiliary agents, emulsifiers, salt for influencing osmotic pressure, buffers and/or colouring substances and the like, which do not deleteriously react with the active compounds.

The route of administration may be any route, which effectively transports the active compound to the appropriate or desired site of action, such as oral, nasal, pulmonary, transdermal or parenteral e.g. rectal, depot, subcutaneous, intravenous, intraurethral, intramuscular, intranasal, ophthalmic solution or an ointment, the oral route being preferred.

If a solid carrier is used for oral administration, the preparation may be tabletted, placed in a hard gelatin capsule in powder or pellet form or it can be in the form of a troche or lozenge. If a liquid carrier is used, the preparation may be in the form of a syrup, emulsion, soft gelatin capsule or sterile injectable liquid such as an aqueous or non-aqueous liquid suspension or solution.

For nasal administration, the preparation may contain a compound of formula I dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

A typical tablet which may be prepared by conventional tabletting techniques may contain:

| Core: | |
|---|---|
| Active compound (as free compound or salt thereof) | 5 mg |
| Colloidal silicon dioxide (Aerosil) | 1.5 mg |
| Cellulose, microcryst. (Avicel) | 70 mg |
| Modified cellulose gum (Ac-Di-Sol) | 7.5 mg |
| Magnesium stearate | Ad. |

| Coating: | |
|---|---|
| HPMC approx. | 9 mg |
| *Mywacett 9-40 T approx. | 0.9 mg |

| | |
|---|---|
| *Acylated monoglyceride used as plasticizer for film coating. | |

If desired, the pharmaceutical composition of the invention may comprise the compound of formula (I) in combination with further pharmacologically active substances such as those described in the foregoing.

The compounds of the invention may be administered to a mammal, especially a human in need of such treatment, prevention, elimination, alleviation or amelioration of diseases related to the regulation of blood sugar.

Such mammals include also animals, both domestic animals, e.g. household pets, and non-domestic animals such as wildlife.

The compounds of the invention are effective over a wide dosage range. A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain of from 0.05 to about 1000 mg, preferably from about 0.1 to about 500 mg, and more preferred from about 0.5 mg to about 200 mg.

Any novel feature or combination of features described herein is considered essential to this invention.

### EXAMPLES

The following examples and general procedures refer to intermediate compounds and final products identified in the specification and in the synthesis schemes. The preparation of the compounds of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed nuclear magnetic resonance (NMR). NMR shifts (δ) are given in parts per million (ppm. Mp is melting point and is given in °C.

The abbreviations as used in the examples have the following meaning:
- THF:: tetrahydrofuran
- DMSO:: dimethylsulfoxide
- CDCl₃:: deutorated chloroform
- DMF:: N,N-dimethylformamide
- min:: minutes
- h:: hours

### General procedure (A)

### Step A:

Reaction a compound of formula (II), 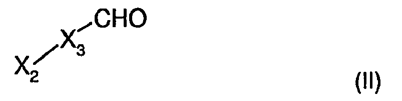 wherein X₂ and X₃ are defined as above, with carbon tetrabromide and triphenylphosphine to give a compound of formula (III) 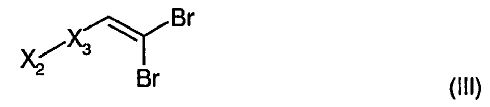 wherein X₂ and X₃ are defined as above.

### Step B:

Reaction the compound of formula (III), wherein X₂ and X₃ are defined as above, with paraformaldehyde in the presence of a strong base like BuLi, to give a compound of formula (IV), 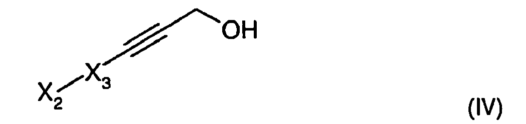 wherein X₂ and X₃ are defined as above.

### Step C:

Reducing a compound of formula (IV), wherein X₂ and X₃ are defined as above, with LiAIH in the presence of a base, like sodium methoxide, followed by treatment with dimethylcarbonate and iodine to give a compound of formula (V), 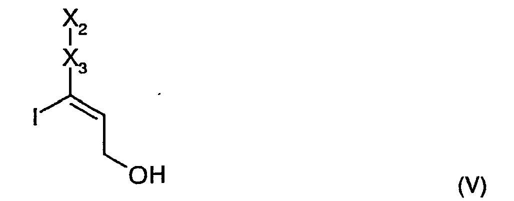 wherein X₂ and X₃ are defined as above.

### Step D:

Converting the hydroxyl function in compound (V) wherein X₂ and X₃ are defined as above, to a leaving group (L), by treating (V) with tetrabromemethane and triphenylphosphine, or methanesulfonyl chloride and base or the like, to a compound of formula (VI) 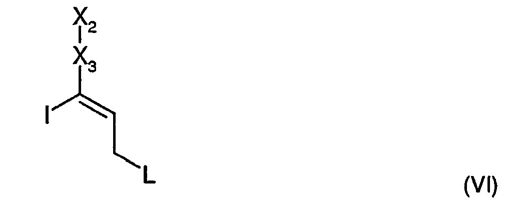 wherein X₂ and X₃ are defined as above, and L is a leaving group, such as p-toluenesulfonate, methanesulfonate, halogen, triflate and the like.

### Step E:

Reacting the compound of formula (VI), wherein L, X₂ and X₃ are defined as above, with a compound of formula (VII) 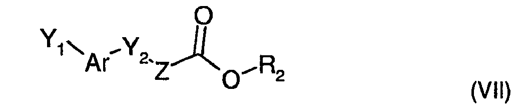 wherein Y₁, Ar, Y₂, Z and R₂ are defined as above, except that R₂ is not hydrogen, to give a compound of formula (VIII) 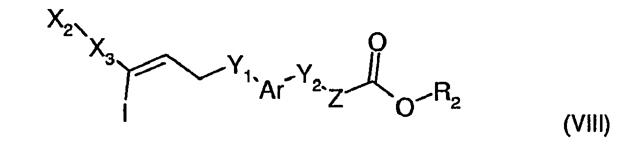 wherein X₂, X₃, Y₁, Y₂, Ar, Z and R₂ are defined as above, except that R₂ is not hydrogen.

### Step F:

Reacting a compound of formula (VIII), wherein X₂, X₃, Y₁, Y₂, Ar, Z and R₂ are defined as above, except that R₂ is not hydrogen, under Heck like conditions with X₁-acetylene in the presence of a palladium catalyst, like Pd₂(dba)₃, cobber (I) and base, like iPr₂NH, to give a compound of formula (I), wherein X₁, X₂, X₃, Y₁, Y₂, Ar, Z, R₁ and R₂ are defined as above, except that R₁ is hydrogen and R₂ is not hydrogen.

### General procedure (B)

### Step A:

Reacting a compound of formula (IX), 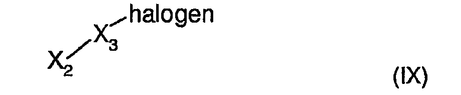 wherein X₂, X₃ and halogen are defined as above, under Heck like conditions with propargylalcohol in the presence of a palladium catalyst, like Pd₂(dba)₃ and cobber (I) to give a compound of formula (IV) wherein X₂ and X₃, are defined as above.

### General procedure (C)

### Step A:

Reaction a compound of formula (V) ,wherein X₂ and X₃ are defined as above, under Heck like conditions with X₁-acetylene in the presence of a palladium catalyst, like Pd₂(dba)₃, cobber (I) and base, like iPr₂NH, to give a compound of formula (X), 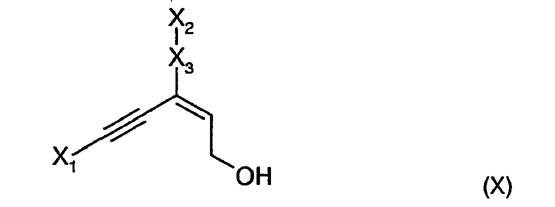 wherein X₁, X₂ and X₃, are defined as above.

### Step B:

Converting the hydroxyl function in the compound of formula (X), wherein X₁, X₂ and X₃, are defined as above, to a leaving group (L), by treating with tetrabromemethane and triphenylphosphine, or methanesulfonyl chloride and base or the like, to give a compound of formula (XI) 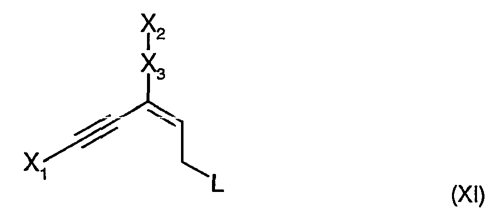 wherein L, X₁, X₂ and X₃ are defined as above.

### Step C:

Reacting the compound of formula (XI), wherein L, X₁, X₂ and X₃ are defined as above, with a compound of formula (VII), wherein Y₁, Ar, Y₂, Z and R₂ are defined as above, except that R₂ is not hydrogen, to give a compound of formula (I), wherein X₁, X₂, X₃, Y₁, Y₂, Ar, Z, R₁ and R₂ are defined as above, except that R₁ is hydrogen and R₂ is not hydrogen.

### General procedure (D)

### Step A:

By chemical or enzymatic saponification of a compound of formula I, wherein X₁, X₂, X₃, X₄, Y₁, Y₂, Ar, Z, R₁ and R₂ are defined as above, except that R₂ is not hydrogen, to give a compound of formula I, wherein X₁, X₂, X₃, X₄, Y₁, Y₂, Ar, Z, R₁ and R₂ are defined as above, except that R₂ is hydrogen.

### Example 1

### (Z)-[4-[3-(4-Bromophenyl)-5-phenylpent-2-en-4-ynylsulfanyl]-2-methylphenoxy]acetic acid

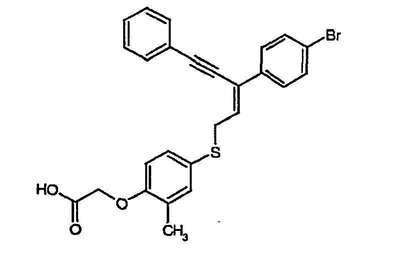

### General procedure (A)

### Step A:

### 1-Bromo-4-(2,2-dibromovinyl)benzene:

Tetrabromomethane (21.5 g, 65.9 mmol) was added to a cooled solution of 4-bromobenzaldehyde (10.0 g, 54.0 mmol) and triphenylphosphine (30.0 g, 130 mmol) in dry methylene chloride (100 mL). Reaction mixture was stirred for 3 h at room temperature. Subsequently, a saturated solution of sodium hydrogencarbonate (50 mL) was added and the organic layer was washed with water (150 mL), dried with anhydrous magnesium sulfate and evaporated in vacuo. Triphenylphosphine oxide was removed from the residue by crystallization from ethyl acetate and hexane. Evaporation of the mother liquor gave 18.4 g of an yellowish oil.
Crude yield: 18.4 g (85%).
R_{F} (SiO₂, hexane) = 0.70.

### Step B:

### 3-(4-Bromophenyl)prop-2-yn-1-ol:

1-Bromo-4-(2,2-dibromovinyl)benzene (8.0 g, 23 mmol) was dissolved in dry tetrahydrofuran (120 mL) and cooled to -78°C under inert atmosphere. 2M Solution of lithium diisopropylamide in tetrahydrofuran (38 mL, 75 mmol) was added dropwise to the reaction mixture and it was stirred for 2 h under cooling. Finely powdered paraformaldehyde (7.0 g, 230 mmol) was added to the mixture and it was stirred for further 3 h warming up the reaction mixture slowly to the room temperature. Brine (50 mL) was added and the mixture was extracted with ether (4x50 mL). The collected organic layers were dried with anhydrous magnesium sulfate and subsequently evaporated in vacuo. The residue was pre-purified by column chromatography (silica gel Fluka 60, hexane/ethyl acetate 1:0 - 3:1) and the obtained crude product was further purified by crystallization from ethyl acetate and hexane yielding 3.0 g of the desired product.
Yield: 3.0 g (66%).
R_{F} = 0.10 (SiO₂, hexane/ethyl acetate 9:1).
¹H NMR spectrum (250 MHz, CDCl₃, δ_{H}): 7.24 - 7.49 (m, 4 H); 4.48 (s, 2 H).

### Step C:

### (Z)-3-(4-Bromophenyl)-3-iodoprop-2-en-1-ol:

A solution of 3-(4-bromophenyl)prop-2-yn-1-ol (2.0 g, 10 mmol) in dry tetrahydrofuran (25 mL) was added dropwise to an ice-cooled solution of lithium aluminum hydride (600 mg, 15 mmol) and sodium methoxide (2 mg, 0.5%) in dry tetrahydrofuran (10 mL). Reaction mixture was stirred for 3 h under nitrogen atmosphere, a solution of dimethyl carbonate (1.2 g, 20 mmol) in dry tetrahydrofuran (10 mL) was added dropwise at 0 °C and the reaction mixture was stirred for further 1 h. Subsequently, a solution of iodine (5.0 g, 20 mmol) in tetrahydrofuran (20 mL) was added and the mixture was allowed to stand overnight in a fridge. Methanol (10mL) was added and the reaction mixture was stirred for further 0.5 h. A saturated solution of sodium thiosulfate (50 mL) and subsequently brine (150 mL) were added and it was extracted with ether (4x150 mL). The collected organic solutions were dried with anhydrous magnesium sulfate and subsequently evaporated in vacuo. The residue was purified by column chromatography (silica gel Fluka 60, hexane/methylene chloride 9:1 - methylene chloride - methylene chloride/methanol 3:1) yielding 2.7 g of the product.
Yeld: 2.7g (84%).
R_{F} = 0.50 (SiO₂, hexane/ethyl acetate 8:2).

### General procedure (C)

### Step A:

### (Z)-3-(4-Bromophenyl)-5-phenylpent-2-en-4-yn-1-ol:

Copper(I) iodide (3.8 mg, 20 µmol) and bis(triphenylphosphine)palladium(II) dichloride (31 mg, 30 µmol) were added to a solution of phenylacetylene (120 µl, 1.1 mmol), (Z)-3-(4-Bromophenyl)-3-iodoprop-2-en-1-ol (338 mg, 1 mmol) and diisopropylamine (170 µl, 1.2 mmol) in dry tetrahydrofuran (5 mL). The reaction flask was washed with nitrogen and the reaction mixture was quickly cooled to 0 °C. The mixture was stirred for 5 h under cooling and subsequently overnight at room temperature. The reaction mixture was diluted with ethyl acetate (10 mL) and filtered through a short path of silica gel. The filtrate was evaporated in vacuo and the residue was purified by column chromatography (silica gel Fluka 60, hexane/ethyl acetate 9:1) yielding 258 mg of the alkyne.
Yield: 258 mg (82%).
R_{F} = 0.35 (SiO₂, hexane/ethyl acetate 75:30).

### Step B-C:

### Ethyl (Z)-[4-[3-(4-bromophenyl)-5-phenylpent-2-en-4-ynylsulfanyl]-2-methylphenoxy]acetate:

A solution of tetrabromomethane (215 mg, 0.65 mmol) in dry methylene chloride (1.5 mL) was added dropwise to an ice-cooled solution of (Z)-3-(4-bromophenyl)-5-phenylpent-2-en-4-yn-1-ol (156 mg, 0.5 mmol) and triphenylphosphine (170 mg, 0.65 mmol) in dry methylene chloride (1 mL). The reaction mixture was stirred at room temperature overnight and the solvent was evaporated in vacuo. In atmosphere of nitrogen, N,N-diisopropylethylamine (0.52 mL, 3 mmol), water (0.2 mL, 11 mmol) and a solution of ethyl (4-mercapto-2-methylphenoxy)acetate (Bioorg. Med. Chem. Lett. 2003, 13, 1517) (158 mg, 0.7 mmol) in dry tetrahydrofuran (3 mL) were added to the residue and the resulting reaction mixture was stirred for 3 h. The mixture was filtered through a short path of silica gel and the filtrate was evaporated in vacuo. The residue was purified by column chromatography (silica gel Fluka 60, hexane/ethyl acetate 1:0 - 9:1) yielding 164 mg of the desired ester.
Yield: 164 mg (65%).
R_{F} = 0.35 (SiO₂, hexane/ethyl acetate 8:2).

### General procedure (D)

### Step A:

### (Z)-[4-[3-(4-bromophenyl)-5-phenylpent-2-en-4-ynylsulfanyl]-2-methylphenoxy]acetic acid:

In atmosphere of nitrogen, lithium hydroxide monohydrate (17 mg, 0.40 mmol) was added to a solution of ethyl (Z)-[4-[3-(4-bromophenyl)-5-phenylpent-2-en-4-ynylsulfanyl]-2-methylphenoxy]acetate (159 mg, 0.312 mmol) in a mixture of tetrahydrofuran, methanol, water (5:1:1, 5 mL) maintaining the reaction temperature below 5 °C. The formed solution was stirred for 2 h under cooling. A diluted aqueous solution of tartaric acid (5 mL) was added and the liberated acid was extracted with ether (4x25 mL). The collected organic solutions were dried with anhydrous magnesium sulfate and subsequently evaporated in vacuo. The residue was purified by column chromatography (silica gel Fluka 60, methylene chloride/- methanol 1:0 - 3:1) yielding 81 mg of the acid.
Yield: 81 mg (54%).
R_{F} = 0.33 (SiO₂, methylene chloride /methanol 9:1).

The above acid (81 mg, 0.17 mmol) was dissolved in a minimal amount of dry methanol (about 2 mL) and L-lysine (25 mg, 0.170 mmol) was added. The reaction mixture was stirred at room temperature for 90 min and acetonitrile (50 mL) was added. Separated solid mass was filtered off and dried in vacuo giving 85 mg of L-lysinate of the title acid. Yield: 85 mg (80 %).
¹H NMR spectrum (300 MHz, CDCl₃, δ_{H}): 7.56 (s, 4 H); 7.49-7.52 (m, 2 H); 7.40-7.56 (m, 2 H); 7.20-7.22 (m, 1 H); 7.11-7.14 (m, 1 H); 6.65 (t, J=8.0 Hz, 1 H); 6.58 (d, J=8.5 Hz, 1 H); 4.12 (s, 2 H); 3.92 (d, J=8.0 Hz, 2 H); 2.70-2.77 (m, 2 H); 2.00 (s, 3 H).

### Example 2

### (Z)-[4-[3-(4-Bromophenyl)-6-hydroxyhex-2-en-4-ynylsulfanyl]-2-methylphenoxy]acetic acid

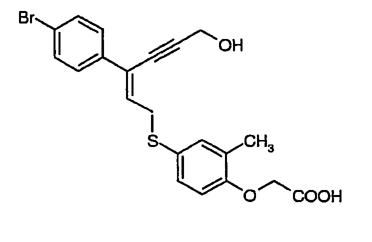

### General procedure (A)

### Step D-E:

### Ethyl (Z)-[4-[3-(4-Bromophenyl)-3-iodoallylsulfanyl]-2-methylphenoxy]acetate:

A solution of tetrabromomethane (2.1 g, 6.6 mmol) in dry methylene chloride (20 mL) was added dropwise to an ice-cooled solution of 3-(4-bromophenyl)-3-iodoprop-2-en-1-ol (1.5 g, 4.4 mmol; example 1) and triphenylphosphine (2.4 g, 9.0 mmol) in dry methylene chloride (50 mL). The reaction mixture was stirred at room temperature for 2 h and the solvent was evaporated in vacuo. Under nitrogen atmosphere, N,N-diisopropylethylamine (1.2 g, 9.0 mmol) and ethyl (4-mercapto-2-methylphenoxy)acetate (Bioorg. Med. Chem. Lett. 2003, 13, 1517) (1.5 g, 6.6 mmol) were added to the residue. The reaction mixture was stirred for 3 h, filtered through a short path of silica gel and the filtrate was evaporated in vacuo. The residue was purified by column chromatography (silica gel Merck 60, hexane /ethyl acetate 1:0 - 9:1) giving 0.80 g of the ester.
Yield: 0.80 g (40%).
R_{F} =0.55 (SiO₂, hexane/ethyl acetate 8:2).

### Step F:

### Ethyl (Z)-[4-[3-(4-Bromophenyl)-6-hydroxyhex-2-en-4-ynylsulfanyl]-2-methylphenoxy]acetate:

A solution of propargyl alcohol (38 µl, 0.66 mmol), diisopropylamine (102 µl, 0.72 mmol) and Ethyl (Z)-[4-[3-(4-bromophenyl)-3-iodoallylsulfanyl]-2-methylphenoxy]acetate (327 mg, 0.60 mmol) in dry tetrahydrofuran (3 mL) was washed by nitrogen and cooled to 0 °C. Subsequently, copper(I) iodide (2.3 mg, 2%) and bis(triphenylphosphine)palladium(II) dichloride (12 mg, 18%) were added to the reaction mixture and it was stirred overnight slowly allowing to reach the room temperature. A further portion of propargyl alcohol (38 µl, 0.66 mmol) was added; the mixture was heated at 60°C for 4 h and then stirred at room temperature for two days. The third portion of propargyl alcohol (38 µl, 0.66 mmol) and bis(triphenylphosphine)palladium(II) dichloride (4 mg) was added and mixture was stirred for 4 h at room temperature. The reaction mixture was evaporated in vacuo and the residue was purified by column chromatography (silica gel Merck 60, hexane/ethyl acetate 1:0 - 7:3) giving 127 mg of the hydroxy derivative.
Yield: 127 mg (47%).
R_{F} = 0.45 (SiO₂, hexane/ethyl acetate 75:25).

### General procedure (D)

### Step A:

### (Z)-[4-[3-(4-Bromophenyl)-6-hydroxyhex-2-en-4-ynylsulfanyl]-2-methylphenoxy]acetic acid:

In atmosphere of nitrogen, lithium hydroxide monohydrate (17 mg, 0.40 mmol) was added to a solution of Ethyl (Z)-[4-[3-(4-bromophenyl)-6-hydroxyhex-2-en-4-ynylsulfanyl]-2-methylphenoxy]acetate (127 mg, 0.27 mmol) in a mixture of tetrahydrofuran, methanol, water (5:1:1, 5 mL) maintaining the reaction temperature below 5 °C. The solution was stirred for 2 h, a diluted aqueous solution of tartaric acid (5 mL) was added and the liberated acid was extracted with ether (4x25 mL). The collected organic solutions were dried with anhydrous magnesium sulfate and subsequently evaporated in vacuo. The residue was purified by column chromatography (silica gel Merck 60, methylene chloride/methanol 1:0 - 4:1) giving 80 mg of the acid.
Yield: 80 mg (66%).
R_{F} = 0.20 (SiO₂, methylene chloride/methanol 4:1).

The above acid (80 mg, 0.180 mmol) was dissolved in a minimal amount of dry methanol (about 2 mL) and L-lysine (26 mg, 0.180 mmol) was added. The reaction mixture was stirred at room temperature for 90 min and acetonitrile (50 mL) was added. The separated solid mass was filtered off and dried in vacuo yielding 75 mg of the L-lysinate of the title acid.
Yield: 75 mg (75%).
¹H NMR spectrum (300 MHz, CDCl₃, δ_{H}): 7.45-7.55 (m, 4 H); 7.11-7.20 (m, 2 H); 7.62 (d, J=8.5 Hz, 1 H); 6.50 (s, J=8.2 Hz, 1 H); 4.24 (s, 2 H); 4.20 (d, 2 H); 3.2 (d, J=7.7 Hz, 2 H); 2.65-2.75 (m, 2 H); 2.08 (s, 3 H).

### Example 3

### [4-[3-(Biphenyl-4-yl)-6-hydroxyhex-2-en-4-ynylsulfanyl]-2-methylphenoxy]acetic acid

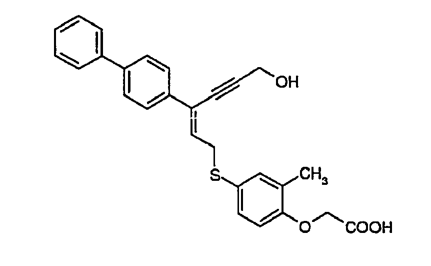

### General procedure (A)

### Step A:

### 1,1-Dibromo-2-(biphenyl-4-yl)ethane:

Tetrabromomethane (21.8 g, 166 mmol) was added to a cooled solution of biphenyl-4-carbaldehyde (10.0 g, 54.9 mmol) and triphenylphosphine (35.5 g, 132 mmol) in dry methylene chloride (100 mL). Reaction mixture was stirred for 3 h at room temperature and saturated solution of sodium hydrogencarbonate (50 mL) was added. The organic layer was washed with water (50 mL), dried with anhydrous magnesium sulfate and subsequently evaporated in vacuo. The crude product was twice re-crystallized from methanol giving 14.9 g of a white solid.
Yield: 14.9 g (80%).
R_{F} = 0.80 (SiO₂, hexane).

### Step B:

### 3-(Biphenyl-4-yl)-prop-2-yn-1-ol:

1,1-Dibromo-2-(biphenyl-4-yl)ethene (3.0 g, 8.9 mmol) was dissolved in dry tetrahydrofuran (100 mL) and under inert atmosphere cooled to -78°C. 2M Solution of n-butyllithium (12 mL, 22 mmol) was added dropwise to the solution and the reaction mixture was stirred for 2 h under cooling. Finely powdered paraformaldehyde (0.7 g, 22 mmol) was added to the mixture and it was stirred for 3h at -60°C slowly increasing the reaction temperature to room temperature. Brine (50 mL) was added and reaction mixture was extracted with ether (4x50 mL). The collected organic layers were dried with anhydrous magnesium sulfate and subsequently evaporated in vacuo. The residue was purified by column chromatography (silica gel Merck 60, hexane /ethyl acetate 1:0 - 3:1) yielding 4.1 g of the alkyne.
Yield: 4.1 g (74%).
R_{F} = 0.45 (SiO₂, hexane/ethyl acetate 4:1).
¹H NMR spectrum (250 MHz, CDCl₃, δ_{H}): 4.52 (s, 2 H); 7.30-7.63 (m, 9 H).

### Step C:

### (Z)-3-(Biphenyl-4-yl)-3-iodoprop-2-en-1-ol:

3-(Biphenyl-4-yl)-prop-2-yn-1-ol (1.0 g, 4.8 mmol) in dry tetrahydrofuran (20 mL) was added dropwise to an ice-cooled solution of lithium aluminum hydride (380 mg, 10 mmol) and sodium methoxide (10 mg, 250 µmol, 5%) in dry tetrahydrofuran (10 mL) under inert atmosphere. The reaction mixture was stirred for 3 h, dimethyl carbonate (900 mg, 10 mmol) in dry tetrahydrofuran (10 mL) was added dropwise at 0°C and the reaction mixture was stirred for further 2 h. A solution of iodine (2.5 g, 10 mmol) in tetrahydrofuran (10 mL) was added and the resulting mixture was allowed to stand overnight at 5oC. Methanol (5 mL) was added and reaction mixture was stirred for 0.5 h. Saturated aqueous solution of sodium thiosulfate (25 mL) and brine (100 mL) were added and the heterogenous mixture was extracted with ether (4x100 mL). The collected organic solutions were dried with anhydrous magnesium sulfate and subsequently evaporated in vacuo. The residue was purified by column chromatography (silica gel Merck 60, hexane/methylene chloride 9:1 - methylene chloride/methanol 3:1)
yielding 1.3 g of the hydroxy derivative.
Yield: 1.3 g (80%).
R_{F} = 0.50 (SiO₂, hexane/ethyl acetate 4:1).

### Step D-E:

### Ethyl (Z)-[4-[3-(Biphenyl-4-yl)-3-iodoallylsulfanyl]-2-methylphenoxy]acetate:

A solution of tetrabromethane (1.0 g, 3.0 mmol) in dry methylene chloride (20 mL) was added dropwise to an ice-cooled solution of (Z)-3-(biphenyl-4-yl)-3-iodoprop-2-en-1-ol (0.6 g, 2.0 mmol) and triphenylphosphine (0.8 g, 3.0 mmol) in dry methylene chloride (50 mL). The reaction mixture was stirred for 2 h at room temperature, N,N-diisopropylethylamine (250 mg, 2 mmol) and a drop of water were added, the solution was stirred for further 0.5 h and evaporated in vacuo. In atmosphere of nitrogen, tetrahydrofuran (25 mL), N,N-diisopropylethylamine (390 µL, 3.0 mmol) and ethyl (4-mercapto-2-methylphenoxy)acetate (560 mg, 2.5 mmol) were added to the residue. The reaction mixture was stirred overnight, filtered through a short path of silica gel and the filtrate was evaporated in vacuo. The residue was purified by column chromatography (silica gel Merck 60, hexane/ethyl acetate 1:0 - 9:1) yielding 0.5 g of the ester.
Yield: 0.5 g (50%).
R_{F}= 0.50 (SiO₂, hexane/ethyl acetate 4:1).

### Step F:

### Ethyl (Z)-[4-[3-(Biphenyl-4-yl)-6-hydroxyhex-2-en-4-ynylsulfanyl]-2-methylphenoxy]acetate:

Copper(I) iodide (2.7 mg, 14 µmol, 2%), bis(benzonitile)palladium(II) dichloride (8.1 mg, 21 µmol, 3%) and tri-tert-butylphosphine (21 µL, 42 µmol, 6%) were added to a solution of ethyl (Z)-[4-[3-(biphenyl-4-yl)-3-iodoallylsulfanyl]-2-methylphenoxy]acetate (400 mg, 0.73 mmol), propargyl alcohol (62 mg, 1.1 mmol) and N,N-diisopropylamine (141 mg, 1.4 mmol) in dry tetrahydrofuran (15 mL) under inert atmosphere. The reaction mixture was stirred at room temperature for 2 h. Suspension was filtered through a short path of silica gel, evaporated and purified by column chromatography (silica gel Merck 60, methylene chloride/methanol 1:0 - 8:2) yielding 150 mg of the ester.
Yield: 150 mg (50 %).
R_{F} = 0.65 (SiO₂, chloroform/methanol 98:2).

### General procedure (D)

### Step A:

### (Z)-[4-[3-(Biphenyl-4-yl)-6-hydroxyhex-2-en-4-ynylsulfanyl]-2-methylphenoxy]acetic acid:

A solution of lithium hydroxide in water (2 eq) was added to a solution of ethyl (Z)-[4-[3-(biphenyl-4-yl)-6-hydroxyhex-2-en-4-ynylsulfanyl]-2-methylphenoxy]acetate (150 mg) in a mixture of tetrahydofuran, methanol and water (4:1:1, 3 mL) and stirred at 0°C for 1 h. Water solution of tartaric acid was added to the mixture and the mixture was extracted with ether. The organic layer was dried, evaporated and purified by column chromatography (silica gel Merck 60, chloroform/methanol 8:2).
R_{F} =0.15 (SiO₂: chloroform/methanol 8:2).

L-lysine was added to the solution of the above acid in methanol. Acetonitrile was added after 1 h, white crystals of the L-lysinate of the title acid were filtered and dried.
Yield: 90 mg (50%).
¹H NMR spectrum (300 MHz, CDCl₃, δ_{H}): 6.55 (t, J=7.7 Hz, 1 H); 3.86 (d, J=7.7 Hz, 2 H); 4.17 (s, 2 H); 2.11 (s, 3 H); 4.29 (s, 2 H); 6.63 (d, J=8.1 Hz, 1 H); 7.06-7.58 (m, 14 H); 2.72 (s, 3 H); 3.13 (s, 1.6 H); 1.3-1.76 (m, 7 H).

### Example 4

### (Z)-[4-[3-(Biphenyl-4-yl)-6-hydroxy-6-methylhept-2-en-4-ynylsulfanyl]-2-methylphenoxy]acetic acid

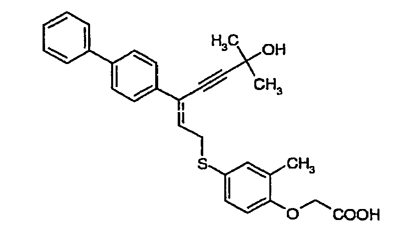

### General procedure (A)

### Step F:

### Ethyl (Z)-[4-[3-(Biphenyl-4-yl)-6-hydroxy-6-methylhept-2-en-4-ynylsulfanyl]-2-methyl-phenoxy]acetate:

Copper(I) iodide (6 mg, 30 µmol, 2%) and bis(triphenylphosphine)palladium(II) dichloride (31 mg, 45 µmol, 3%) were added to a solution of ethyl (Z)-[4-[3-(biphenyl-4-yl)-3-iodoallylsulfanyl]-2-methylphenoxy]acetate (800 mg, 1.5 mmol, example 3), 2-methyl-3-butyn-2-ol (252 mg, 3 mmol) and diisopropylamine (300 mg, 3 mmol, 2 eq) in dry tetrahydrofuran (20 mL) under inert atmosphere. The reaction mixture was stirred at room temperature for 2 h. Suspension was filtered through a short path of silica gel, evaporated and purified by column chromatography (silica gel Merck 60, hexane/ethyl acetate 1:0 - 6:4) yielding 600 mg of the ester.
Yield: 600 mg (80%).
R_{F} = 0.30 (SiO₂, hexane/ethyl acetate 8:2).
¹H NMR spectrum (300 MHz, CDCl₃, δ_{H}): 6.44 (t, J=7.9 Hz, 1 H); 3.81 (d, J=7.9 Hz, 2 H); 1.53 and 1.56 (2 x s, 2 x 3 H); 2.24 (s, 3 H); 4.62 (s, 2 H); 4.32 (q, J=7.2 Hz, 2 H); 1.27 (t, J=7.2 Hz, 3 H); 6.59 (d, J=6.6 Hz, 1 H); 7.19-7.62 (m, 12 H).

### General procedure (D)

### Step A:

### (Z)-[4-[3-(Biphenyl-4-yl)-6-hydroxy-6-methylhept-2-en-4-ynylsulfanyl]-2-methylphenoxy]acetic acid:

A solution of lithium hydroxide in water (2 eq) was added to a solution of the above ester in a mixture of tetrahydrofuran, methanol and water (4:1:1, 3 mL). The reaction mixture was stirred at 0°C for 1 h, water solution of tartaric acid was added and the mixture was extracted with ether. Organic layer was dried, evaporated and purified by column chromatography (silica gel Merck 60, chloroform/methanol 8:2) yielding 400 mg of the title acid.
Yield: 400 mg (60%).
R_{F}= 0.20 (SiO₂, chloroform/methanol 8:2).

One equivalent of L-lysine was added to a solution of the above acid in methanol (2 mL) and after 3 h acetonitrile (150 mL) was added. White crystals of the L-lysinate of the title acid were filtered and dried.
Yield of lysine salt was 250 mg.
¹H NMR spectrum (300 MHz, DMSO-d₆): 6.54 (t, J=7.7 Hz, 1 H); 3.89 (d, J=7.7 Hz, 2 H); 1.47 (s, 3 H); 2.12 (s, 2 x 3 H); 4.18 (s, 2 H); 6.64 (d, J=8.1 Hz, 1 H); 3.16 (bs, 1 H); 2.66-2.76 (m, 2 H); 1.30-1.75 (m, 4 H); 7.12-7.74 (m, 11 H).

### Example 5

### (Z)-[4-[5-(4-Methoxyphenyl)-3-(biphenyl-4-yl)-pent-2-en-4-ynylsulfanyl]-2-methylphenoxy]-acetic acid

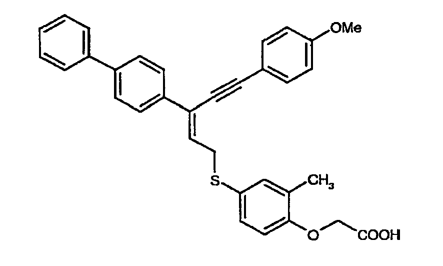

### General procedure (A)

### Step F:

### Ethyl (Z)-[4-[5-(4-Methoxyphenyl)-3-(biphenyl-4-yl)-pent-2-en-4-ynylsulfanyl]-2-methyl-phenoxy]acetate:

Cooper(I) iodide (2 mg, 10 µmol, 2%) and bis(triphenylphosphine)palladium(II) dichloride (11 mg, 15 µmol, 3%) were added to a solution of ethyl (Z)-[4-[3-(biphenyl-4-yl)-3-iodoallylsulfanyl]-2-methylphenoxy]acetate (300 mg, 0.55 mmol, example 3), 1-ethynyl-4-methoxybenzene (130 mg, 1 mmol) and diisopropylamine (100 mg, 1 mmol) in dry tetrahydrofuran (20mL) under inert atmosphere. The reaction mixture was stirred at room temperature for 2 h. Suspension was filtered through a short path of silica gel, the filtrate was evaporated and purified by column chromatography (silica gel Merck 60, hexane/ethyl acetate 1:0 - 6:4) giving 180 mg of the ester.
Yield: 180 mg (60%).
R_{F} = 0.35 (SiO₂, hexane/ethyl acetate 8:2).
¹H NMR spectrum (300 MHz, CDCl₃, δ_{H}): 6.45 (t, J=7.7 Hz, 1 H); 3.97 (d, J=7.7 Hz, 2 H); 3.85 (s, 3 H); 2.19 (s, 3 H); 4.45 (s, 3 H); 4.23 (q, J=7.2 Hz, 2 H); 1.27 (t, J=7.2 Hz, 3 H); 6.57 (d, J=8.5 Hz, 1 H); 7.31-7.70 (m, 17 H).

### General procedure (D)

### Step A:

### (Z)-[4-[5-(4-Methoxyphenyl)-3-(biphenyl-4-yl)-pent-2-en-4-ynylsulfanyl]-2-methylphenoxy]-acetic acid:

A solution of lithium hydroxide in water (2 eq) was added to a solution of the above ester in tetrahydrofuran, methanol and water (4:1:1, 3 mL) and stirred at 0°C for 1 h. Water solution of tartaric acid was added and the mixture was extracted with ether. Organic layer was dried, evaporated and purified by column chromatography (silica gel Merck 60, chloroform /methanol 8:2) yielding 135 mg of the title acid.
Yield: 135 mg (80%).
R_{F} = 0.25 (SiO₂, CHCl₃/methanol 8:2).

One equivalent of L-lysine was added to a solution of the above acid in methanol (2 mL) and acetonitrile (150 mL) was added after 3 h. White crystals of the L-lysinate of the title acid were filtered and dried.
¹H NMR spectrum (300 MHz, DMSO-d₆): 6.62 (t, J=7.7 Hz, 1 H); 3.97 (d, J= 7.7 Hz, 2 H); 3.81 (s, 3 H); 2.04 (s, 3 H); 4.13 (s, 2 H); 3.16 (s, 1 H); 2.66-2.76 (m, 2 H); 1.25-1.75 (m, 4 H); 6.96-7.76 (m, 15 H).

### Example 6

### (Z)-[4-[5-(4-methoxyphenyl)-3-(4-trifluoromethylphenyl)pent-2-en-4-ynylsulfanyl]-2-methylphenoxy]acetic acid

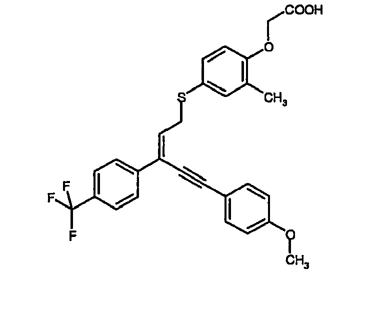

### General procedure (A)

### Step A:

### 1,1-Dibromo-1-(4-trifuoromethylphenyl)-ethane:

A solution of 4-trifluoromethylbenyaldehyde (13.3 mL; 0.1 mol), carbon tetrabromide (36.2 g; 0.11 mol) and triphenylphosphine (28.8 g; 0.11 mol) in anhydrous dichloromethane (250 mL) were stirred overnight. The solid precipitation was filtered off and washed with small amount of dichloromethane. The solution was concentrated, solid material was filtered off again and washed with small amount of dichloromethane. Solvent was evaporated and the product was purified by destilation at oil pump *vacuo* ( 84-99°C, ∼1 torr) giving 28 g (84%) of liquid.

### Step B:

### 3-(4-Trifluoromethyl)-prop-3-yn-1-ol:

To the reaction flask with rubber septum 1,1-dibromo-2-(4-trifuoromethylphenyl) ethane (21,1 g; 64 mmol) was placed and dissolved in anhydrous THF. The reaction was cooled to -78°C and butyllithium (106 mL, 1.5 M solution in hexane; 0.16 mol) was added slowly. The reaction mixture was stirred at -78 °C for additional 0.5 h and paraformaldehyde (4.8 g, 0.16 mmol) was added. The reaction mixture was stirred without cooling until reached the room temperature, poured into water and extracted with ethylacetate (3 x). Combined organic layers were dried over magnesium sulfate and evaporated. Chromatography on silica gel (250 g, gradient elution hexanes-ethylacetate 9:1, 8:2, 7:3) afforded 5.49 g (42 %) of product.

### Step C:

### Z-3-Iodo-3-(4-trimethylphenyl)-prop-2-en-1-ol:

The solution of lithium aluminium hydride in THF (33 mL, 1 M solution in THF, 33 mmol), sodium methoxide (54 mg, 1 mmol) and THF (30 mL) was cooled to 0 °C. The solution of 3-(4-trifluoromethyl)- prop-3-yn-1-ol (5.49 g; 27.5 mmol) in THF (20 mL) was added slowly and stirred 2 h at 0 °C. Dimethylcarbonate (2.78 mL, 33 mmol) was added over 5 min. After 10 min the mixture was cooled to -78 °C and iodine (10 g, 40 mmol) was added. The reaction mixture was stirred without cooling until reached the room temperature, and methanol (10 mL) was added. After 1 h the mixture was poured into water, acidified with HCl and extracted with ethylacetate (3x). Combined organic layers were dried with magnesium sulfate and evaporated. Chromatography on silica gel (hexanes-ethylacetate gradient 9:1-8:2-7:3) afforded 6.42 g (71%) of compound.

### Step D-E:

### Ethyl-Z(4-(3-lodo-3-(4-trimethylphenyl)-prop-2-en-1-yl sulfanyl)-2-methylphenyloxy) acetate:

Solution of Z3-lodo-3-(4-trimethylphenyl)-prop-2-en-1-ol (3.28 g, 10 mmol), carbon tetrabromide (3.98 g, 12 mmol) and triphenylphosphine (3.14 g, 12 mmol) in methylenchloride was stirred overnight at room temperature under nitrogen. The solution of diisopropylethylamine (20 mL, 116 mmol), water (4 mL, 222 mmol) in THF (40 mL) was added and the reaction was kept under nitrogen atmosphere. The ethyl-4-merkapto-2-methylphenyloxy)-acetate (2.94 g, 13 mmol) was added (neat). The reaction was stirred overnight, diluted with ethylacetate and filtered through silica. Solvent was evaporated and mixture was chromatographed on silica gel (100 g, hexan-ethylacetate gradient 95:5 to 80:20) giving 3.96 g (76%).
¹H NMR spectrum (300 MHz, CDCl₃, δ_{H}): 7.4-7.6 (m, 4 H); 7.21-7.33 (m, 2 H); 6.62 (d, J=8.5 Hz, 1 H); 6.00 (d, J=7.28.5 Hz, 1 H); 4.22 (q, J=7.28.5 Hz, 2 H); 3.70 (d, 2 H); 2.26 (s, 3 H); 1.27 (s, J=7.28.5 Hz, 3 H).

### Step F:

### Ethyl (Z)-[4-[5-(4-methoxyphenyl)-3-(4-trifluoromethylphenyl)pent-2-en-4-ynylsulfanyl]-2-methylphenoxy]acetate:

A mixture of dry zinc bromide (11 mg, 50 µmol), dry triethylamine (3 mL) and tetrahydrofuran (5 mL) was stirred for 20 min and then a solution of ethyl (Z)-[4-[3-iodo-3-(4-trifluoromethylphenyl)allylsulfanyl]-2-methylphenoxy]acetate (200 mg, 0.37 mmol) and 4-ethynylanisole (80 mg, 0.6 mmol) in dry tetrahydrofuran (10 mL) was added, followed by addition of tetrakis(triphenylphosphine)palladium (6 mg, 11 µmol, 3%). The reaction mixture was stirred overnight at room temperature. The next day the solution was filtered through a short path of silica gel and evaporated. Crude product was purified by column chromatography (silica gel Merck 60, hexane/ethyl acetate 1:0 - 6:4) giving 130 mg of the ester.
Yield: 130 mg (65%).
R_{F} = 0.3 (SiO₂, hexane/ethyl acetate 8:2).
¹H NMR spectrum (250 MHz, CDCl₃, δ_{H}): 7.55-7.71 (m, 4 H); 7.35-7.42 (m, 2 H); 7.30 (d, 1 H, J = 2.284); 7.23 (dd, 1 H, J = 2.28, 8.37); 6.85 - 6.92 (m, 2 H); 6.57 (d, 1 H, J = 8.37); 6.45 (t, 1 H, J = 7.84); 4.54 (s, 2 H); 4.23 (q, 2 H, J = 7.08); 3.94 (d, 2 H, J = 7.84); 3.84 (s, 3H); 2.17 (s, 3 H); 1.26 (t, 3 H, J = 7.08).

### General procedure (D)

### Step A:

### (Z)-[4-[5-(4-methoxyphenyl)-3-(4-trifluoromethylphenyl)pent-2-en-4-ynylsulfanyl]-2-methylphenoxy]acetic acid:

Lithium hydroxide monohydrate (20 mg, 0.450 mmol, 2 eq) was added to a solution of the above ester in the mixture of tetrahydrofuran, methanol and water (5:1:1, 5 mL). The mixture was stirred for 2 h under inert gas maintaining the reaction temperature below 5 °C. A solution of tartaric acid was added and the liberated acid was extracted with ether. Crude product after drying and evaporating of solvents was purified by column chromatography (silica gel Merck 60, dichloromethane/methanol 1:0 - 8:2) yielding 60 mg of the title acid.
Yield: 60 mg (50%).
R_{F}= 0.75 (SiO₂, chloroform/methanol 8:2).

The above acid (60 mg, 0.117 mmol) was dissolved in a minimal amount of dry methanol (about 2 mL) and L-lysine (17.5 mg, 0.120 mmol) was added. The reaction mixture was stirred at room temperature for 90 min and then acetonitrile (50 mL) was added. Precipitated solid was filtered off and dried in vacuo yielding 50 mg of L-lysinate of the title acid.
Yield: 50 mg (65 %).
¹H NMR spectrum (250 MHz, DMSO-d₆): 7.89-7.70 (m, 4 H); 7.49 (d, 2 H); 7.24 (d, 1 H); 7.20 (dd, 1 H); 7.02 (d, 2 H); 6.73 (t, 1 H); 6.65 (d, 1 H); 4.42 (s, 2 H); 3.98 (d, 2 H); 3.83 (s, 3 H); 3.19 (t, 1 H); 2.75 (t, 2 H); 2.06 (s, 3 H); 1.24-1.77 (m, 6 H).

### Example 7

### (Z) [4-(3,5-Diphenyl-pent-2-en-4-ynyloxy)-2-methyl-phenoxy]-acetic acid

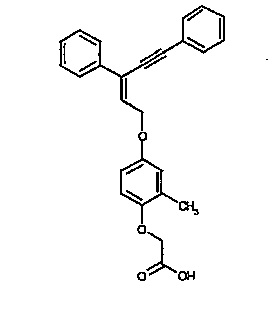

Sodium methoxide (27 mg, 0.50 mmol) was added to a solution of 3-phenylprop-2-yn-1-ol (661 mg, 5.0 mmol; prepared according to J. Org. Chem. 1986, 51, 46) in dry tetrahydrofuran (20 mL). The resulting solution was cooled down to 0 °C, the reaction flask was flushed with nitrogen and 1 M solution of lithium aluminum hydride in tetrahydrofuran (6 mL, 6.0 mmol) was added. The reaction mixture was stirred for 1 h under cooling in atmosphere of nitrogen; then warmed up to 15 °C and dimethyl carbonate (900 mg, 10 mmol) was added. The mixture was stirred for 30 min, cooled down to -78 °C, a solution of iodine (2.54 g, 10 mmol) in dry tetrahydrofuran (5 mL) was added and the mixture was stirred for 3 h under cooling and then was kept overnight in a refrigerator. The reaction mixture was poured into aqueous solution of sodium thiosulfate and extracted with ethyl acetate (3 x 20 mL). The combined organic solutions were filtered through a short path of silica gel, silica gel was washed ethyl acetate (20 mL) and the filtrates were dried with anhydrous sodium sulfate. Evaporation of the organic solution gave sufficiently pure crude (Z)-3-iodo-3-phenylprop-2-en-1-ol.
Yield: 1.20 g (92 %).
R_{F} (SiO₂, hexane/ethyl acetate 75:25) 0.55.

In atmosphere of nitrogen, methyl (4-hydroxy-2-methylphenoxy)acetate (849 mg, 4.33 mmol), triphenylphosphine (1138 mg, 4.33 mmol) and diisopropyl azodicarboxylate (1.71 ml, 8.66 mmol) were added to a degassed solution of the above hydroxy derivative (939 mg, 3.61 mmol) in dry tetrahydrofuran (40 mL). The reaction mixture was stirred for 20 h at ambient temperature, then filtered through a short path of silica gel and silica gel was washed with ether (100 mL). Water (150 mL) was added to the combined filtrates and the mixture was extracted with ether (3 x 60 mL). The combined organic extracts were dried with anhydrous sodium sulfate and solvents were evaporated *in vacuo.* The residue was purified by column chromatography (silica gel Fluka 60, hexane/ethyl acetate 90:10) yielding methyl (Z)-[4-(3-iodo-3-phenylallyloxy)-2-methylphenoxy]acetate.
Yield: 371 mg (23 %).
R_{F} (SiO₂, hexane/ethyl acetate 75:25) 0.60.
¹H NMR spectrum (200 MHz, CDCl₃, δ_{H}): 7.51-7.46 (m, 2 H); 7.32-7.26 (m, 3 H); 6.79 (s, 1 H); 6.68 (s, 2 H); 6.34 (t, J=5.1 Hz, 1 H), 4.69 (d, J=5.1 Hz, 2 H); 4.60 (s, 2 H); 3.80 (s, 3 H); 2.29 (s, 3 H).

In atmosphere of nitrogen, copper(I) iodide (2 mg, 8 µmol), bis(triphenylphosphine)-palladium dichloride (8 mg, 12 µmol) were added to a solution of the above above iodo derivate (171 mg, 0.39 mmol) in dry in dry tetrahydrofuran (10 mL). In next step, N,N-diisopropylamine (110 µl, 0.78 mmol) and phenylethyne (86 µl, 0.78 mmol) were added and the reaction mixture was heated at 40 °C for 5 h. The solution was filtered through a short path of silica gel, the combined filtrates were diluted with ethyl acetate (50 mL), the solution was washed with hydrochloric acid (3 %) and saturated aqueous solution of sodium bicarbonate (3 x 30 mL) and dried with anhydrous sodium sulfate. Solvents were evaporated *in vacuo* and the residue was purified by column chromatography (silica gel Fluka 60, hexane/ethyl acetate 95:5) yielding (Z) [4-(3,5-Diphenyl-pent-2-en-4-ynyloxy)-2-methyl-phenoxy]-acetic acid methyl ester.
Yield: 77 mg (48 %).
R_{F} (SiO₂, hexane/ethyl acetate 90:10) 0.55.
¹H NMR spectrum (200 MHz, CDCl₃, δ_{H}): 7.72-7.36 (m, 10 H); 6.85-6.59 (m, 4 H); 5.03 (d, J=6.2 Hz, 2 H); 4.60 (s, 2 H); 3.79 (s, 3 H); 2.28 (s, 3 H).

In atmosphere of nitrogen, lithium hydroxide monohydrate (10 mg, 0.22 mmol) was added to a solution of the above ester (77 mg, 0.19 mmol) in methanol/water/tetrahydrofuran mixture (2:2:3; 5 mL). The reaction mixture was stirred for 1 h at ambient temperature and then diluted aqueous of tartaric acid (5 mL) and ether (10 mL) were added. The organic layer was dried with anhydrous sodium sulfate and solvents were evaporated *in vacuo.* The obtained (Z) [4-(3,5-Diphenyl-pent-2-en-4-ynyloxy)-2-methyl-phenoxy]-acetic acid as a white solid was not further purified.
Yield: 65 mg (86 %).
R_{F} (SiO₂, dichloromethane/methanol 90:10) 0.36.
¹H NMR spectrum (200 MHz, CDCl₃, δ_{H}): 7.73-7.35 (m, 10 H); 6.86-6.71 (m, 3 H); 6.61 (t, J=6.3 H, 1 H); 5.04 (d, J=6.2 Hz, 2 H); 4.62 (s, 2 H); 2.27 (s, 3 H).

A solution of L-lysine (24 mg, 0.16 mmol) in methanol/water mixture (1:1, 1.5 mL) was added to a solution of the above acid (65 mg, 0.16 mmol) in methanol (2 mL). After 30 min, the solvents were evaporated *in vacuo,* the residue was diluted with methanol (1.5 mL) and then acetonitrile (30 mL) was added portionwise. The separated L-lysinate of the title acid was filtered off and dried *in vacuo.*
Yield: 51 mg (59 %)
M.p. 160-170 °C.
¹H NMR spectrum (200 MHz, CD₃COOD, δ_{H}): 7.62-7.32 (m, 10 H); 6.68-6.77 (m, 3 H); 6.73 (bt, 1 H); 5.06 (d, J=6.4 Hz, 2 H); 4.75 (s, 1 H); 4.67 (s, 2 H); 4.08 (bt, 2 H); 3.08 (bt, 6 H); 2.23 (s, 3 H).

### Example 8

### (Z)-[4-[3-(4-Bromophenyl)-5-phenylpent-2-en-4-ynyloxy]-2-methylphenoxy]acetic acid

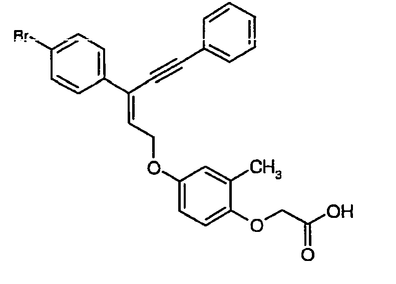

(*Z*)-3-(4-Bromophenyl)-3-iodoprop-2-en-1-ol (0.950 g, 2.8 mmol), carbon tetrabromide (0.913 g, 3 mmol) and triphenylphosphine (0.786 g, 3 mmol) were mixed in anhydrous dichloromethane (10 mL) and the mixture was stirred at 0 °C for 3 h and then at 20 °C overnight. Ether (50 mL) and hexanes (30mL) were added and the mixture was filtrated through a paddle of silica to remove precipitated triphenylphosphine oxide. Solvents were removed by evaporation *in vacuo* and crude 1-bromo-4-(*(Z)*-3-bromo-1-iodopropenyl)benzene was prepared in quantitative yield and was subsequently used without further purification.
Yield: 1.20 g (100 %).
R_{F} (SiO₂, hexanes/ethyl acetate 9:1) 0.90.

The above allyl bromide (0.787 g, 1.96 mmol), methyl (4-hydroxy-2-methylphenoxy)acetate (0.652 g, 2.00 mmol) and cesium carbonate (0.652 g, 2.00 mmol) were stirred at 20 °C overnight. The mixture was filtered and evaporated in *vacuo.* The residue was submitted to column chromatography (silica gel Fluka 60, hexanes/ethyl acetate 95:5) affording methyl (*Z*)-[4-[3-(4-bromophenyl)-3-iodoallyloxy]-2-methylphenoxy]acetate. Yield: 0.722 g (71 %).
R_{F} (SiO₂, hexanes/ethyl acetate 9:1) 0.35.

Methyl (*Z*)-[4-[3-(4-Bromophenyl)-3-iodoallyloxy]-2-methylphenoxy]acetate (0.43 g, 0.83 mmol), phenyl acetylene (0.110 mL, 1 mmol); diisopropyl ethyl amine (0.14 mL, 1 mmol), bis(triphenylphosphine)palladium dichloride (16 mg, 0.04 mmol) and copper(I) iodide (3.8 mg, 0.02 mmol) were mixed in anhydrous tetrahydrofuran (10 mL). The solution was evacuated, put under nitrogen and stirred at 65 °C for 16 h. The solvents were evaporated *in vacuo* and the residue was submitted to column chromatography (silica gel Fluka 60, hexanes/ethyl acetate 90:10) affording methyl (*Z*)-[4-[3-(4-bromophenyl)-5-phenylpent-2-en-4-ynyloxy]-2-methylphenoxy]acetate.
Yield: 0.383 g (94 %).
R_{F} (SiO₂, hexanes/ethyl acetate 9:1) 0.15.
¹H NMR spectrum (300 MHz, CDCl₃, δ_{H}): 7.60-7.46 (m, 5 H); 7.40-7.35 (m, 4 H), 6.83 (d, J=2.7 Hz, 1 H), 6.76-6.64 (m, 2 H); 6.61 (t, J=6.1 Hz, 1 H); 4.99 (d, J=6.1 Hz, 2 H); 4.59 (s, 2 H), 3.79 (s, 3 H); 2.28 (s, 3 H).

The above ester (0.150 g, 0.31 mmol) was dissolved in a mixture of tetrahydrofuran (3 mL) and methanol (1 mL). The solution of lithium hydroxide monohydrate (22 mg, 0.5 mmol) was added and the mixture was left to stand for 2 h. The reaction solution was diluted with water (15 mL) and acidified with 1 M aqueous hydrochloric acid. The precipitated material was filtered off, washed with water and dried on the air.
Yield: 0.128 g (86 %).
R_{F} (SiO₂, dichloromethane/methanol 9:1) 0.25.
M.p. 151-155 °C.
¹H NMR spectrum (300 MHz, CDCl₃, δ_{H}): 12.88 (s, 1 H); 7.80-7.40 (m, 9 H); 6.90-6.70 (m, 4 H); 4.97 (d, J=5.9 Hz, 2 H); 4.60 (s, 2 H); 2.16 (s, 3 H).

### Example 9

### (Z)-[4-[3-(4-Bromophenyl)-5-(pyridin-2-yl)pent-2-en-4-ynyloxy]-2-methylphenoxy]acetic acid

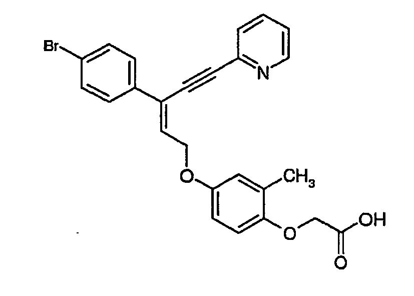

Methyl (*Z*)-[4-[3-(4-Bromophenyl)-3-iodoallyloxy]-2-methylphenoxy]acetate (0.292 g, 0.56 mmol), 2-ethynylpyridine (0.05 mL, 0.5 mmol); diisopropyl ethyl amine (0.071 mL, 0.5 mmol), bis(triphenylphosphine)palladium dichloride (6 mg, 0.02 mmol) and copper(I) iodide (1.2 mg, 0.01 mmol) were mixed in anhydrous tetrahydrofuran (5 mL). The solution was evacuated, put under nitrogen and stirred at 65 °C for 16 h. The solvents were evaporated *in vacuo* and the residue was submitted to column chromatography (silica gel Fluka 60, hexanes/ethyl acetate 90:10) affording methyl (*Z*)-[4-[3-(4-bromophenyl)-5-(pyridin-2-yl)pent-2-en-4-ynyloxy]-2-methylphenoxy]acetate.
Yield: 0.114 g (46 %).
R_{F} (SiO₂, hexanes/ethyl acetate 9:1) 0.15.

The above ester (0.114 g, 0.23 mmol) was dissolved in a mixture of tetrahydrofuran (3 mL) and methanol (1 mL) and a solution of lithium hydroxide monohydrate (22 mg, 0.5 mmol) was added. The mixture was left to stand for 2 h and then diluted with saturated aqueous solution of ammonium chloride (20 mL). The resulting mixture was extracted with ethyl acetate (3 x 15 mL); the organic layers were combined and dried with anhydrous sodium sulfate. The precipitated material was filtered off, washed with water and dried on the air.
Yield: 0.065 g (61 %).
R_{F} (SiO₂, dichloromethane/methanol 9:1) 0.20.
M.p. 178-186 °C.
¹H NMR spectrum (300 MHz, CDCl₃, δ_{H}): 8.63 (d, J=5.0 Hz, 1 H); 7.92-7.90 (m, 7 H); 6.98 (t, J=6.1 Hz, 1 H); 6.86 (bs, 1 H); 6.80-6.70 (m, 2 H); 4.98 (d, J=6.3 Hz, 2 H); 4.54 (s, 2 H); 2.15 (s, 3 H).

### Example 10

### {4-[3-(4-Bromo-phenyl)-5-(5-methyl-thiophen-2-yl)-pent-2-en-4-ynyloxy]-2-methyl-phenoxy}-acetic acid

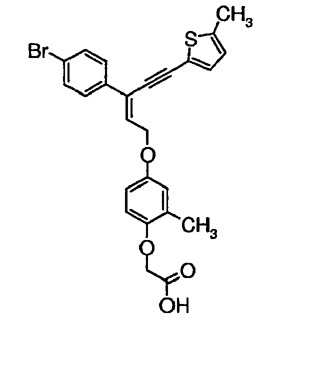

### Example 11

### {2-Methyl-4-[3-(2-methyl-benzofuran-5-yl)-5-(5-methyl-thiophen-2-yl)-pent-2-en-4-ynyloxy]-phenoxy}-acetic acid

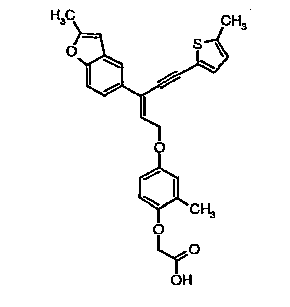

### Example 12

### {4-[3-(4-Bromo-phenyl)-6-morpholin-4-yl-hex-2-en-4-ynyloxy]-2-methyl-phenoxy}-acetic acid

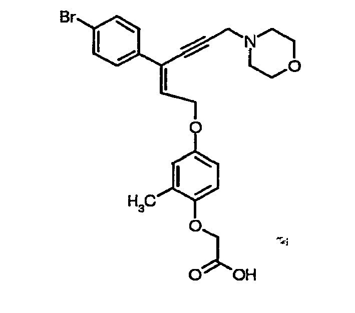

### Example 13

### (2-Methyl-4-[3-[4-(5-methyl-thiophen-2-yl)-phenyl]-5-phenyl-pent-2-en-4-ynyloxy}-phenoxy)-acetic acid

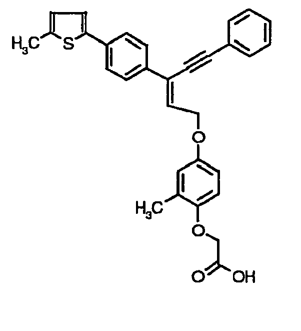

### PHARMACOLOGICAL METHODS

### In vitro PPARalpha, PPARgamma and PPARdelta activation activity

The PPAR transient transactivation assays are based on transient transfection into human HEK293 cells of two plasmids encoding a chimeric test protein and a reporter protein respectively. The chimeric test protein is a fusion of the DNA binding domain (DBD) from the yeast GAL4 transcription factor to the ligand binding domain (LBD) of the human PPAR proteins. The PPAR-LBD moiety harbored in addition to the ligand binding pocket also the native activation domain (activating function 2 = AF2) allowing the fusion protein to function as a PPAR ligand dependent transcription factor. The GAL4 DBD will direct the chimeric protein to bind only to Gal4 enhancers (of which none existed in HEK293 cells). The reporter plasmid contained a Gal4 enhancer driving the expression of the firefly luciferase protein. After transfection, HEK293 cells expressed the GAL4-DBD-PPAR-LBD fusion protein. The fusion protein will in turn bind to the Gal4 enhancer controlling the luciferase expression, and do nothing in the absence of ligand. Upon addition to the cells of a PPAR ligand luciferase protein will be produced in amounts corresponding to the activation of the PPAR protein. The amount of luciferase protein is measured by light emission after addition of the appropriate substrate.

### CELL CULTURE AND TRANSFECTION

HEK293 cells where grown in DMEM + 10% FCS. Cells were seeded in 96-well plates the day before transfection to give a confluency of 50-80 % at transfection. A total of 0,8 µg DNA containing 0,64 µg pM1α/γLBD, 0,1 µg pCMVβGal, 0,08 µg pGL2(Gal4)₅ and 0,02 µg pADVANTAGE was transfected per well using FuGene transfection reagent according to the manufacturers instructions (Roche). Cells were allowed to express protein for 48 h followed by addition of compound.
Plasmids: Human PPAR α, γ and δ was obtained by PCR amplification using cDNA synthesized by reverse transcription of mRNA from human liver, adipose tissue and plancenta respectively. Amplified cDNAs were cloned into pCR2.1 and sequenced. The ligand binding domain (LBD) of each PPAR isoform was generated by PCR (PPARα: aa 167 - C-terminus; PPARγ: aa 165 - C-terminus; PPARδ: aa 128 - C-terminus) and fused to the DNA binding domain (DBD) of the yeast transcription factor GAL4 by subcloning fragments in frame into the vector pM1 (Sadowski et al. (1992), Gene 118, 137) generating the plasmids pM1αLBD, pM1γLBD and pM1δ. Ensuing fusions were verified by sequencing. The reporter was constructed by inserting an oligonucleotide encoding five repeats of the GAL4 recognition sequence (5 x CGGAGTACTGTCCTCCG(AG)) (Webster et al. (1988), Nucleic Acids Res. 16, 8192) into the vector pGL2 promotor (Promega) generating the plasmid pGL2(GAL4)₅. pCMVβGal was purchased from Clontech and pADVANTAGE was purchased from Promega.

### IN VITRO TRANSACTIVATION ASSAY

Compounds: All compounds were dissolved in DMSO and diluted 1:1000 upon addition to the cells. Compounds were tested in quadruple in concentrations ranging from 0.001 to 300 µM. Cells were treated with compound for 24 h followed by luciferase assay. Each compound was tested in at least two separate experiments.

Luciferase assay: Medium including test compound was aspirated and 100 µl PBS incl. 1 mM Mg++ and Ca++ was added to each well. The luciferase assay was performed using the LucLite kit according to the manufacturers instructions (Packard Instruments). Light emission was quantified by counting on a Packard LumiCounter. To measure β-galactosidase activity 25 µl supernatant from each transfection lysate was transferred to a new microplate. β-gaiactosidase assays were performed in the microwell plates using a kit from Promega and read in a Labsystems Ascent Multiscan reader. The β-galactosidase data were used to normalize (transfection efficiency, cell growth etc.) the luciferase data.

### STATISTICAL METHODS

The activity of a compound is calculated as fold induction compared to an untreated sample. For each compound the efficacy (maximal activity) is given as a relative activity compared to Wy14,643 for PPARα, Rosiglitazone for PPARγ and Carbacyclin for PPARδ. The EC50 is the concentration giving 50% of maximal observed activity. EC50 values were calculated via non-linear regression using GraphPad PRISM 3.02 (GraphPad Software, San Diego, Ca). The results were expressed as means ± SD.

## Claims

1. A compound of the general formula (I): 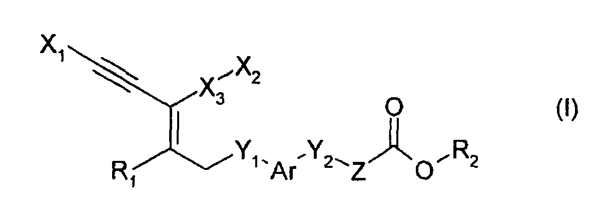
wherein X₁ is aryl or heteroaryl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₈-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkylcarbonyl, arylcarbonyl, heteroarylcarbonyl, C₁₋₅-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloaikyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more halogens, CN and OH; or
X₁ is C₁₋₆-alkyl, C₃₋₆-cycloalkyl, C₂₋₆-alkenyl or C₃₋₆-cycloalkyl-C₁₋₆-alkyl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,anylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkysulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylamino-carbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more halogens, CN and OH; or
X₁ is aralkyl or heteroaralkyl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylaminocarbonyl, di-(C₁₋₆-alkyl)aminocarbonyl, C₁₋₆-alkylamino, di-(C₁₋₆-alkyl)amino or C₃₋₆-cycloalkylamino, each of which is optionally substituted with one or more halogens, CN and OH; and
X₂ is aryl or heteroaryl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₈-alkynyl, aryl, aralkyl, heteroaryl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylamino.carbonyl, di-(C₁₋₆-alkyl)aminocarbonyl, C₁₋₆-alkylamino, di-(C₁₋₆-alkyl)amino or C₃₋₆-cycloalkylamino, each of which is optionally substituted with one or more halogens, CN and OH; or
X₂ is selected from
• hydrogen, halogen, hydroxy, cyano, amino or carboxy; or
• C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylaminocarbonyl, di-(C₁₋₆-alkyl)-aminocarbonyl, C₁₋₆-alkylamino, di-(C₁₋₆-alkyl)amino or C₃₋₆-cycloalkylamino, each of which is optionally substituted with one or more halogens, CN and OH; and
X₃ is arylene or heteroarylene each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cycloalkylamino, each of which is optionally substituted with one or more halogens; and
Ar is phenylene which is optionally substituted with one or more substituents selected from
• halogen; or
• C₁₋₆-alkyl, optionally substituted with one or more halogens; and
Y₁ is O or S; and
Y₂ is O or S; and
Z is -(CH₂)ₙ- wherein n is 1, 2 or 3; and
R₁ is hydrogen, halogen or a substituent selected from
• C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio, each of which is optionally substituted with one or more halogens; and R₂ is hydrogen, C₁₋₆-alkyl, C₃₋₆-cycloalkyl, C₂₋₆-alkenyl, C₂₋₆-alkynyl, C₄₋₆-alkenynyl or aryl;
wherein "heterocyclyl" represents a saturated 3 to 12 membered monocyclic ring containing one or more heteroatoms selected from nitrogen, oxygen, sulfur, S(=O) and S(=O)₂ or a saturated bicyclic ring containing one or more heteroatoms selected from nitrogen, oxygen, sulfur, S(=O) and S(=O)₂, or a saturated heterocyclic ring containing one or more heteroatoms selected from nitrogen, oxygen, sulfur, S(=O) and S(=O)₂ and having one or two bridges, or a saturated heterocyclic ring containing one or more heteroatoms selected from nitrogen, oxygen, sulfur, S(=O) and S(=O)₂ and containing one or more spiro atoms; or
a pharmaceutically acceptable salt thereof, or a pharmaceutically acceptable solvate thereof, or any tautomeric forms, stereoisomers, mixture of stereoisomers including a racemic mixture, or polymorphs.

2. A compound according to claims 1 wherein X₁ is aryl or heteroaryl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, C₁₋₆-alkoxy, C₃₋₆-cycloalkoxy, C₃₋₆-cycloalkyl-C1-6-alkoxy, aryloxy, heteroaryloxy, aralkoxy, heteroaralkoxy, C₁₋₆-alkylthio, C₃₋₈-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cydoalkylcarbonyl, C₃₋₆-cycloalkyl- C₁₋₆-alkylcarbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more halogens, CN and OH; or
X₁ is C₁₋₆-alkyl, C₃₋₆-cydoalkyl, C₂₋₆-alkenyl or C₃₋₆-cydoalkyl-C₁₋₆-alkyl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio, arylthio, heteroarylthio, aryl-C₁₋₆-aikylthio, heteroaryl-C₁₋₆-alkylthio, C₁₋₆-alkylcarbonyl, C₃₋₆-cycloalkylcarbonyl, C₃₋₆-cycloalkyl-C₁₋₆-alkyl-carbonyl, arylcarbonyl, heteroarylcarbonyl, C₁₋₆-alkylsulfonyl, C₃₋₆-cycloalkylsulfonyl, C₃₋₆-cydoalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl. C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more halogens, CN and OH; or
X₁ is aralkyl or heteroaralkyl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylaminocarbonyl, di-(C₁₋₆-alkyl)aminocarbonyl, C₁₋₆-alkylamino, di-(C₁₋₆-alkyl)amino or C₃₋₆-cycloalkylamino, each of which is optionally substituted with one or more halogens, CN and OH; and
X₂ is aryl or heteroaryl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C-₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylaminocarbonyl, di-(C₁₋₆-alkyl)aminocarbonyl, C₁₋₆-alkylamino, di-(C₁₋₆-alkyl)amino or C₃₋₆-cycloalkylamino, each of which is optionally substituted with one or more halogens, CN and OH; or
X₂ is selected from
• hydrogen, halogen, hydroxy, cyano, amino or carboxy; or
• C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, heteroaryloxy, aralkoxy, heteroaralkoxy, C₁₋₆-alkylthio, arylthio, heteroarylthio, aryl-C₁₋₆-alkylthio, heteroaryl-C₁₋₆-alkylthio, C₃₋₆-cycloalkylthio, C₁₋₆-alkylcarbonyl, arylcarbonyl, C₁₋₆-alkylsulfonyl, arylsulfonyl, heteroarylsulfonyl, C₁₋₆-alkoxycarbonyl, C₁₋₆-alkylamido, arylamido, C₁₋₆-alkylaminocarbonyl, di-(C₁₋₆-alkyl)aminocarbonyl, C₁₋₆-alkylamino, di-(C₁₋₆-alkyl)amino or C₃₋₈-cycloalkylamino, each of which is optionally substituted with one or more halogens, CN and OH; and
X₃ is arylene or heteroarylene each of which is optionally substituted with one or more substituents selected from
• halogen, hydroxy, cyano, amino or carboxy; or
• C₁₋₆-alkyl, C₃₋₆-cycloalkyl, C₂₋₈-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cycloalkylamino, each of which is optionally substituted with one or more halogens; and
Ar is phenylene which is optionally substituted with one or more substituents selected from
• halogen; or
• C₁₋₆-alkyl, optionally substituted with one or more halogens; and
Y₁ is O or S; and
Y₂ is O or S; and
Z is -(CH₂)ₙ- wherein n is 1, 2 or 3; and
R₁ is hydrogen, halogen or a substituent selected from
• C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio, each of which is optionally substituted with one or more halogens; and
R₂ is hydrogen, C₁₋₆-alkyl, C₃₋₆-cycloalkyl, C₂₋₆-alkenyl, C₂₋₆-alkynyl, C₄₋₆-alkenynyl or aryl; or
a pharmaceutically acceptable salt thereof, or a pharmaceutically acceptable solvate thereof, or any tautomeric forms, stereoisomers, mixture of stereoisomers including a racemic mixture, or polymorphs.

3. A compound according to claim 1 or 2, wherein X₁ is aryl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkyaminocarbonyl, C₁₋₆-alkylamino or C₁₋₆-dialkylamino each of which is optionally substituted with one or more halogens.

4. A compound according to claim 3, wherein X₁ is aryl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₆-alkylthio, arylthio or C₁₋₈-dialkylaminocarbonyl, each of which is optionally substituted with one or more halogens.

5. A compound according to claim 4, wherein X₁ is aryl optionally substituted with one or more substituents selected from
• halogen; or
• C₁₋₆-alkyl, C₁₋₆-alkoxy or C₁₋₆-alkylthio, each of which is optionally substituted with one or more halogens.

6. A compound according to any one of the preceding claims, wherein X₁ is phenyl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is opiionaiiy substituted with one or more halogens.

7. A compound according to claim 6, wherein X₁ is phenyl optionally substituted with one or more substituents selected from
• halogen; or
• C₁₋₆-alkyl, C₁₋₆-alkoxy or C₁₋₆-alkylthio each of which is optionally substituted with one or more halogens.

8. A compound according to any one of the preceding claims, wherein X₁ is phenyl.

9. A compound according to claim 1 or 2, wherein X₁ is heteroaryl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

10. A compound according to claim 9, wherein X₁ is heteroaryl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₆-alkylthio, arylthio or C₁₋₆-dialkylaminocarbonyl, each of which is optionally substituted with one or more halogens.

11. A compound according to claim 10, wherein X₁ is heteroaryl optionally substituted with one or more substituents selected from
• halogen; or
• C₁₋₆-alkyl, C₁₋₆-alkoxy or C₁₋₆-alkylthio each of which is optionally substituted with one or more halogens.

12. A compound according to claim 1, wherein X₁ is C₁₋₆-alkyl optionally substituted with one or more substituents selected from
• halogen or hydroxy; or
• aryl, heteroaryl, heterocyclyl, C₁₋₆-alkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

13. A compound according to claim 12, wherein X₁ is C₁₋₆-alkyl optionally substituted with one or more substituents selected from
• halogen or hydroxy; or
• aryl, heteroaryl, C₁₋₆-alkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

14. A compound according to claim 13, wherein X₁ is C₁₋₆-alkyl optionally substituted with one or more substituents selected from
• halogen or hydroxy; or
• aryl, heteroaryl, C₁₋₆-alkoxy, C₁₋₆-alkylthio, C₁₋₆-alkylcarbonyl, C₁₋₆-alkylsulfonyl, C₁₋₆-alkylamido, C₁₋₆-alkylaminocarbonyl, C₁₋₆-dialkylaminocrbonyl, C₁₋₆-alkylamino, C₁₋₆-dialkylamino or C₃₋₆-cycloalkylamino, each of which is optionally substituted with one or more halogens.

15. A compound according to claim 14, wherein X₁ is C₁₋₆-alkyl optionally substituted with one or more substituents selected from halogen or hydroxy.

16. A compound according to claim 1 or 2, wherein X₁ is C₃₋₆-cycloalkyl optionally substituted with one or more substituents selected from
• halogen or hydroxy; or
• aryl, heteroaryl, C₁₋₆-alkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₅-dialkylaminocarbonyl, C₁₋₆-alkylamino, C₁₋₆-dialkylamino or C₃₋₆-cycloalkylamino, each of which is optionally substituted with one or more halogens.

17. A compound according to claim 16, wherein X₁ is C₃₋₆-cycloalkyl optionally substituted with one or more substituents selected from
• halogen or hydroxy; or
• aryl, heteroaryl, C₁₋₆-alkoxy, C₁₋₆-alkylthio, C₁₋₆-alkylcarbonyl, C₁₋₆-alkylsuifonyl, C₁₋₆-alkylamido, C₁₋₆-alkylaminocarbonyl, C₁₋₆-dialkylaminocarbonyl, C₁₋₆-alkylamino, C₁₋₆-dialkylamino or C₃₋₆-cydoalkylamino, each of which is optionally substituted with one or more halogens.

18. A compound according to claim 17, wherein X₁ is C₃₋₆-cydoalkyl optionally substituted with one or more substituents selected from halogen or hydroxy.

19. A compound according to claim 1 or 2, wherein X₁ is aralkyl or heteroaralkyl each of which is optionally substituted with one or more substituents selected from
• halogen or hydroxy; or
• C₁₋₆-alkoxy, C₁₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino, C₁₋₆-dialkylamino or C₃₋₆-cycloalkylamino, each of which is optionally substituted with one or more halogens.

20. A compound according to claim 19, wherein X₁ is aralkyl or heteroaralkyl each of which is optionally substituted with one or more substituents selected from
• halogen or hydroxy; or
• C₁₋₆-alkoxy which is optionally substituted with one or more halogens.

21. A compound according to any one of the preceding claims, wherein X₂ is aryl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

22. A compound according to claim 21, wherein X₂ is aryl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₈-alkylthio, arylthio or C₁₋₆-dialkylaminocarbonyl, each of which is optionally substituted with one or more halogens.

23. A compound according to claim 22, wherein X₂ is aryl optionally substituted with one or more substituents selected from
• halogen; or
• C₁₋₆-alkyl, C₁₋₆-alkoxy or C₁₋₆-alkylthio each of which is optionally substituted with one or more halogens.

24. A compound according to any one of the preceding claims 21-23, wherein X₂ is phenyl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

25. A compound according to claim 24, wherein X₂ is phenyl optionally substituted with one or more substituents selected from
• halogen; or
• C₁₋₆-alkyl, C₁₋₆-alkoxy or C₁₋₆-alkylthio each of which is optionally substituted with one or more halogens.

26. A compound according to claim 25, wherein X₂ is phenyl

27. A compound according to any one of the claims 1-20, wherein X₂ is heteroaryl optionally substituted with one or more substituents selected from
• halogen, hydroxy; or
• C₁₋₆-alkyl, aryl, heteroaryl, C₁₋₆-alkoxy, aryloxy, aralkoxy, heteroaralkoxy, C₁₋₆-alkylthio, arylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-dialkylaminocarbonyl, C₁₋₆-alkylamino or C₁₋₆-dialkylamino each of which is optionally substituted with one or more halogens.

28. A compound according to claim 27, wherein X₂ is heteroaryl optionally substituted with one or more substituents selected from
• halogen, hydroxy; or
• C₁₋₆-alkyl, aryl, heteroaryl, C₁₋₆-alkoxy, aryioxy, aralkoxy, heteroaralkoxy, C₁₋₆-alkylthio, arylthio or C₁₋₆-dialkylaminocarbonyl, each of which is optionally substituted with one or more halogens.

29. A compound according to claim 28, wherein X₂ is heteroaryl optionally substituted with one or more substituents selected from
• halogen; or
• C₁₋₆-alkyl, C₁₋₆-alkoxy or C₁₋₆-alkylthio each of which is optionally substituted with one or more halogens.

30. A compound according to any one of the preceding claims 1-20, wherein X₂ is hydrogen, halogen, hydroxy or cyano; or
• C₁₋₆-alkyl, aralkyl or heteroaralkyl each of which is optionally substituted with one or more halogens, CN and OH.

31. A compound according to claim 30, wherein X₂ is halogen or hydroxy; or C₁₋₆-alkyl optionally substituted with one or more halogens.

32. A compound according to claim 31, wherein X₂ is halogen.

33. A compound according to any one of the preceding claims, wherein X₃ is arylene optionally substituted with one or more substituents selected from
• halogen or
• C₁₋₆-alkyl, C₁₋₆-alkoxy or C₁₋₆-alkylthio each of which is optionally substituted with one or more halogens.

34. A compound according to claim 33, wherein X₃ is phenylene optionally substituted with one or more substituents selected from
• halogen or
• C₁₋₆-alkyl, C₁₋₆-alkoxy or C₁₋₆-alkylthio each of which is optionally substituted with one or more halogens.

35. A compound according to claim 34, wherein X₃ is phenylene optionally substituted with one or more substituents selected from halogen or C₁₋₆-alkyl, which is optionally substituted with one or more halogens.

36. A compound according to claim 35, wherein X₃ is phenylene.

37. A compound according to any one of the preceding claims 1-32, wherein X₃ is heteroarylene optionally substituted with one or more substituents selected from
• halogen or
• C₁₋₆-alkyl, C₁₋₆-alkoxy or C₁₋₆-alkylthio each of which is optionally substituted with one or more halogens.

38. A compound according to claim 37, wherein X₃ is heteroarylene optionally substituted with one or more substituents selected from halogen or C₁₋₆-alkyl which is optionally substituted with one or more halogens.

39. A compound according to any one of the preceding claims, wherein Y₁ is S.

40. A compound according to any one of the preceding claims, wherein Y₂ is O.

41. A compound according to any one of the preceding claims, wherein n is 1.

42. A compound according to any one of the preceding claims, wherein R₁ is hydrogen or a substituent selected from
• C₁₋₆-alkyl, aralkyl, C₁₋₆-alkoxy, aryloxy, aralkoxy each of which is optionally substituted with one or more halogens.

43. A compound according to claim 42, wherein R₁ is hydrogen or a substituent selected from
• C₁₋₆-alkyl, C₁₋₆-alkoxy each of which is optionally substituted with one or more halogens.

44. A compound according to claim 43, wherein R₁ is hydrogen.

45. A compound according to any one of the preceding claims, wherein R₂ is hydrogen or C₁₋₆-alkyl.

46. A compound according to claim 45, wherein R₂ is hydrogen.

47. A compound according to any one of the preceding claims, which is:
(Z)-[4-[3-(4-Bromophenyl)-5-phenylpent-2-en-4-ynylsulfanyl]-2-methylphenoxy]acetic acid;
(Z)-[4-[3-(4-Bromophenyl)-6-hydroxyhex-2-en-4-ynylsulfanyl]-2-methylphenoxy]acetic acid;
[4-[3-(Biphenyl-4-yl)-6-hydroxyhex-2-en-4-ynylsulfanyl]-2-methylphenoxy]acetic acid;
(Z)-[4-[3-(Biphenyl-4-yl)-6-hydroxy-6-methylhept-2-en-4-ynylsulfanyl]-2-methylphenoxy]acetic acid;
(Z)-[4-[5-(4-Methoxyphenyl)-3-(biphenyl-4-yl)-pent-2-en-4-ynylsulfanyl]-2-methylphenoxy]-acetic acid;
(Z)-[4-[5-(4-methoxyphenyl)-3-(4-trifluoromethylphenyl)pent-2-en-4-ynylsulfanyl]-2-methyl-phenoxy]acetic acid; or
a salt thereof with a pharmaceutically acceptable acid or base, or any optical isomer or mixture of optical isomers, including a racemic mixture, or any tautomeric forms.

48. A compound according to any one of the preceding claims 1-46, which is:
(Z) [4-(3,5-Diphenyl-pent-2-en-4-ynyloxy)-2-methyl-phenoxy]-acetic acid;
*(Z)*-[4-[3-(4-Bromophenyl)-5-phenylpent-2-en-4-ynyloxy]-2-methylphenoxy]acetic acid;
*(Z)*-[4-[3-(4-Bromophenyl)-5-(pyridin-2-yl)pent-2-en-4-ynyloxy]-2-methylphenoxy]acetic acid;
{4-[3-(4-Bromo-phenyl)-5-(5-methyl-thiophen-2-yl)-pent-2-en-4-ynyloxy]-2-methyl-phenoxy}-acetic acid;
{2-Methyl-4-[3-(2-methyl-benzofuran-5-yl)-5-(5-methyl-thiophen-2-yl)-pent-2-en-4-ynyloxy]-phenoxy}-acetic acid;
{4-[3-(4-Bromo-phenyl)-6-morpholin-4-yl-hex-2-en-4-ynyloxy]-2-methyl-phenoxy}-acetic acid; (2-Methyl-4-{3-[4-(5-methyl-thiophen-2-yl)-phenyl]-5-phenyl-pent-2-en-4-ynyloxy}-phenoxy)-acetic acid; or
a salt thereof with a pharmaceutically acceptable acid or base, or any optical isomer or mixture of optical isomers, including a racemic mixture, or any tautomeric forms.

49. A combination of a compound as defined in any one of claims 1 to 48 and one or more further pharmacologically active substances.

50. A pharmaceutical composition comprising, as an active ingredient, at least one compound according to any one of claims 1-48 or a combination according to claim 49, together with one or more pharmaceutically acceptable carriers or excipients.

51. A pharmaceutical composition according to claim 50 in unit dosage form, comprising from 0.05 mg to 1000 mg, preferably from 0.1 to 500 mg, and especially preferred from 0.5 mg to 200 mg per day of compound according to any one of the claims 1-48.

52. A pharmaceutical composition according to claim 50 or claim 51 for oral, nasal, transdermal, pulmonal, or parenteral administration.

53. A compound as defined in any one of claims 1 to 48, a combination as defined in claim 49, or a composition as defined in any one of claims 50 to 52, for use as a medicament.

54. A compound, combination or composition according to claim 53 for the treatment and/or prevention of conditions mediated by nuclear receptors, in particular the Peroxisome Proliferator-Activated Receptors (PPAR).

55. A compound, combination or composition according to claim 53 for the treatment and/or prevention of Type 1 diabetes; Type 2 diabetes; dyslipidemia; syndrome X, including the metabolic syndrome, i.e. impaired glucose tolerance, insulin resistance, hypertrigyceridaemia and/or obesity; cardiovascular diseases, including atherosclerosis; and hypercholesteremia.

56. A compound, combination or composition according to claim 53 for the treatment and/or prevention of Type 1 diabetes, Type 2 diabetes, impaired glucose tolerance, insulin resistance, or obesity.

57. A compound, combination or composition according to claim 55 or 56 wherein the effective amount of the compound according to any one of claims 1 to 48 is in the range of from 0.05 mg to 1000 mg, preferably from 0.1 to 500 mg, and especially preferred from 0.5 mg to 200 mg per day.

58. Use of a compound according to any one of the claims 1-48 for the preparation of a pharmaceutical composition for the treatment and/or prevention of conditions mediated by nuclear receptors, in particular the Peroxisome Proliferator-Activated Receptors (PPAR).

59. Use of a compound according to any one of the claims 1-48 for the preparation of a pharmaceutical composition for the treatment and/or prevention of Type 1 diabetes; Type 2 diabetes; dyslipidemia; syndrome X, including the metabolic syndrome, i.e. impaired glucose tolerance, insulin resistance, hypertrigyceridaemia and/or obesity; cardiovascular diseases, including atherosclerosis; and hypercholesteremia.

## Patentansprüche

1. Verbindung der allegemeinen Formel (I): 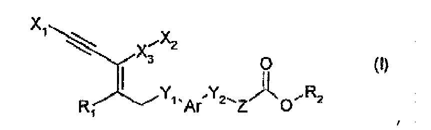
worin X₁ Aryl oder Heteroaryl ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-Cycloalkyl, C₂₋₆-Alkenyl, C₂₋₆-Alkinyl, C₃₋₆-Cycloalkyl-C₁₋₅-Alkyl, Aryl, Aralkyl, Heteroaryl, Heteroaralkyl, C₁₋₆-Alkoxy, C₃₋₆-Cycloalkoxy, C₃₋₆-Cycloalkyl-C₁₋₆-Alkoxy, Aryloxy, Heteroaryloxy, Aralkoxy, Heteroaralkoxy, C₁₋₆-Alkylthio, C₃₋₆-Cycloalkylthio, C₃₋₆-Cycloalkyl-C₁₋₆-Alkylthio, Arylthio, Heteroarylthio, Aryl-C₁₋₆-Alkylthio, Heteroaryl-C₁₋₆-Alkylthio, C₁₋₆-Alkylcarbonyl, C₃₋₆-Cycloalkylcarbonyl, C₃₋₆-Cycloalkyl-C₁₋₆-Alkylcarbonyl, Arylcarbonyl, Heteroarylcarbonyl, C₁₋₆-Alkylsulfonyl, C₃₋₆-Cycloalkylsulfonyl, C₃₋₆-Cycloalkyl-C₁₋₆-Alkylsulfonyl, Arylsulfonyl, Heteroarylsulfonyl, C₁₋₆-Alkylsulfamoyl, Di-(C₁₋₆-Alkyl)sulfamoyl, C₁₋₆-Alkoxycarbonyl, C₃₋₆-Cycloalkoxycarbonyl, C₃₋₆-Cycloalkyl-C₁₋₆-Alkoxycarbonyl, Amino-C₁₋₆-Alkyl, C₁₋₆-Alkylamino-C₁₋₆-Alkyl, Di-(C₁₋₆-Alkyl)amino-C₁₋₆-Alkyl, C₁₋₅-Alkylamido, C₃₋₆-Cycloalkylamido, C₃₋₆-Cycloalkyl-C₁₋₆-Alkylamido, Arylamido, C₁₋₆-Alkylaminocarbonyl, C₃₋₆-Cycloalkylaminocarbonyl, C₃₋₆-Cycloalkyl-C₁₋₆-Alkylaminocarbonyl, Di-(C₁₋₆-Alkyl)aminocarbonyl, Di-(C₃₋₆-Cycloalkyl-C₁₋₆-Alkyl)aminocarbonyl, C₁₋₆-Alkylamino, C₃₋₆-Cycloalkylamino, C₃₋₅-Cycloalkyl-C₁₋₆-Alkylamino, Di-(C₁₋₆-Alkyl)amino, Di-(C₃₋₆-Cycloalkyl)amino oder Di-(C₃₋₆-Cycloalkyl-C₁₋₆-Alkyl)amino, von denen jedes optional mit einem oder mehreren Halogenen, CN und OH substituiert ist; oder
X₁ C₁₋₅-Alkyl, C₃₋₆-Cycloalkyl, C₂₋₆-Alkenyl oder C₃₋₆-Cycloalkyl-C₁₋₅-Alkyl ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-Cycloalkyl, C₂₋₆-Alkenyl, C₂₋₆-Alkinyl, C₃₋₆-Cycloalkyl-C₁₋₆-Alkyl, Aryl, Aralkyl, Heteroaryl, Heteroaralkyl, Heterocyclyl, C₁₋₆-Alkoxy, C₃₋₆-Cycloalkoxy, C₃₋₆-Cycloalkyl-C₁₋₆-Alkoxy, Aryloxy, Heteroaryloxy, Aralkoxy, Heteroaralkoxy, C₁₋₆-Alkylthio, C₃₋₆-Cycloalkylthio, C₃₋₆-Cycloalkyl-C₁₋₆-Alkylthio, Arylthio, Hetexoarylthio, Aryl-C₁₋₆-Alkylthio, Heteroaryl-C₁₋₆-Alkylthio, C₁₋₆-Alkylcarbonyl, C₃₋₆-Cycloalkylcarbonyl, C₃₋₆-Cycloalkyl-C₁₋₆-Alkylcarbonyl, Arylcarbonyl, Heteroarylcarbonyl, C₁₋₆-Alkylsulfonyl, C₃₋₆-Cycloalkylsulfonyl, C₃₋₆-Cycloalkyl-C₁₋₆-Alkylsulfonyl, Arylsulfonyl, Heteroarylsulfonyl, C₁₋₆-Alkylsulfamoyl, Di-(C₁₋₆-Alkyl)sultamoyl, C₁₋₅-Alkoxycarbonyl, C₃₋₆-Cycloalkoxycarbonyl, C₃₋₆-Cycloalkyl-C₁₋₆-Alkoxycarbonyl, Amino-C₁₋₆-Alkyl, C₁₋₆-Alkylamino-C₁₋₆-Akyl, Di-(C₁₋₆-Alkyl)amino-C₁₋₆-Alkyl, C₁₋₆-Alkylamido, C₃₋₆-Cyoloalkylamido, C₃₋₆-Cycloalkyl-C₁₋₆-Alkylamido, Arylamido, C₁₋₆-Alkylaminocarbonyl, C₃₋₆-Cycloalkylaminocarbonyl, C₃₋₆-Cycloalkyl-C₁₋₆-Alkylaminocarbonyl, Di-(C₁₋₆-Alkyl)aminocarbonyl, Di-(C₃₋₆-Cycloalkyl-C₁₋₆-Alkyl)aminocarbonyl, C₁₋₆-Alkylamino, C₃₋₆-Cycloalkylamino, C₃₋₆-Cycloalkyl-C₁₋₆-Alkylamino, Di-(C₁₋₆-Alkyl)amino, Di-(C₃₋₆-Cycloalkyl)amino oder Di-(C₃₋₆-Cycloalkyl-C₁₋₆-Alkyl)amino, von denen jedes optional mit einem oder mehreren Halogenen, CN und OH substituiert ist; oder
X₁ Aralkyl oder Heteroaralkyl ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-Cycloalkyl, C₂₋₆-Alkenyl, C₂₋₆-Alkinyl, Aryl, Aralkyl, Heteroaryl, Heteroaralkyl, C₁₋₆-Alkoxy, C₃₋₆-Cycloalkoxy, Aryloxy, Heteroaryloxy, Aralkoxy, Heteroaralkoxy, C₁₋₆-Alkylthio, Arylthio, Heteroarylthio, Aryl-C₁₋₆-Alkylthio, Heteroaryl-C₁₋₆-Alkylthio, C₃₋₆-Cycloalkylthio, C₁₋₆-Alkylcarbonyl, Arylcarbonyl, C₁₋₆-Alkylsulfonyl, Arylsulfonyl, Heteroarylsulfonyl, C₁₋₆-Alkoxycarbonyl, C₁₋₆-Alkylamido, Arylamido, C₁₋₆-Alkylaminocarbonyl, Di-(C₁₋₆-Alkyl)aminocarbonyl, C₁₋₆-Alkylamino, Di-(C₁₋₆-Alkyl)amino oder C₃₋₆-Cycloalkylamino, von denen jedes optional mit einem oder mehreren Halogenen, CN und OH substituiert ist; und
X₂ Aryl oder Heteroaryl ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-Cycloalkyl, C₂₋₆-Alkenyl, C₂₋₆-Alkinyl, Aryl, Aralkyl, Heteroaryl, Heteroaralkyl, C₁₋₆-Alkoxy, C₃₋₆-Cycloalkoxy, Aryloxy, Heteroaryloxy, Aralkoxy, Heteroaralkoxy, C₁₋₆-Alkylthio, Arylthio, Heteroarylthio, Aryl-C₁₋₆-Alkylthio, Heteroaryl-C₁₋₆-Alkylthio, C₃₋₆-Cycloalkylthio, C₁₋₆-Alkylcarbonayl, Arylcarbonyl, C₁₋₆-Alkylsulfonyl, Arylsulfonyl, Heteroarylsulfonyl, C₁₋₆-Alkoxycarbonyl, C₁₋₆-Alkylamido, Arylamido, C₁₋₆-Alkylaminocarbonyl, Di-(C₁₋₆-Alkyl)aminocarbonyl, C₁₋₆-Alkylamino, Di-(C₁₋₆-Alkyl)amino oder C₃₋₆-Cycloalkylamino, von denen jedes optional mit einem oder mehreren Halogenen, CN und OH substituiert ist; oder
X₂ aus Folgenden ausgewählt ist:
• Wasserstoff, Halogen, Hydroxy, Cyano, Amino oder Carboxy; oder
• C₁₋₅-Alkyl, C₃₋₆-Cycloalkyl, C₂₋₆-Alkenyl, C₂₋₆-Alkinyl, Aralkyl, Heteroaralkyl, C₁₋₆-Alkoxy, C₃₋₆-Cycloalkoxy, Aryloxy, Heteroaryloxy, Aralkoxy, Heteroaralkoxy, C₁₋₆-Alkylthio, Arylthio, Heteroarylthio, Aryl-C₁₋₆-Alkylthio, Hetercaryl-C₁₋₆-Alkylthio, C₃₋₆-Cycloalkylthio, C₁₋₆-Alkylcarbonyl, Arylcarbonyl, C₁₋₆-Alkylsulfonyl, Arylsulfonyl, Heteroarylsulfonyl, C₁₋₆-Alkoxycarbonyl, C₁₋₆-Alkylamido, Arylamido, C₁₋₆-Alkylaminocarbonyl, Di-(C₁₋₆-Alkyl)aminocarbonyl, C₁₋₆-Alkylamin, Di-(C₁₋₆-Alkyl)amino oder C₃₋₆-Cycloalkylamino, von denen jedes optional mit einem oder mehreren Halogenen, CN und OH substituiert ist; und
X₃ Arylen oder Heteroarylen ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-Cycloalkyl, C₂₋₆-Alkenyl, C₂₋₆-Alkinyl, C₁₋₆-Alkoxy, C₃₋₆-Cycloalkoxy, C₁₋₆-Alkylthio, C₃₋₆-Cycloalkylthio, C₁₋₆-Alkylamino, C₁₋₆-Dialkylamino oder C₃₋₆-Cycloalkylamino, von denen jedes optional mit einem oder mehreren Halogenen substituiert ist; und
Ar Phenylen ist, das optional mit einem oder mehreren Substituenten, die aus Folgenden ausgewählt sind, substituiert ist:
• Halogen; oder
• C₁₋₆-Alkyl, das optional mit einem oder mehreren
Halogenen substituiert ist; und
Y₁ O oder S ist; und
Y₂ O oder S ist; und
Z -(CH₂)ₙ- ist, worin n 1, 2 oder 3 ist; und
R₁ Wasserstoff, Halogen oder ein Substituent ist, der aus Folgenden ausgewählt ist:
• C₁₋₆-Alkyl, C₃₋₆-Cycloalkyl, C₂₋₆-Alkenyl, C₂₋₆-Alkinyl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-Cycloalkylthio, von denen jedes optional mit einem oder mehreren Halogenen substituiert ist; und
R₂ Wasserstoff, C₁₋₆-Alkyl, C₃₋₆-Cycloalkyl, C₂₋₆-Alkenyl, C₂₋₆-Alkinyl, C₄₋₆-Alkeninyl oder Aryl ist;
worin "Heterocyclyl" Folgendes darstellt: einen gesättigten 3- bis 12-gliedrige monocyclischen Ring, der ein oder mehrere Heteroatome enthält, die aus Stickstoff, Sauerstoff, Schwefel, S(=O) und S(O)₂ ausgewählt sind, oder einen gesättigten bicyclischen Ring, der ein oder mehrere Heteroatome enthält, die aus Stickstoff, Sauerstoff, Schwefel, S(=O) und S(O)₂ ausgewählt sind, oder einen gesättigten heterocyclischen Ring, der ein oder mehrere Heteroatome enthält, die aus Stickstoff, Sauerstoff, Schwefel, S(=O) und S(O)₂ ausgewählt sind, und eine oder zwei Brücken hat, oder einen gesättigten heterocyclischen Ring, der ein oder mehrere Heteroatome enthält, die aus Stickstoff, Sauerstoff, Schwefel, S(=O) und S(O)₂ ausgewählt sind, und ein oder mehrere Spiroatome enthält; oder
ein pharmazeutisch verträgliches Salz davon oder ein pharmazeutisch verträgliches Solvat davon oder jedwede tautomeren Formen, Stereoisomere, Mischung von Stereoisomeren, einschließlich einer racemischen Mischung, oder Polymorphe.

2. Verbindung nach Anspruch 1, worin X₁ Aryl oder Heteroaryl ist, von denen jedes optional mit einem oder mehreren Substituenten, die aus Folgenden ausgewählt sind, substituiert ist:
• Halogen, Hydroxy, Cyan, Amino oder Carboxy; oder
• C₁₋₆-Alkyl, C₃₋₆-Cycloalkyl, C₂₋₆-Alkenyl, C₂₋₆-Alkinyl, C₃₋₆-Cycloalkyl-C₁₋₆-Alkyl, Aryl, Aralkyl, Heteroaryl, Heteroaralkyl, C₁₋₆-Alkoxy, C₃₋₆-Cycloalkoxy, C₃₋₆-Cycloalkyl-C₁₋₆-Alkoxy, Aryloxy, Heteroaryloxy, Aralkoxy, Heteroaralkoxy, C₁₋₆-Alkylthio, C₃₋₆-Cycloalkylthio, C₃₋₆-cycloalkyl-C₁₋₆-Alkylthio, Arylthio, Heteroarylthio, Aryl-C₁₋₆-Alkylthio, Heteroaryl-C₁₋₆-Alkylthio, C₁₋₆-Alkylcarbonyl, C₃₋₆-Cycloalkylcaybonyl, C₃₋₆-Cycloalkyl-C₁₋₆-Alkylcarbonyl, Arylcarbonyl, Heteroarylcarbonyl, C₁₋₆-Alkylsulfonyl, C₃₋₆-Cycloalkylsulfonyl, C₃₋₆-Cycloalkyl-C₁₋₆-Alkylsulfonyl, Arylsulfonyl, Heteroarylsulfonyl, C₁₋₆-Alkylsulfamoyl, Di-(C₁₋₆-Alkyl)sulfamoyl, C₁₋₆-Alkoxycarbonyl, C₃₋₆-Cycloalkoxycarbonyl, C₃₋₆-Cycloalkyl-C₁₋₆-Alkoxycarbonyl, Amino-C₁₋₆-Alkyl, C₁₋₆-Alkylamino-C₁₋₆-Alkyl, Di-(C₁₋₆-Alkyl)amino-C₁₋₆-Alkyl, C₁₋₆-Alkylamido, C₃₋₆-Cycloalkylamido, C₃₋₆-Cycloalkyl-C₁₋₆-Alkylamido, Arylamido, C₁₋₆-Alkylaininocarbonyl, c₃₋₆-Cycloalkylaminocarbonyl, C₃₋₆-Cycloalkyl-C₁₋₆-Alkylaminocarbonyl, Di-(C₁₋₆-Alkyl)aminocarbonyl, Di-(C₃₋₆-Cycloalkyl-C₁₋₆-Alkyl)aminocarbonyl, C₁₋₆-Alkylamino, C₃₋₆-Cycloalkylamino, C₃₋₆-Cycloalkyl-C₁₋₆-Alkylamin, Di-(C₁₋₆-Alkyl)amino, Di-(C₃₋₆-Cycloalkyl)amino oder Di-(C₃₋₆-Cycloalkyl-C₁₋₆-Alkyl)amino, von denen jedes optional mit einem oder mehreren Halogenen, CN und OH substituiert ist; oder
X₁ C₁₋₆-Alkyl, C₃₋₆-Cycloalkyl, C₂₋₆-Alkenyl oder C₃₋₆-cycloalkyl-C₁₋₆-Alkyl ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-Cycloalkyl, C₂₋₆-Alkenyl, C₂₋₆-Alkinyl, C₃₋₆-Cycloalkyl-C₁₋₅-Alkyl, Aryl, Aralkyl, Heteroaryl, Heteroaralkyl, C₁₋₆-Alkoxy, C₃₋₆-Cyoloalkoxy, C₃₋₆-Cycloalkyl-C₁₋₆-Alkoxy, Aryloxy, Heteroaryloxy, Aralkoxy, Heteroaralkoxy, C₁₋₆-Alkylthio, C₃₋₅-Cycloalkylthio, C₃₋₆-Cycloalkyl-C₁₋₆-Alkylthio, Arylthio, Heteroarylthio, Axyl-C₁₋₆-Alkylthio, Hetercaryl-C₁₋₆-Alkylthio, C₁₋₆-Alkylcarbonyl, C₃₋₆-Cycloalkylcarbonyl, C₃₋₆-Cycloalkyl-C₁₋₆-Alkylcarbonyl, Arylcarbonyl, Heteroarylcarbonyl, C₁₋₆-Alkylsulfonyl, C₃₋₆-Cycloalkylsulfonyl, C₃₋₆-cycloalkyl-C₁₋₆-Alkylsulfonyl, Arylsulfonyl, Heteroarylsulfornyl, C₁₋₆-Alkylsulfamoyl, Di-(C₁₋₆-Alkyl)sulfamoyl, C₁₋₆-Alkoxycarbonyl, C₃₋₆-Cycloalkoxycarbonyl, C₃₋₆-Cycloalkyl-C₁₋₆-Alkoxycarbonyl, Amino-C₁₋₆-Alkyl, C₁₋₆-Alkylamino-C₁₋₆-Alkyl, Di-(C₁₋₆-Alkyl)amino-C₁₋₆-Alkyl, C₁₋₆-Alkylamido, C₃₋₆-Cycloalkylamido, C₃₋₆-Cycloalkyl-C₁₋₆-Alkylamido, Arylamido, C₁₋₆-Alkylaminocarbonyl, C₃₋₆-Cycloalkylaminocarbonyl, C₃₋₆-Cycloalkyl-C₁₋₆-Alkylaminocarbonyl, Di-(C₁₋₆-Alkyl)aminocarbonyl, Di-(C₃₋₆-Cycloalkyl-C₁₋₆-Alkyl)aminocarbonyl, C₁₋₆-Alkylamino, C₃₋₆-Cycloalkylamino, C₃₋₆-Cycloalkyl-C₁₋₆-Alkylamino, Di-(C₁₋₆-Alkyl)amino, Di-(C₃₋₆-Cycloalkyl)amino oder Di-(C₃₋₆-Cycloalkyl-C₁₋₆-Alkyl)amino, von denen jedes optional mit einem oder mehreren Halogenen, CN und OH substituiert ist; oder
X₁ Aralkyl oder Heteroaralkyl ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-cycloalkyl, C₂₋₆-Alkenyl, C₂₋₆-Alkinyl, Aryl, Aralkyl, Heteroaryl, Heteroaralkyl, C₁₋₆-Alkoxy, C₃₋₆-Cycloalkoxy, Aryloxy, Heteroaryloxy, Aralkoxy, Heteroaralkoxy, C₁₋₆-Alkylthio, Arylthio, Heteroarylthio, Aryl-C₁₋₆-Alkylthio, Heteroaryl-C₁₋₆-Alkylthio, C₃₋₆-Cycloalkylthio, C₁₋₆-Alkylcarbonyl, Arylcarbonyl, C₁₋₆-Alkylsulfonyl, Arylsulfonyl, Heteroarylsulfonyl, C₁₋₆-Alkoxycarbonyl, C₁₋₆-Alkylamido, Arylamido, C₁₋₆-Alkylaminocarbonyl, Di-(C₁₋₆-Alkyl)aminocarbonyl, C₁₋₆-Alkylamino, Di-(C₁₋₆-Alkyl)amino oder C₃₋₆-cycloalkylamino, von denen jedes optional mit einem oder mehreren Halogenen, CN und OH substituiert ist; und
X₂ Aryl oder Heteroaryl ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-Cycloalkyl, C₂₋₆-Alkenyl, C₂₋₆-Alkinyl, Aryl, Aralkyl, Heteroaryl, Heteroaralkyl, C₁₋₆-Alkoxy, C₃₋₆-cycloalkoxy, Aryloxy, Heteroaryloxy, Aralkoxy, Heteroaralkoxy, C₁₋₆-Alkylthio, Arylthio, Heteroarylthio, Aryl-C₁₋₆-Alkylthio, Hetercaryl-C₁₋₆-Alkylthio, C₃₋₆-Cycloalkylthio, C₁₋₅-Alkylcarbonyl, Arylcarbonyl, C₁₋₆-Alkylsulfonyl, Arylsulfonyl, Hetexoaxylsulfonyl, C₁₋₆-Alkoxycarbonyl, C₁₋₆-Alkylamido, Arylamido, C₁₋₆-Alkylaminocarbonyl, Di-(C₁₋₆-Alkyl)aminocarbonyl, C₁₋₆-Alkylamino, Di-(C₁₋₆-Alkyl)amirlo oder C₃₋₆-Cycloalkylamino, von denen jedes optional mit einem oder mehreren Halogenen, CN und OH substituiert ist; oder
X₂ aus Folgenden ausgewählt ist:
• Wasserstoff, Halogen, Hydroxy, Cyano, Amino oder Carboxy; oder
• C₁₋₆-Alkyl, C₃₋₆-Cycloalkyl, C₂₋₆-Alkenyl, C₂₋₆-Alkinyl, Aralkyl, Heteroaralkyl, C₁₋₆-Alkoxy, C₃₋₆-Cycloalkoxy, Aryloxy, Heteroaryloxy, Aralkoxy, Heteroaralkdxy, C₁₋₆-Alkylthio, Arylthio, Heteroarylthio, Aryl-C₁₋₆-Alkylthio, Heteroaryl-C₁₋₆-Alkylthio, C₃₋₆-Cycloalkylthio, C₁₋₅-Alkylcarbonyl, Arylcarbonyl, C₁₋₆-Alkylsulfonyl, Arylsulfonyl, Heteroarylsulfonyl, C₁₋₆-Alkoxycarbonyl, C₁₋₆-Alkylamido, Arylamido, C₁₋₆-Alkylaminocarbonyl, Di-(C₁₋₆-Alkyl)aminocarbonyl, C₁₋₆-Alkylamino, Di-(C₁₋₆-Alkyl)amino oder C₃₋₆-Cycloalkylamino, von denen jedes optional mit einem oder mehreren Halogenen, CN und OH substituiert ist; und
X₃ Arylen oder Heteroarylen ist, von denen jedes optional mit einem oder mehreren Substituenten, die aus Folgenden ausgewählt sind, substituiert ist:
• Halogen, Hydroxy, Cyano, Amino oder Carboxy; oder
• C₁₋₆-Alkyl, C₃₋₆-cycloalkyl, C₂₋₆-Alkenyl, C₂₋₅-Alkinyl, C₁₋₆-Alkoxy, C₃₋₆-Cycloalkoxy, C₁₋₆-Alkylthio, C₃₋₆-Cycloalkylthio, C₁₋₆-Alkylamino, C₁₋₆-Dialkylamino oder C₃₋₆-Cycloalkylamino, von denen jedes optional mit einem oder mehreren Halogenen substituiert ist; und
Ar Phenylen ist, das optional mit einem oder mehreren Substituenten, die aus Folgenden ausgewählt sind, substituiert ist:
• Halogen; oder
• C₁₋₆-Alkyl, das optional mit einem oder mehreren Halogenen substituiert ist; und
Y₁ O oder S ist; und
Y₂ O oder S ist; und
Z -(CH₂)ₙ- ist, worin n 1, 2 oder 3 ist; und
R₁ Wasserstoff, Halogen oder ein Substituent ist, der aus Folgenden ausgewählt ist:
• C₁₋₆-Alkyl, C₃₋₆-Cycloalkyl, C₂₋₆-Alkenyl, C₂₋₆-Alkinyl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-Cycloalkylthio, von denen jedes optional mit einem oder mehreren Halogenen substituiert ist; und
R₂ Wasserstoff, C₁₋₆-Alkyl, C₃₋₆-Cycloalkyl, C₂₋₆-Alkenyl, C₂₋₆-Alkinyl, C₄₋₆-Alkeninyl oder Aryl ist; oder
ein pharmazeutisch verträgliches Salz davon oder ein pharmazeutisch verträgliches Solvat davon oder jedwede tautomeren Formen, Stereoisomere, Mischung von Stereoisomeren, einschließlich einer racemischen Mischung, oder Polymorphe.

3. Verbindung nach Anspruch 1 oder 2, worin X₁ Aryl ist, das optional mit einem oder mehreren Substituenten, die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamino oder C₁₋₆-Dialkylamino, von denen jedes optional mit einem oder mehreren Halogenen substituiert ist.

4. Verbindung nach Anspruch 3, worin X₁ Aryl ist das optional mit einem oder mehreren Substituenten, die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio oder C₁₋₆-Dialkylaminocarbonyl, von denen jedes optional mit einem oder mehreren Halogenen substituiert ist.

5. Verbindung nach Anspruch 4, worin X₁ Aryl ist, das optional mit einem oder mehreren Substituenten, die aus Folgenden ausgewählt sind, substituiert ist:
• Halogen; oder
• C₁₋₆-Alkyl, C₁₋₆-Alkoxy oder C₁₋₆-Alkylthio, von denen jedes optional mit einem oder mehreren Halogenen substituiert ist.

6. verbindung nach einem der vorstehenden Ansprüche, worin X₁ Phenyl ist, das optional mit einem oder mehreren Substituenten, die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthlo, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamino oder C₁₋₆-Dialkylamino, von denen jedes optional mit einem oder mehreren Halogenen substituiert ist,

7. Verbindung nach Anspruch 6, worin X₁ Phenyl ist, das optional mit einem oder mehreren Substituenten, die aus Folgenden ausgewählt sind, substituiert ist:
• Halogen; oder
C₁₋₆-Alkyl, C₁₋₆-Alkoxy oder C₁₋₆-Alkylthio, von denen jedes optional mit einem oder mehreren Halogenen substituiert ist.

8. Verbindung nach einem der vorstehenden Ansprüche, worin X₁ Phenyl ist.

9. Verbindung nach Anspruch 1 oder 2, worin X₁ Heteroaryl ist, das optional mit einem oder mehreren Substituenten, die aus Folgenden ausgewählt sind, substituiert ist:
• Halogen Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamino oder C₁₋₆-Dialkylamino, von denen jedes optional mit einem oder mehreren Halogenen substituiert ist.

10. Verbindung nach Anspruch 9, worin X₁ Heteroaryl ist, das optional mit einem oder mehreren Substituenten die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio oder C₁₋₆-Dialkylaminocarbonyl, von denen jedes optional mit einem oder mehreren Halogenen substituiert ist.

11. Verbindung nach Anspruch 10, worin X₁ Heteroaryl ist, das optional mit einem oder mehreren Substituenten, die aus Folgenden ausgewählt sind, substituiert ist:
• Halogen; oder
• C₁₋₆-Alkyl, C₁₋₆-Alkoxy oder C₁₋₆-Alkylthio, von denen jedes optional mit einem oder mehreren Halogenen substituiert ist.

12. Verbindung nach Anspruch 1, worin X₁ C₁₋₅-Alkyl ist, das optional mit einem oder mehreren Substituenten, die aus Folgenden ausgewählt sind, substituiert ist:
• Halogen oder Hydroxy; oder
• Aryl, Heteroaryl, Heterocyclyl, C₁₋₆-Alkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamino, C₁₋₆-Dialkylamino oder C₃₋₆-Cycloalkylamino, von denen jedes optional mit einem oder mehreren Halogenen substituiert ist.

13. Verbindung nach Anspruch 12, worin X₁ C₁₋₆-Alkyl ist, das optional mit einem oder mehreren Substituenten die aus Folgenden ausgewählt sind, substituiert ist:
• Halogen oder Hydroxy; oder
• Aryl, Heteroaryl, C₁₋₆-Alkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₅-Alkylamino, C₁₋₆-Dialkylamino oder C₃₋₆-Cycloalkylamino, von denen jedes optional mit einem oder mehreren Halogenen substituiert ist.

14. Verbindung nach Anspruch 13, worin X₁ C₁₋₆-Alkyl ist, das optional mit einem oder mehreren Substituenten, die aus Folgenden ausgewählt sind, substituiert ist:
• Halogen oder Hydroxy; oder
• Aryl, Heteroaryl, C₁₋₆-Alkoxy, C₁₋₆-Alkylthio, C₁₋₆-Alkylcarbonyl, C₁₋₆-Alkylsulfonyl, C₁₋₆-Alkylamido, C₁₋₆-Alkylaminocarbonyl, C₁₋₆-Dialkylaminocarbonyl, C₁₋₆-Alkylamino, C₁₋₆-Dialkylamino oder C₃₋₆-Cycloalkylamino, von denen jedes optional mit einem oder mehreren Halogenen substituiert ist.

15. Verbindung nach Anspruch 14, worin X₁ C₁₋₆-Alkyl ist, das optional mit einem oder mehreren Substituenten die aus Halogen oder Hydroxy ausgewählt sind, substituiert ist.

16. Verbindung nach Anspruch 1 oder 2, worin X₁ C₃₋₆-Cycloalkyl ist, das optional mit einem oder mehreren Substituenten, die aus Folgenden ausgewählt sind, substituiert ist:
• Halogen oder Hydroxy; oder
• Aryl, Heteroaryl, C₁₋₆-Alkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamin, C₁₋₆-Dialkylamino oder C₃₋₆-Cycloalkylamino, von denen jedes optional mit einem oder mehreren Halogenen substituiert ist.

17. Verbindung nach Anspruch 16, worin X₁ C₃₋₆-Cycloalkyl ist, das optional mit einem oder mehreren Substituenten, die aus Folgenden ausgewählt sind, substituiert ist:
• Halogen oder Hydroxy; oder
• Aryl, Heteroaryl, C₁₋₆-Alkoxy, C₁₋₆-Alkylthio, C₁₋₆-Alkylcarbonyl, C₁₋₆-Alkylsulfonyl, C₁₋₆-Alkylamido, C₁₋₅-Alkylaminocarbonyl, C₁₋₆-Dialkylaminocarbonyl, C₁₋₆-Alkylamino, C₁₋₆-Dialkylamino oder C₃₋₆-Cycloalkylamino, von denen jedes optional mit einem oder mehreren Halogenen substituiert ist.

18. Verbindung nach Anspruch 17, worin X₁ C₃₋₆-Cycloalkyl ist, das optional mit einem oder mehreren Substituenten, die aus Halogen oder Hydroxy ausgewählt sind, substituiert ist.

19. Verbindung nach Anspruch 1 oder 2, worin X₁ Aralkyl oder Heteroaralkyl ist, von denen jedes optional mit einem oder mehreren Substituenten, die aus Folgenden ausgewählt sind, substituiert ist:
• Halogen oder Hydroxy; oder
• C₁₋₆-Alkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamino, C₁₋₆-Dialkylamino oder C₃₋₆-Cycloalkylamino, von denen jedes optional mit einem oder mehreren Halogenen substituiert ist.

20. Verbindung nach Anspruch 19, worin X₁ Aralkyl oder Heteroaralkyl ist, von denen jedes optional mit einem oder mehreren Substituenten, die aus Folgenden ausgewählt sind, substituiert ist:
• Halogen oder Hydroxy; oder
• C₁₋₆-Alkoxy, das optional mit einem oder mehreren Halogenen substituiert ist.

21. Verbindung nach einem der vorstehenden Ansprüche, worin X₂ Aryl ist, das optional mit einem oder mehreren Substituenten, die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamino oder C₁₋₆-Dialkylamino, von denen jedes optional mit einem oder mehreren Halogenen substituiert ist.

22. Verbindung nach Anspruch 21, worin X₂ Aryl ist, das optional mit einem oder mehreren Substituenten, die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio oder C₁₋₆-Dialkylaminocarbonyl, von denen jedes optional mit einem oder mehreren Halogenen substituiert ist.

23. Verbindung nach Anspruch 22, worin X₂ Aryl ist, das optional mit einem oder mehreren Substituenten, die aus Folgenden ausgewählt sind, substituiert ist:
• Halogen; oder
• C₁₋₆-Alkyl, C₁₋₆-Alkoxy oder C₁₋₆-Alkylthio, von denen jedes optional mit einem oder mehreren Halogenen substituiert ist.

24. Verbindung nach einem der vorstehenden Ansprüche 21-23, worin X₂ Phenyl ist, das optional mit einem oder mehreren Substituenten, die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamino oder C₁₋₆-Dialkylamino, von denen jedes optional mit einem oder mehreren Halogenen substituiert ist.

25. Verbindung nach Anspruch 24, worin X₂ Phenyl ist, das optional mit einem oder mehreren Substituenten, die aus Folgenden ausgewählt sind, substituiert ist:
• Halogen; oder
• C₁₋₆-Alkyl, C₁₋₆-Alkoxy oder C₁₋₆-Alkylthio, von denen jedes optional mit einem oder mehreren Halogenen substituiert ist.

26. Verbindung nach Anspruch 25, worin X₂ Phenyl ist.

27. Verbindung nach einem der Ansprüche 1-20, worin X₂ Heteroaryl ist, das optional mit einem oder mehreren Substituenten, die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Dialkylaminocarbonyl, C₁₋₆-Alkylamino oder C₁₋₆-Dialkylamino, von denen jedes optional mit einem oder mehreren Halogenen substituiert ist.

28. Verbindung nach Anspruch 27, worin X₂ Heteroaryl ist, das optional mit einem oder mehreren Substituenten, die aus Folgenden ausgewählt sind, substituiert ist:
• Halogen, Hydroxy; oder
• C₁₋₆-Alkyl, Aryl, Heteroaryl, C₁₋₆-Alkoxy, Aryloxy, Aralkoxy, Heteroaralkoxy, C₁₋₆-Alkylthio, Arylthio oder C₁₋₆-Dialkylaminocarbonyl, von denen jedes optional mit einem oder mehreren Halogenen substituiert ist.

29. Verbindung nach Anspruch 28, worin X₂ Heteroaryl ist, das optional mit einem oder mehreren Substituenten die aus Folgenden ausgewählt sind, substituiert ist:
• Halogen; oder
• C₁₋₆-Alkyl, C₁₋₆-Alkoxy oder C₁₋₆-Alkylthio, von denen jedes optional mit einem oder mehreren Halogenen substituiert ist.

30. Verbindung nach einem der vorstehenden Ansprüche 1-20, worin X₂ Wasserstoff, Halogen, Hydroxy oder Cyano; oder Folgendes ist:
• C₁₋₆-Alkyl, Aralkyl oder Heteroaralkyl, von denen jedes optional mit einem oder mehreren Halogenen, CN und OH substituiert ist.

31. Verbindung nach Anspruch 30, worin X₂ Halogen oder Hydroxy ist; oder C₁₋₆-Alkyl ist, das optional mit einem oder mehreren Halogenen substituiert ist.

32. Verbindung nach Anspruch 31, worin X₂ Halogen ist.

33. Verbindung nach einem der vorstehenden Ansprüche, worin X₃ Arylen ist, das optional mit einem oder mehreren Substituenten, die aus Folgenden ausgewählt sind, substituiert ist:
• Halogen oder
• C₁₋₆-Alkyl, C₁₋₆-Alkoxy oder C₁₋₆-Alkylthio, von denen jedes optional mit einem oder mehreren Halogenen substituiert ist.

34. Verbindung nach Anspruch 33, worin X₃ Phenylen ist, das optional mit einem oder mehreren Substituenten, die aus Folgenden ausgewählt sind, substituiert ist:
• Halogen oder
• C₁₋₆-Alkyl, C₁₋₆-Alkoxy oder C₁₋₆-Alkylthio, von denen jedes optional mit einem oder mehreren Halogenen substituiert ist.

35. Verbindung nach Anspruch 34, worin X₃ Phenylen ist, das optional mit einem oder mehreren Substituenten substituiert ist, die aus Halogen oder C₁₋₆-Alkyl, das optional mit einem oder mehreren Halogenen substituiert ist, ausgewählt sind.

36. Verbindung nach Anspruch 35, worin x₃ Phenylen ist.

37. Verbindung nach einem der vorstehenden Ansprüche 1-32, worin X₃ Heteroarylen ist, das optional mit einem oder mehreren Substituenten, die aus Folgenden ausgewählt sind, substituiert ist:
• Halogen oder
• C₁₋₆-Alkyl, C₁₋₆-Alkoxy oder C₁₋₆-Alkylthio, von denen jedes optional mit einem oder mehreren Halogenen substituiert ist.

38. Verbindung nach Anspruch 37, worin X₃ Heteroarylen ist, das optional mit einem oder mehreren Substituenten substituiert ist, die aus Halogen oder C₁₋₆-Alkyl, das optional mit einem oder mehreren Halogenen substituiert ist, ausgewählt sind.

39. Verbindung nach einem der vorstehenden Ansprüche, worin Y₁ S ist.

40. Verbindung nach einem der vorstehenden Ansprüche, worin Y₂ O ist.

41. Verbindung nach einem der vorstehenden Ansprüche, worin n 1 ist.

42. Verbindung nach einem der vorstehenden Ansprüche, worin R₁ Wasserstoff oder ein Substituent ist, der aus Folgenden ausgewählt ist:
• C₁₋₆-Alkyl, Aralkyl, C₁₋₆-Alkoxy, Aryloxy, Aralkoxy, von denen jedes optional mit einem oder mehreren Halogenen substituiert ist.

43. Verbindung nach Anspruch 42, worin R₁ Wasserstoff oder ein Substituent ist, der aus Folgenden ausgewählt ist:
• C₁₋₆-Alkyl, C₁₋₆-Alkoxy, von denen jedes optional mit einem oder mehreren Halogenen substituiert ist.

44. Verbindung nach Anspruch 43, worin R₁ Wasserstoff ist.

45. Verbindung nach einem der vorstehenden Ansprüche, worin R₂ Wasserstoff oder C₁₋₆-Alkyl ist.

46. Verbindung nach Anspruch 45, worin R₂ Wasserstoff ist.

47. Verbindung nach einem der vorstehenden Ansprüche, die
(Z)-[4-[3-(4-Bromphenyl)-5-phenylpent-2-en-4-inylsulfanyl]-2-methylphenoxy]essigsäure;
(Z)-[4-[3-(4-Bromphenyl)-6-hydroxyhex-2-en-4-inylsulfanyl]-2-methylphenoxy]essigsäure;
[4-[3-(Biphenyl-4-yl)-6-hydroxyhex-2-en-4-inylsulfanyl]-2-methylphenoxy]essigsäure;
(Z)-[4-[3-(Biphenyl-4-yl)-6-hydroxy-6-methylhept-2-en-4-inylsulfanyl]-2-methylphenoxy]essigsäure;
(Z)-[4-[5-(4-Methoxyphenyl)-3-(biphenyl-4-yl)pent-2-en-4-inylsulfanyl]-2-methylphenoxy]essigsäure;
(Z)-[4-[5-(4-Methoxyphenyl)-3-(4-trifluormethylphenyl)pent-2-en-4-inylsulfanyl]-2-methylphenoxy]essigsäure ist; oder
ein Salz davon mit einer pharmazeutisch verträglichen Säure oder Base oder jedwedes optische Isomer oder jedwede Mischung von optischen Isomeren, einschließlich einer racemischen Mischung, oder jedwede tautomeren Formen.

48. Verbindung nach einem der vorstehenden Ansprüche 1-46, die
(Z)-[4-(3,5-Diphenylpent-2-en-4-inyloxy)-2-methylphenoxy]essigsäure;
(Z)-[4-[3-(4-Bromphenyl)-5-phenylpent-2-en-4-inyloxy]-2-methylphenoxy]essigsäure;
(Z)-[4-[3-(4-Bromphenyl)-5-(pyridin-2-yl)pent-2-en-4-inyloxy]-2-methylphenoxy]essigsäure;
{4-[3-(4-Bromphenyl)-5-(5-methylthiophen-2-yl)pent-2-en-4-inyloxy]-2-methylphenoxy}essigsäure;
{2-Methyl-4-[3-(2-methylbenzofuran-5-yl)-5-(5-methylthiophen-2-yl)pent-2-en-4-inyloxy]phenoxy}essigsäure;
{4-[3-(4-Bromphenyl)-6-morpholin-4-ylhex-2-en-4-inyloxy]-2-methylphenoxy}essigsäure;
(2-Methyl-4-{3-[4-(5-methylthiophen-2-yl)phenyl]-5-phenylpent-2-en-4-inyloxy}phenoxy)essigsäure ist; oder
ein Salz davon mit einer pharmazeutisch verträglichen Säure oder Base oder jedwedes optische Isomer oder jedwede Mischung von optischen Isomeren, einschließlich einer racemischen Mischung, oder jedwede tautomeren Formen.

49. Kombination aus einer Verbindung, wie in einem der Ansprüche 1 bis 48 definiert, und einem oder mehreren weiteren pharmakologischen Wirkstoffen.

50. Pharmazeutische Zusammensetzung, die, als Wirkstoff, mindestens eine Verbindung nach einem der Ansprüche 1-48 oder eine Kombination nach Anspruch 49 zusammen mit einem oder mehreren pharmazeutisch verträglichen Trägern oder Hilfsstoffen umfasst.

51. Pharmazeutische Zusammensetzung nach Anspruch 50 in Einheitsdosierungsform, die von 0,05 mg bis 1000 mg, bevorzugt von 0,1 bis 500 mg und besonders bevorzugt von 0,5 mg bis 200 mg pro Tag der Verbindung nach einem der Ansprüche 1-48 umfasst.

52. Pharmazeutische Zusammensetzung nach Anspruch 50 oder Anspruch 51 zur oralen, nasalen, transdermalen, pulmonalen oder parenteralen Verabreichung.

53. Verbindung, wie in einem der Ansprüche 1 bis 48 definiert, eine Kombination, wie in Anspruch 49 definiert, oder eine Zusammensetzung, wie in einem der Ansprüche 50 bis 52 definiert, zur Verwendung als Medikament.

54. Verbindung, Kombination oder Zusammensetzung nach Anspruch 53 zur Behandlung und/oder Prävention von Zuständen, die durch nukleäre Rezeptoren, insbesondere die Peroxisome Proliferator-Activated Receptors (PPAR), vermittelt werden.

55. Verbindung, Kombination oder Zusammensetzung nach Anspruch 53 zur Behandlung und/oder Prävention von Typ-1-Diabetes; Typ-2-Diabetes; Dyslipidämie; Syndrom X, einschließlich des metabolischen Syndroms, d. h. gestörter Glucosetoleranz, Insulinresistenz, Hypertriglyzeridämie und/oder Adipositas; kardiovaskulären Erkrankungen, einschließlich Atherosklerose; und Hypercholesterinämie.

56. Verbindung, Kombination oder Zusammensetzung nach Anspruch 53 zur Behandlung und/oder Prävention von Typ-1-Diabetes, Typ-2-Diabetes, gestörter Glucosetoleranz, Insulinresistenz oder Adipositas.

57. Verbindung, Kombination oder Zusammensetzung nach Anspruch 55 oder 56, worin die wirksame Menge der Verbindung nach einem der Ansprüche 1 bis 48 im Bereich von 0,05 mg bis 1000 mg, bevorzugt von 0,1 bis 500 mg und besonders bevorzugt von 0,5 mg bis 200 mg pro Tag liegt.

58. Verwendung einer Verbindung nach einem der Ansprüche 1-48 zur Herstellung einer pharmazeutischen Zusammensetzung zur Behandlung und/oder Prävention von Zuständen, die durch nukleäre Rezeptoren, insbesondere die Peroxisome Proliferator-Activated Receptors (PPAR), vermittelt werden.

59. Verwendung einer Verbindung nach einem der Ansprüche 1-48 zur Herstellung einer pharmazeutischen Zusammensetzung zur Behandlung und/oder Prävention von Typ-1-Diabetes; Typ-2-Diabetes; Dyslipidämie; Syndrom X, einschließlich des metabolischen Syndroms, d. h. gestörter Glucosetoleranz, Insulinresistenz, Hypertriglyzeridämie und/oder Adipositas; kardiovaskulären Erkrankungen, einschließlich Atherosklerose; und Hypercholesterinämie.

## Revendications

1. Composé de la formule générale (I) : 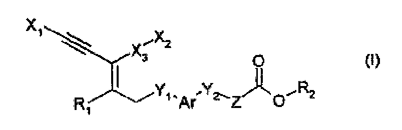 dans laquelle X₁ est aryle ou hétéroaryle, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, C₃₋₆-cycloalkyl-C₁₋₆-alkyle, aryle, aralkyle, hétéroaryle, hétéroaralkyle, alkoxy en C₁₋₆, cycloalkoxy en C₃₋₆, C₃₋₆-cycloalkylC₁₋₆-alkoxy, aryloxy, hétéroaryloxy, aralkoxy, hétéroaralkoxy, alkylthio en C₁₋₆, cycloalkylthio en C₃₋₆, C₃₋₆-cycloalkyl-C₁₋₆-alkylthio, arylthio, hétéroarylthio, aryl-C₁₋₆-alkylthio, hétéroaryl-C₁₋₆-alkylthio, alkylcarbonyle en C₁₋₆, cycloalkylcarbonyle en C₃₋₆, C₃₋₆-cycloalkyl-C₁₋₆-alkylcarbonyle, arylcarbonyle, hétéroarylcarbonyle, alkylsulfonyle en C₁₋₆, cycloalkylsulfonyle en C₃₋₆, C₃₋₆-cycloalkyl-C₁₋₆-alkylsulfonyle, arylsulfonyle, hétéroarylsulfonyle, alkylsulfamoyle en C₁₋₆, di-(C₁₋₆-alkyl)sulfamoyle, alkoxycarbonyle en C₁₋₆, cycloalkoxycarbonyle en C₃₋₆, C₃₋₆-cycloalkyl-C₁₋₆-alkoxycarbonyle, amino-C₁₋₆-alkyle, C₁₋₆-alkylamino-C₁₋₆-alkyle, di-(C₁₋₆-alkyl)amino-C₁₋₆-alkyle, alkylamido en C₁₋₆, cycloalkylamido en C₃₋₆, C₃₋₆-cycloalkyl-C₁₋₆-alkylamido, arylamido, alkylaminocarbonyle en C₁₋₆, cycloalkylaminocarbonyle en C₃₋₆, C₃₋₆-cycloalkyl-C₁₋₆-alkylaminocarbonyle, di-(C₁₋₆-alkyl)aminocarbonyle, di-(C₃₋₆-cycloalkyl-C₁₋₆-alkyl)aminocarbonyle, alkylamino en C₁₋₆, cycloalkylamino en C₃₋₆, C₃₋₆-cycloalkyl-C₁₋₆-alkylamino, di-(C₁₋₆-alkyl)amino, di-(C₃₋₆-cycloalkyl)amino ou di-(C₃₋₆-cycloalkyl-C₁₋₆-alkyl)amino, chacun desquels est éventuellement substitué par un ou plusieurs halogènes, CN et OH ; ou
X₁ est alkyle en C₁₋₆, cycloalkyle en C₃₋₆, alcényle en C₂₋₆ ou C₃₋₆-cycloalkyl-C₁₋₆-aryle, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, C₃₋₆-cycloalkyl-C₁₋₆-alkyle, aryle, aralkyle, hétéroaryle, hétéroaralkyle, hétérocyclyle, alkoxy en C₁₋₆, cycloalkoxy en C₃₋₆, C₃₋₆-cyclcalkylC₁₋₆-alkoxy, aryloxy, hétéroaryloxy, aralkoxy, hétéroaralkoxy, alkylthio en C₁₋₆, cycloalkylthio en C₃₋₆, C₃₋₆-cycloalkyl-C₁₋₆-alkylthio, arylthio, hétéroarylthio, aryl-C₁₋₆-alkylthio, hétércaryl-C₁₋₆-alkylthio, alkylcarbonyle en C₁₋₆, cycloalkylcarbonyle en C₃₋₆, C₃₋₆-cycloalkyl-C₁₋₆-alkylcarbonyle, arylcarbonyle, hétéroarylcarbonyle, alkylsulfonyle en C₁₋₆, cycloalkylsulfonyle en C₃₋₆, C₃₋₆-cycloalkyl-C₁₋₆-alkylsulfonyle, arylsulfonyle, hétéroarylsulfonyle, alkylsulfamoyle en C₁₋₆, di-(C₁₋₆-alkyl)sulfamoyle, alkoxycarbonyle en C₁₋₆, cycloalkoxycarbonyle en C₃₋₆, C₃₋₆-cycloalkyl-C₁₋₆-alkoxycarbonyle, amino-C₁₋₆-alkyle, C₁₋₆-alkylamino-C₁₋₆-alkyle, di-(C₁₋₆-alkyl)amino-C₁₋₆-alkyle, alkylamido en C₁₋₆, cycloalkylamido en C₃₋₅, C₃₋₆-cycloalkyl-C₁₋₆-alkylamido, arylamido, alkylaminocarbonyle en C₁₋₆, cycloalkylaminocarbonyle en C₃₋₆, C₃₋₆-cycloalkyl-C₁₋₆-alkylaminocarbonyle, di-(C₁₋₆-alkyl)aminocarbonyle, di-(C₃₋₆-cycloalkyl-C₁₋₆-alkyl)aminocarbonyle, alkylamino en C₁₋₆, cycloalkylamino en C₃₋₆, C₃₋₆-cycloalkyl-C₁₋₆-alkylamino, di-(C₁₋₆-alkyl)amino, di-(C₃₋₆-cycloalkyl)amino ou di-(C₃₋₆-cycloalkyl-C₁₋₆-alkyl)amino, chacun desquels est éventuellement substitué par un ou plusieurs halogènes, CN et OH ; ou
X₁ est aralkyle ou hétéroaralkyle, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, aryle, aralkyle, hétéroaryle, hétéroaralkyle, alkoxy en C₁₋₆, cycloalkoxy en C₃₋₆, aryloxy, hétéroaryloxy, aralkoxy, hétéroaralkoxy, alkylthio en C₁₋₆, arylthio, hétéroarylthio, aryl-C₁₋₆-alkylthio, hétéroaryl-C₁₋₆-alkylthio, cycloalkylthio en C₃₋₆, alkylcarbonyle en C₁₋₆, arylcarbonyle, alkylsulfonyle en C₁₋₆, arylsulfonyle, hétéroarylsulfonyle, alkoxycarbonyle en C₁₋₆, alkylamido en C₁₋₆, arylamido, alkylaminocarbonyle en C₁₋₆, di-(C₁₋₆-alkyl)aminocarbonyle, alkylamino en C₁₋₆, di-(C₁₋₆-alkyl)amino ou cycloalkylamino en C₃₋₆, chacun desquels est éventuellement substitué par un ou plusieurs halogènes, CN et OH ; et
X₂ est aryle ou hétéroaryle, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, aryle, aralkyle, hétéroaryle, hétéroaralkyle, alkoxy en C₁₋₆, cycloalkoxy en C₃₋₆, aryloxy, hétéroaryloxy, aralkoxy, hétéroaralkoxy, alkylthio en C₁₋₆, arylthio, hétéroarylthio, aryl-C₁₋₆-alkylthio, hétéroaryl-C₁₋₆-alkylthio, cycloalkylthio en C₃₋₆, alkylcarbonyle en C₁₋₆, arylcarbonyle, alkylsulfonyle en C₁₋₆, arylsulfonyle, hétéroarylsulfonyle, alkoxycarbonyle en C₁₋₆, alkylamido en C₁₋₆, arylamido, alkylaminocarbonyle en C₁₋₆, di-(C₁₋₆-alkyl)aminocarbonyle, alkylamino en C₁₋₆, di-(C₁₋₆-alkyl)amino ou cycloalkylamino en C₃₋₆, chacun desquels est éventuellement substitué par un ou plusieurs halogènes, CN et OH ; ou
X₂ est sélectionné parmi
• hydrogène, halogène, hydroxy, cyano, amino ou carboxy ; ou
• alkyle en C₁₋₆, cycloalkyle en C₃₋₆, alcényle en C₂₋₆, alcynyle en C₂₋₆, aralkyle, hétéroaralkyle, alkoxy en C₁₋₆, cycloalkoxy en C₃₋₆, aryloxy, hétéroaryloxy, aralkoxy, hétéroaralkoxy, alkylthio en C₁₋₆, arylthio, hétéroarylthio, aryl-C₁₋₆-alkylthio, hétéroaryl-C₁₋₆-alkylthio, cycloalkylthio en C₃₋₆, alkylcarbonyle en C₁₋₆, arylcarbonyle, alkylsulfonyle en C₁₋₆, arylsulfonyle, hétéroarylsulfonyle, alkoxycarbonyle en C₁₋₆, alkylamido en C₁₋₆, arylamido, alkylaminocarbonyle en C₁₋₆, di- (C₁₋₆-alkyl)aminocarbonyle, alkylamino en C₁₋₆, di-(C₁₋₆-alkyl)amino ou cycloalkylamino en C₃₋₆, chacun desquels est éventuellement substitué par un ou plusieurs halogènes, CN et OH ; et
X₃ est arylène ou hétéroarylène, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, alkoxy en C₁₋₆, cycloalkoxy en C₃₋₆, alkylthio en C₁₋₆, cycloalkylthio en C₃₋₆, alkylamino en C₁₋₆, dialkylamino en C₁₋₆ ou cycloalkylamino en C₃₋₆, chacun desquels est éventuellement substitué par un ou plusieurs halogènes ; et
Ar est phénylène, qui est éventuellement substitué par un ou plusieurs substituants sélectionnés parmi
• halogène; ou
• alkyle en C₁₋₆, éventuellement substitué par un ou plusieurs halogènes ; et
Y₁ est O ou s ; et
Y₂ est O ou S ; et
Z est -(CH₂)ₙ-, dans lequel n est 1, 2, ou 3 ; et
R₁ est hydrogène, halogène ou un substituant sélectionné parmi
• alkyle en C₁₋₆, cycloalkyle en C₃₋₆, alcényle en C₂₋₆, alcynyle en C₂₋₆, aralkyle, hétéroaralkyle, alkoxy en C₁₋₆, cycloalkoxy en C₃₋₆, aryloxy, aralkoxy, hétéroaralkoxy, alkylthio en C₁₋₆, arylthio ou cycloalkylthio en C₃₋₆, chacun desquels est éventuellement substitué par un ou plusieurs halogènes ; et
R₂ est hydrogène, alkyle en C₁₋₆, cycloalkyle en C₃₋₆, alcényle en C₂₋₆, alcynyle en C₂₋₆, alcénynyle en C₄₋₆ ou aryle ;
dans lequel « hétércyclyle » représente un noyau monocyclique saturé à 3 à 12 chaînons contenant un ou plusieurs hétéroatomes sélectionnés parmi azote, oxygène, soufre, S(=O) et S(=O)₂ ou un noyau bicyclique saturé contenant un ou plusieurs hétéroatomes sélectionnés parmi azote, oxygène, soufre, S(=O) et S(=O)₂, ou un noyau hétérocyclique saturé contenant un ou plusieurs hétéroatomes sélectionnés parmi azote, oxygène, soufre S(=O) et S(=O)₂ et ayant un ou deux ponts, ou un noyau hétérocyclique saturé contenant un ou plusieurs hétéroatomes sélectionnés parmi azote, oxygène, so°afre, S(=O) et S(=O)₂ et contenant un ou plusieurs atomes spiraniques ; ou
un sel pharmaceutiquement acceptable de ceux-ci, ou un solvate pharmaceutiquement acceptable de ceux-ci, ou toutes formes tautomères, stéréoisomères, mélanges de stéréoisomères y compris un mélange racémique, ou polymorphes.

2. Composé selon la revendication 1, dans lequel X₁ est aryle ou hétéroaryle, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, C₃₋₆-cycloalkyl-C₁₋₆-alkyle, aryle, aralkyle, hétéroaryle, hétéroaralkyle, alkoxy en C₁₋₆, cycloalkoxy en C₃₋₆, C₃₋₅-cycloalkylC₁₋₆-alkoxy, aryloxy, hétéroaryloxy, aralkoxy, hétéroaralkoxy, alkylthio en C₁₋₆, cycloalkylthio en C₃₋₆, C₃₋₆-cycloalkyl-C₁₋₆-alkylthio, arylthio, hétéroarylthio, aryl-C₁₋₆-alkylthio, hétéroaryl-C₁₋₆-alkylthio, alkylcarbonyle en C₁₋₆, cycloalkylcarbonyle en C₃₋₆, C₃₋₆-cycloalkyl-C₁₋₆-alkylcarbonyle, arylcarbonyle, hétéroarylcarbonyle, alkylsulfonyle en C₁₋₆, cycloalkylsulfonyle en C₃₋₆, C₃₋₆-cycloalkyl-C₁₋₆-alkylsulfonyle, arylsulfonyle, hétéroarylsulfonyle, alkylsulfamoyle en C₁₋₆, di-(C₁₋₆-alkyl)sulfambyle, alkoxycarbonyle en C₁₋₆, cycloalkoxycarbonyle en C₃₋₆, C₃₋₆-cycloalkyl-C₁₋₆-alkoxycarbonyle, amino-C₁₋₆-alkyle, C₁₋₆-alkylamino-C₁₋₆-alkyle, di-(C₁₋₆-alkyl)amino-C₁₋₆-alkyle, alkylamido en C₁₋₆, cycloalkylamido en C₃₋₆, C₃₋₆-cycloalkyl-C₁₋₆-alkylamido, arylamido, alkylaminocarbonyle en C₁₋₆, cycloalkylaminocarbonyle en C₃₋₆, C₃₋₆-cycloalkyl-C₁₋₆-alkylaminocarbonyle, di-(C₁₋₆-alkyl)aminocarbonyle, di-(C₃₋₆-cycloalkyl-C₁₋₆-alkyl)aminocarbonyle, alkylamino en C₁₋₆, cycloalkylamino en C₃₋₆, C₃₋₆-cycloalkyl-C₁₋₆-alkylamino, di-(C₁₋₆-alkyl)amino, di-(C₃₋₆-cycloalkyl)amino ou di-(C₃₋₆-cycloalkyl-C₁₋₆-alkyl)amino, chacun desquels est éventuellement substitué par un ou plusieurs halogènes, CN et OH ; ou
X₁ est alkyle en C₁₋₆, cycloalkyle en C₃₋₆, alcényle, en C₂₋₆ ou C₃₋₆-cycloalkyl-C₁₋₆-alkyle, chacun desquels est éventuellement substitué par un ou plusieurs substituants sélectionnes parmi
• halogène, hydroxy, cyano, amino ou carboxy ; ou
• alkyle en C₁₋₆, cycloalkyle en C₃₋₆, alcényle en C₂₋₆, alcynyle en C₂₋₆, C₃₋₆-cycloalkyl-C₁₋₆-alkyle, aryle, aralkyle, hétéroaryle, hétéroaralkyle, alkoxy en C₁₋₆, cycloalkoxy en C₃₋₆, C₃₋₆-cycloalkylC₁₋₆-alkoxy, aryloxy, hétéroaryloxy, aralkoxy, hétéroaralkoxy, alkylthio en C₁₋₆, cycloalkylthio en C₃₋₆, C₃₋₆-cycloalkyl-C₁₋₆-alkylthio, aryltkrio, hétéroarylthio, aryl-C₁₋₆-alkylthio, hétéroaryl-C₁₋₆-alkylthio, alkylcarbonyle en C₁₋₆, cycloalkylcarbonyle en C₃₋₆, C₃₋₆-cycloalkyl-C₁₋₆-alkylcarbonyle, arylcarbonyle, hétéroarylcarbonyle, alkylsulfonyle en C₁₋₆, cycloalkylsulfonyle en C₃₋₆, C₃₋₆-cycloalkyl-C₁₋₆-alkylsulfonyle, arylsulfonyle, hétéroarylsulfonyle, alkylsulfamoyle en C₁₋₆, di-(C₁₋₆-alkyl)sulfaryoyle, alkoxycarbonyle en C₁₋₆, cycloalkoxycarbonyle en C₃₋₆, C₃₋₆-cycloalkyl-C₁₋₆-alkoxycarbonyle, amino-C₁₋₆-alkyle, C₁₋₆-alkylamino-C₁₋₆-alkyle, di-(C₁₋₆-alkyl)amino-C₁₋₆-alkyle, alkylamido en C₁₋₆, cycloalkylamido en C₃₋₆, C₃₋₆-cycloalkyl-C₁₋₆-alkylamido, arylamido, alkylaminocarbonyle en C₁₋₆, cycloalkylaminocarbonyle en C₃₋₆, C₃₋₆-cycloalkyl-C₁₋₆-alkylaminocarbonile, di-(C₁₋₆-alkyl)aminocarbonyle, di-(C₃₋₆-cycloalkyl-C₁₋₆-alkyl)aminocarbonyle, alkylamino en C₁₋₆, cycloalkylamino en C₃₋₆, C₃₋₆-cycloalkyl-C₁₋₆-alkylamino, di-(C₁₋₆-alkyl)amino, di-(C₃₋₆-cycloalkyl)amino ou di-(C₃₋₆-cycloalkyl-C₁₋₆-alkyl)amino, chacun desquels est éventuellement substitué par un ou plusieurs halogènes, CN et OH ; ou
X₁ est aralkyle ou hétéroaralkyle, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, aryle, aralkyle, hétéroaryle, hétéroaralkyle, alkoxy en C₁₋₆, cycloalkoxy en C₃₋₆, aryloxy, hétéroaryloxy, aralkoxy, hétéroaralkoxy, alkylthio en C₁₋₆, arylthio, hétéroarylthio, aryl-C₁₋₆-alkylthio, hétéroaryl-C₁₋₆-alkylthio, cycloalkylthio en C₃₋₆, alkylcarbonyle en C₁₋₆, arylcarbonyle, alkylsulfonyle en C₁₋₆, arylsulfonyle, hétéroarylsulfonyle, alkoxycarbonyle en C₁₋₆, alkylamido en C₁₋₆, arylamido, alkylaminocarbonyle en C₁₋₆, di-(C₁₋₆-alkyl)aminocarbonyle, alkylamino en C₁₋₆, di-(C₁₋₆-alkyl)amino ou cycloalkylamino en C₃₋₆, chacun desquels est éventuellement substitué par un ou plusieurs halogènes, CN et OH ; et
X₂ est aryle ou hétéroaryle, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, aryle, aralkyle, hétéroaryle, hétéroaralkyle, alkoxy en C₁₋₆, cycloalkoxy en C₃₋₆, aryloxy, hétéroaryloxy, aralkoxy, hétéroaralkoxy, alkylthio en C₁₋₆, arylthio, hétéroarylthio, aryl-C₁₋₆-alkylthio, hétéroaryl-C₁₋₆-alkylthic, cycloalkylthio en C₃₋₆, alkylcarbonyle en C₁₋₆, arylcarbonyle, alkylsulfonyle en C₁₋₆, arylsulfonyle, hétéroarylsulfonyle, alkoxycarbonyle en C₁₋₆, alkylamino en C₁₋₆, arylamido, alkylaminocarbonyle en C₁₋₆, di-(C₁₋₆-alkyl)aminocarbonyle, alkylamino en C₁₋₆, di-(C₁₋₆-alkyl)amino ou cycloalkylamino en C₃₋₆, chacun desquels est éventuellement substitué par un ou plusieurs halogènes, CN et OH ; ou
X₂ est sélectionné parmi
• hydrogène, halogène, hydroxy, cyano, amino ou carboxy ; ou
• alkyl en C₁₋₆, cycloalkyle en C₃₋₆, alcényle en C₂₋₆, alcynyle en C₂₋₆, aralkyle, hétéroaralkyle, alkoxy en C₁₋₆, cycloalkoxy en C₃₋₆, aryloxy, hétéroaryloxy, aralkoxy, hétéroaralkoxy, alkylthio en C₁₋₆, arylthio, hétéroarylthio, aryl-C₁₋₆-alkylthio, hétéroaryl-C₁₋₆-alkylthio, cycloalkylthio en C₃₋₆, alkylcarbonyle en C₁₋₆, arylcarbonyle, alkylsulfonyle en C₁₋₆, arylsulfonyle, hétéroarylsulronyle, alkoxycarbonyle en C₁₋₆, alkylamido en C₁₋₆, arylamido, alkylaminocarbonyle en C₁₋₆, di-(C₁₋₆-alkyl)aminocarbonyle, alkylamino en C₁₋₆, di-(C₁₋₆-alkyl)amino ou cycloalkylamino en C₃₋₆, chacun desquels est éventuellement substitué par un ou plusieurs halogènes, CN et OH ; et
X₃ est arylène ou hétéroarylène, chacun desquels est éventuellement substitué par un ou plusieurs substituants sélectionnés parmi
• halogène, hydroxy, cyano, amino ou carboxy ; ou
• alkyle en C₁₋₆, cycloalkyle en C₃₋₆, alcényle en C₂₋₆, alcynyle en C₂₋₆, alkoxy en C₁₋₆, cycloalkoxy en C₃₋₆, alkylthio en C₁₋₆, cycloalkylthio en C₃₋₆, alkylamino en C₁₋₆, dialkylamino en C₁₋₆ ou cycloalkylamino en C₃₋₆, chacun desquels est éventuellement substitué par un ou plusieurs halogènes ; et
Ar est phénylène, qui est éventuellement substitué par un ou plusieurs substituants sélectionnés parmi
• halogène ; ou
• alkyle en C₁₋₆, éventuellement substitué par un ou plusieurs halogènes ; et
Y₁ est O ou S ; et
Y₂ est O ou S ; et
Z est -(CH₂)ₙ-, dans lequel n est 1, 2, ou 3 ; et
R₁ est hydrogène, halogène ou un substituant sélectionné parmi
• alkyle en C₁₋₆, cycloalkyle en C₃₋₆, alcényle en C₂₋₆, alcynyle en C₂₋₆, aralkyle, hétéroaralkyle, alkoxy en C₁₋₆, cycloalkoxy en C₃₋₆, aryloxy, aralkoxy, hétéroaralkoxy, alkylthio en C₁₋₆, arylthio ou cycloalkylthio en C₃₋₆, chacun desquels est éventuellement substitué par un ou plusieurs halogènes ; et
R₂ est hydrogène, alkyle en C₁₋₆, cycloalkyle en C₃₋₆, alcényle en C₂₋₆, alcynyle en C₂₋₆, alcénynyle en C₄₋₆ ou aryle ; ou
un sel pharmaceutiquement acceptable de ceux-ci, ou un solvate pharmaceutiquement acceptable de ceux-ci, ou toutes formes tautomères, stéréoisomères, mélanges de stéréoisomères y compris un mélange racémique, ou polymorphes.

3. Composé selon la revendication 1 ou la revendication 2, dans lequel X₁ est aryle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆ ou dialkylamino en C₁₋₆, chacun desquels est éventuellement substitué par un ou plusieurs halogènes.

4. Composé selon la revendication 3, dans lequel X₁ est aryle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio ou dialkylaminocarbonyle en C₁₋₆, chacun desquels est éventuellement substitué par un ou plusieurs halogènes.

5. Composé selon la revendication 4, dans lequel X₁ est aryle éventuellement substitué par un ou plusieurs substituants sélectionnés parmi
• halogène ; ou
• alkyle en C₁₋₆, alkoxy en C₁₋₆, alkylthio en C₁₋₆, chacun desquels est éventuellement substitué par un ou plusieurs halogènes.

6. Composé selon l'une quelconque des revendications précédentes, dans lequel X₁ est phényle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆ ou dialkylamino en C₁₋₆, chacun desquels est éventuellement substitué par un ou plusieurs halogènes.

7. Composé selon la revendication 6, dans lequel X₁ est phényle éventuellement substitué par un ou plusieurs substituants sélectionnés parmi
• halogène ; ou
• alkyle en C₁₋₆, alkoxy en C₁₋₆ ou alkylthio en C₁₋₆, chacun desquels est éventuellement substitué par un ou plusieurs halogènes.

8. Composé selon l'une quelconque des revendications précédentes, dans lequel X₁ est phényle.

9. Composé selon la revendication 1 ou la revendication 2, dans lequel X₁ est hétéroaryle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆ ou dialkylamino en C₁₋₆, chacun desquels est éventuellement substitué par un ou plusieurs halogènes.

10. Composé selon la revendication 9, dans lequel X₁ est hétéroaryle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio ou dialkylaminocarbonyle en C₁₋₆, chacun desquels est éventuellement substitué par un ou plusieurs halogènes.

11. Composé selon la revendication 10, dans lequel X₁ est hétéroaryle éventuellement substitué par un ou plusieurs substituants sélectionnés parmi
• halogène ; ou
• alkyle en C₁₋₆, alkoxy en C₁₋₆ ou alkylthio en C₁₋₆, chacun desquels est éventuellement substitué par un ou plusieurs halogènes.

12. Composé selon la revendication 1, dans lequel X₁ est alkyle en C₁₋₆ éventuellement substitué par un ou plusieurs substituants sélectionnés parmi
• halogène ou hydroxy ; ou
• aryle, hétéroaryle, hétérocyclyle, alkoxy en C₁₋₆,
• alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆, dialkylamino en C₁₋₆ ou cycloalkylamino en C₃₋₆, chacun desquels est éventuellement substitué par un ou plusieurs halogènes.

13. Composé selon la revendication 12, dans lequel X₁ est alkyl en C₁₋₆ éventuellement substitué par un ou plusieurs substituants sélectionnés parmi
• halogène ou hydroxy ; ou
• aryle, hétéroaryle, alkoxy en C₁₋₆, alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆, dialkylamino en C₁₋₆ ou cycloalkylamino en C₃₋₆, chacun desquels est éventuellement substitué par un ou plusieurs halogènes.

14. Composé selon la revendication 13, dans lequel X₁ est alkyle en C₁₋₆ éventuellement substitué par un ou plusieurs substituants sélectionnés parmi
• halogène ou hydroxy ; ou
• aryle, hétéroaryle, alkoxy en C₁₋₆, alkylthio en C₁₋₆, alkylcarbonyle en C₁₋₆, alkylsulfonyle en C₁₋₆, alkylamido en C₁₋₆, alkylaminocarbonyle en C₁₋₆, dialkylaminocarbonyle en C₁₋₆, alkylamino en C₁₋₆, dialkylamino en C₁₋₆ ou cycloalkylamino en C₃₋₅, chacun desquels est éventuellement substitué par un ou plusieurs halogènes.

15. Composé selon la revendication 14, dans lequel X₁ est alkyle en C₁₋₆ éventuellement substitué par un ou plusieurs substituants sélectionnés parmi halogène ou hydroxy.

16. Composé selon la revendication 1 ou la revendication 2, dans lequel X₁ est cycloalkyle en C₃₋₆ éventuellement substitué par un ou plusieurs substituants sélectionnés parmi
• halogène ou hydroxy ; ou
• aryle, hétéroaryle, alkoxy en C₁₋₆, alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆, dialkylamino en C₁₋₆ ou cycloalkylamino en C₃₋₆, chacun desquels est éventuellement substitué par un ou plusieurs halogènes.

17. Composé selon la revendication 16, dans lequel X₁ est cycloalkyle en C₃₋₆ éventuellement substitué par un ou plusieurs substituants sélectionnés parmi
• halogène ou hydroxy ; ou
• aryle, hétéroaryle, alkoxy en C₁₋₆, alkylthio en C₁₋₆, alkylcarbonyle en C₁₋₆, alkylsulfonyle en C₁₋₆, alkylamido en C₁₋₆, alkylaminocarbonyle en C₁₋₆, dialkylaminocarbonyle en C₁₋₆, alkylamino en C₁₋₆, dialkylamino en C₁₋₆ ou cycloalkylamino en C₃₋₆, chacun desquels est éventuellement substitué par un ou plusieurs halogènes.

18. Composé selon la revendication 17, dans lequel X₁ est cycloalkyle en C₃₋₆ éventuellement substitué par un ou plusieurs substituants sélectionnés parmi halogène ou hydroxy.

19. Composé selon la revendication 1 ou la revendication 2, dans lequel X₁ est aralkyle ou hétéroaralkyle, chacun desquels est éventuellement substitué par un ou plusieurs substituants sélectionnés parmi
• halogène ou hydroxy ; ou
• alkoxy en C₁₋₆, alkylthio en C₁₋₆, arylthio, alkylcarbonyle en C₁₋₆, arylcarbonyle, alkylsulfonyle en C₁₋₆, arylsulfonyle, alkylamino en C₁₋₆, arylamido, alkylaminocarbonyle en C₁₋₆, dialkylaminocarbonyle en C₁₋₆, alkylamino en C₁₋₆, dialkylamino en C₁₋₆ ou cycloalkylamino en C₃₋₆, chacun desquels est éventuellement substitué par un ou plusieurs halogènes.

20. Composé selon la revendication 19, dans lequel X₁ est aralkyle ou hétéroaralkyle, chacun desquels est éventuellement substitué par un ou plusieurs substituants sélectionnés parmi
• halogène ou hydroxy ; ou
• alkoxy en C₁₋₆ qui est éventuellement substitué par un ou plusieurs halogènes.

21. Composé selon l'une quelconque des revendications précédentes, dans lequel X₂ est aryle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆ ou dialkylamino en C₁₋₆, chacun desquels est éventuellement substitué par un ou plusieurs halogènes.

22. Composé selon la revendication 21, dans lequel X₂ est aryle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio, ou dialkylaminocarbonyle en C₁₋₆, chacun desquels est éventuellement substitué par un ou plusieurs halogènes.

23. Composé selon la revendication 22, dans lequel X₂ est aryle éventuellement substitué par un ou plusieurs substituants sélectionnés parmi
• halogène ; ou
• alkyle en C₁₋₆, alkoxy en C₁₋₆ ou alkylthio en C₁₋₆, chacun desquels est éventuellement substitué par un ou plusieurs halogènes.

24. Composé selon l'une quelconque des revendications précédentes 21 à 23, dans lequel X₂ est phényle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆ ou dialkylamino en C₁₋₆, chacun desquels est éventuellement substitué par un ou plusieurs halogènes.

25. Composé selon la revendication 24, dans lequel x₂ est phényle éventuellement substitué par un ou plusieurs substituants sélectionnés parmi
• halogène ; ou
• alkyle en C₁₋₆, alkoxy en C₁₋₆ ou alkylthio en C₁₋₆, chacun desquels est éventuellement substitué par un ou plusieurs halogènes.

26. Composé selon la revendication 25, dans lequel x₂ est phényle.

27. Composé selon l'une quelconque des revendications 1 à 20, dans lequel X₂ est hétéroaryle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio, alkylcarbonyle en C₁₋₆, arylcarbonyle, alkylsulfonyle en C₁₋₆, arylsulfonyle, alkylamido en C₁₋₆, arylamido, alkylaminocarbonyle en C₁₋₆, dialkylaminocarbonyle en C₁₋₆, alkylamino en C₁₋₆ ou dialkylamino en C₁₋₆, chacun desquels est éventuellement substitué par un ou plusieurs halogènes.

28. Composé selon la revendication 27, dans lequel X₂ est hétéroaryle éventuellement substitué par un ou plusieurs substituants sélectionnés parmi
• halogène, hydroxy ; ou
• alkyle en C₁₋₆, aryle, hétéroaryle, alkoxy en C₁₋₆, aryloxy, aralkoxy, hétéroaralkoxy, alkylthio en C₁₋₆, arylthio ou dialkylaminocarbonyle en C₁₋₆, chacun desquels est éventuellement substitué par un ou plusieurs halogènes.

29. Composé selon la revendication 28, dans lequel X₂ est hétéroaryle éventuellement substitué par un ou plusieurs substituants sélectionnés parmi
• halogène ; ou
• alkyle en C₁₋₆, alkoxy en C₁₋₆ ou alkylthio en C₁₋₆, chacun desquels est éventuellement substitué par un ou plusieurs halogènes.

30. Composé selon l'une quelconque des revendications précédentes 1 à 20, dans lequel X₂ est hydrogène, halogène, hydroxy ou cyano ; ou
• alkyle en C₁₋₆, aralkyle ou hétéroaralkyle, chacun desquels est éventuellement substitué par un ou plusieurs halogènes, CN ou OH.

31. Composé selon la revendication 30, dans lequel X₂ est halogène ou hydroxy ; ou alkyle en C₁₋₆ éventuellement substitué par un ou plusieurs halogènes.

32. Composé selon la revendication 31, dans lequel X₂ est halogène.

33. Composé selon l'une quelconque des revendications précédentes, dans lequel X₃ est arylène, éventuellement substitué par un ou plusieurs substituants sélectionnés parmi
• halogène ou
• alkyle en C₁₋₆, alkoxy, en C₁₋₆ ou alkylthio en C₁₋₆, chacun desquels est éventuellement substitué par un ou plusieurs halogènes.

34. Composé selon la revendication 33, dans lequel X₃ est phénylène éventuellement substitué par un ou plusieurs substituants sélectionnés parmi
• halogène ou
• alkyle en C₁₋₆, alkoxy en C₁₋₆ ou alkylthio en C₁₋₆, chacun desquels est éventuellement substitué par un ou plusieurs halogènes.

35. Composé selon la revendication 34, dans lequel X₃ est phénylène éventuellement substitué par un ou plusieurs substituants sélectionnés parmi halogène ou alkyle en C₁₋₆, qui est éventuellement substitué par un ou plusieurs halogènes.

36. Composé selon la revendication 35, dans lequel X₃ est phénylène.

37. Composé selon l'une quelconque des revendications précédentes 1 à 32, dans lequel X₃ est hétéroarylène éventuellement substitué par un ou plusieurs substituants sélectionnés parmi
• halogène ou
• alkyle en C₁₋₆, alkoxy en C₁₋₆ ou alkylthio en C₁₋₆, chacun desquels est éventuellement substitué par un ou plusieurs halogènes.

38. Composé selon la revendication 37, dans lequel X₃ est hétéroarylène éventuellement substitué par un ou plusieurs substituants sélectionnés parmi halogène ou alkyle en C₁₋₆, qui est éventuellement substitué par un ou plusieurs halogènes.

39. Composé selon l'une quelconque des revendications précédentes, dans lequel Y₁ est S.

40. Composé selon l'une quelconque des revendications précédentes, dans lequel Y₂ est O.

41. Composé selon l'une quelconque des revendications précédentes, dans lequel n est 1.

42. Composé selon l'une quelconque des revendications précédentes, dans lequel R₁ est hydrogène ou un substituant sélectionné parmi
• alkyle en C₁₋₆, aralkyle, alkoxy en C₁₋₆, aryloxy, aralkoxy, chacun desquels est éventuellement substitué par un ou plusieurs halogènes.

43. Composé selon la revendication 42, dans lequel R₁ est hydrogène ou un substituant sélectionné parmi
• alkyle en C₁₋₆, alkoxy en C₁₋₆, chacun desquels est éventuellement substitué par un ou plusieurs halogènes.

44. Composé selon la revendication 43, dans lequel R₁ est hydrogène.

45. Composé selon l'une quelconque des revendications précédentes, dans lequel R₂ est hydrogène ou alkyle en C₁₋₆.

46. Composé selon la revendication 45, dans lequel R₂ est hydrogène.

47. Composé selon l'une quelconque des revendications précédentes, qui est :
acide acétique de (Z)-[4-[3-(4-bromophényl)-5-phénylpent-2-en-4-ynylsulfanyl]-2-méthylphénoxy] ;
acide acétique de (Z)-[4-[3-(4-bromophényl)-6-hydroxyhex-2-en-4-ynylsulfanyl]-2-méthylphénoxyl ;
acide acétique de [4-[3-(biphényl-4-yl)-6-hydroxyhex-2-en-4-ynylsulfanyl]-2-méthylphénoxy] ;
acide acétique de (Z)-[4-[3-(biphényl-4-yl)-6-hydroxy-6-méthylhept-2-en-4-ynylsulfanyl]-2-méthylphénoxyl ;
acide acétique de (Z)-[4-[5-(4-méthoxyphényl)-3-(biphényl-4-yl)-pent-2-en-4-ynylsulfanyl]-2-méthylphénoxyl ;
acide acétique de (Z)-[4-[5-(4-méthoxyphényl)-3-(4-trifluorométhylphényl)-pent-2-en-4-ynylsulfanyl-1-2-méthylphénoxy] ; ou
un sel de ceux-ci avec un acide ou une base pharmaceutiquement acceptable, ou tout isomère optique ou mélange d'isomères optiques, y compris un mélange racémique, ou toutes formes tautomères.

48. Composé selon l'une quelconque des revendications précédentes 1 à 46, qui est :
acide acétique de (Z)[4-(3,5-diphényl-pent-2-en-4-ynyloxy)-2-méthylphénoxy] ;
acide acétique de (Z)-[4-[3-(4-bromophényl)-5-phénylpent-2-en-4-ynyloxyl-2-méthylphénoxy] ;
acide acétique de (Z)-[4-[3-(4-bromophényl)-5-(pyridin-2-yl)pent-2-en-4-ynyloxyl-2-méthylphénoxyl ;
acide acétique de {4-[3-(4-bromophényl)-5-(6-méthyl-thiophèn-2-yl)-pent-2-en-4-ynyloxy] -2-méthylphénoxy} ;
acide acétique de {2-méthyl-4-[3-(2-méthyl-benzofuran-5-yl)-5-(5-méthyl-thiophèn-2-yl)-pent-2-en-4-ynyloxy]-phénoxy} ;
acide acétique de {4-[3-(4-bromophényl)-6-morpholin-4-yl-hex-2-en-4-ynyloxyl-2-méthylphénoxy} ;
acide acétique de (2-méthyl-4-{3-[4-(5-méthyl-thiophèn-2-yl)-phényl]-5-phényl-pent-2-en-4-ynyloxy}-phénoxy) ; ou
un sel de ceux-ci avec un acide ou une base pharmaceutiquement acceptable, ou tout isomère optique ou mélange d'isomères optiques, y compris un mélange racémique, ou toutes formes tautomères.

49. Combinaison d'un composé tel que défini dans l'une quelconque des revendications 1 à 48 et d'une ou plusieurs substances pharmacologiquement actives supplémentaires.

50. Composition pharmaceutique comprenant, en tant qu'ingrédient actif, au moins un composé selon l'une quelconque des revendications 1 à 48 ou une combinaison selon la revendication 49, avec un ou plusieurs supports ou excipients pharmaceutiquement acceptables.

51. Composition pharmaceutique selon la revendication 50 sous forme de dosage unitaire, comprenant de 0,05 mg à 1000 mg, de préférence de 0,1 à 500 mg, et particulièrement préféré de 0,5 mg à 200 mg par jour de composé selon l'une quelconque des revendications 1 à 48.

52. Composition pharmaceutique selon la revendication 50 ou la revendication 51, pour une administration orale, nasale, transdermique, pulmonaire, ou parentérale.

53. Composé tel que défini dans l'une quelconque des revendications 1 à 48, combinaison telle que définie à la revendication 49, ou composition telle que définie dans l'une quelconque des revendications 50 à 52, à utiliser comme médicament.

54. Composé, combinaison ou composition selon la revendication 53, pour le traitement et/ou la prévention de conditions médiées par des récepteurs nucléaires, en particulier les récepteurs activés par les proliférateurs des peroxysomes (PPAR).

55. Composé, combinaison ou composition selon la revendication 53, pour le traitement et/ou la prévention du diabète de type 1 ; diabète de type 2 ; dyslipidémie ; syndrome x, y compris le syndrome métabolique, c.-à-d. l'intolérance au glucose, la résistance à l'insuline, l'hypertriglycéridémie et/ou l'obésité ; maladies cardiovasculaires, y compris l'athérosclérose ; et hypercholestérolémie.

56. Composé, combinaison ou composition selon la revendication 53, pour le traitement et/ou la prévention du diabète de type 1, diabète de type 2, intolérance au glucose, résistance à l'insuline, ou obésité.

57. Composé, combinaison ou composition selon la revendication 55 ou la revendication 56, dans lequel la quantité efficace du composé selon l'une quelconque des revendications 1 à 48 est dans la gamme de 0,05 mg à 1000 mg, de préférence de 0,1 à 500 mg, et particulièrement préféré de 0,5 mg à 200 mg par jour.

58. utilisation d'un composé selon l'une quelconque des revendications 1 à 48, pour la préparation d'une composition pharmaceutique pour le traitement et/ou la prévention de conditions médiées par des récepteurs nucléaires, en particulier les récepteurs activés par les proliférateurs des peroxysomes (PPAR).

59. Utilisation d'un composé selon l'une quelconque des revendications 1 à 48, pour la préparation d'une composition pharmaceutique pour le traitement et/du la prévention du diabète de type 1 ; diabète de type 2 ; dyslipidémie ; syndrome x, y compris le syndrome métabolique, c.-à-d. l'intolérance au glucose, la résistance à l'insuline, l'hypertriglycéridémie et/ou l'obésité ; maladies cardiovasculaires, y compris l'athérosclérose ; et hypercholestérolémie.
